# EUROPEAN PATENT APPLICATION

(11) **EP 4 714 951 A1**
(43) Date of publication of application: **25.03.2026**
(21) Application number: 24810297.2
(22) Date of filing: 16.05.2024
(51) Int. Cl.: C07D 471/04, C12Q 1/68

(54) **FLUORESCENT DYE, SYNTHESIS THEREFOR AND USE THEREOF**

(30) Priority: 19.05.2023 WO PCT/CN2023/095322
(71) Applicant: MGI Tech Co., Ltd., Shenzhen, Guangdong 518083 (CN); Wuhan MGI Tech Co., Ltd., Wuhan, Hubei 430075 (CN)
(72) Inventor: LU, Zhenhao, Wuhan, Hubei 430075 (CN); JIA, Man, Shenzhen, Guangdong 518083 (CN); XU, Chongjun, Shenzhen, Guangdong 518083 (CN); TANG, Xintong, Wuhan, Hubei 430075 (CN); FENG, Zhiwen, Wuhan, Hubei 430075 (CN); XING, Chengmei, Shenzhen, Guangdong 518083 (CN)
(74) Representative: Hoffmann Eitle
(86) International application number: PCT/CN2024/093716
(87) International publication number: WO 2024/240051

(57) **Abstract**

The present invention relates to a dye compound and a use thereof as a fluorescent marker, and also relates to a method for preparing the compound, a nucleotide or oligonucleotide labeled by the compound, and a nucleic acid sequencing method.

## Description

### Technical field

The present invention relates to the fields of organic chemistry, fluorescent dyes, and gene sequencing. In particular, the present invention relates to a dye compound and a use thereof as a fluorescent marker, and also relates to a method for preparing the compound, a nucleotide or oligonucleotide labeled by the compound, and a nucleic acid sequencing method.

### Background art

Next-generation sequencing, also known as high-throughput sequencing, achieves sequencing-by-synthesis through the introduction of reversible terminators. During DNA replication, the DNA sequence is determined by capturing the fluorescent dye labels attached to the newly added bases. Currently, most commercially available sequencers rely on next-generation sequencing based on the sequencing-by-synthesis method. In the sequencing process, base identification is realized by detecting the fluorescent dyes modified on the bases. The dyes used to modify the four types of bases typically have emission wavelengths in two regions: green light and red light. This results in partial overlap of the emission wavelengths of the two types of base dyes, and the poor distinguishability between these dyes is a key factor leading to increased sequencing error rates or decreased sequencing quality. Therefore, the development of new dyes that have emission wavelengths in other visible light regions for use in gene sequencing will help improve sequencing accuracy.

### Contents of the invention

Acridine (azaanthracene) is a macrocyclic conjugated system with a tricyclic rigid planar structure and strong fluorescence. It can serve as a type of excellent fluorescent marker and be applied in fields such as in vitro diagnosis, immunoassay, and molecular labeling. Generally, dyes with acridine as the basic parent nucleus are synthesized using aniline derivatives as starting materials, and acridine derivatives are synthesized through multiple steps of reactions.

During the structural modification of the substituents of dye AF532, the inventors found that under ammonia/methanol conditions, AF532 undergoes a substitution reaction due to the influence of its own substituents (1,8-disulfonic groups), and the parent nucleus changes from the original xanthene to an acridine structure.

The present invention uses substituted xanthenes as raw materials to synthesize acridine dyes under ammonia/methanol reaction conditions. The corresponding maximum excitation wavelength and emission wavelength of the synthesized dyes undergo a blue shift; under the maximum excitation wavelength of blue light, their emission wavelength falls in the cyan light region. Thus, a dye capable of emitting cyan light is obtained.

The present application provides the following invention:

### Dye compound

In one aspect, the present application provides a compound represented by formula (I), an ester thereof, or a salt thereof, 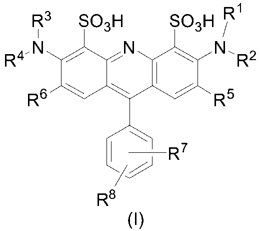
wherein, R¹, R², R³ and R⁴, the same or different from each other, are each independently selected from H, C₁-C₆ alkyl and halo-C₁-C₆ alkyl;
R⁵ and R⁶, the same or different, are each independently selected from H, C₁-C₆ alkyl, halogen and halo-C₁-C₆ alkyl; R⁷ and R⁸, the same or different, are each independently selected from H, - COOH, -C(O)NH-(C₁-C₆ alkyl) and -C(O)NH₂, and R⁷ and R⁸ are not simultaneously H;
optionally, -NR¹R², together with the benzene ring to which it is attached, forms a benzo 5-to 6-membered nitrogen-containing heterocyclic group, said 5- to 6-membered nitrogen-containing heterocyclic group being optionally substituted with one or more groups selected from C₁-C₆ alkyl, halogen and halo-C₁-C₆ alkyl;
optionally, -NR³R⁴, together with the benzene ring to which it is attached, forms a benzo 5-to 6-membered nitrogen-containing heterocyclic group, said 5- to 6-membered nitrogen-containing heterocyclic group being optionally substituted with one or more groups selected from C₁-C₆ alkyl, halogen and halo-C₁-C₆ alkyl.

In certain embodiments, R¹ and R² are the same. In certain embodiments, R³ and R⁴ are the same. In certain embodiments, R¹, R², R³ and R⁴ are the same.

In certain embodiments, R¹, R², R³ and R⁴ are each H.

In certain embodiments, R¹, R², R³ and R⁴ are each C₁-C₆ alkyl (such as methyl, ethyl).

In certain embodiments, R¹, R², R³ and R⁴ are each Halo-C₁-C₆ alkyl (such as trifluoromethyl, trifluoroethyl).

In certain embodiments, R¹ and R³ are the same. In certain embodiments, R² and R⁴ are the same.

In certain embodiments, R¹ and R³ are the same, each being H, R² and R⁴ are the same, each being halo-C₁-C₆ alkyl (such as trifluoromethyl, trifluoroethyl).

In certain embodiments, R⁵ and R⁶ are the same.

In certain embodiments, R⁵ and R⁶ are each H.

In certain embodiments, R⁵ and R⁶ are each halogen (such as F).

In certain embodiments, R⁷ is carboxyl.

In certain embodiments, R⁸ is H.

In certain embodiments, R⁸ is carboxyl.

In certain embodiments, -NR¹R², together with the benzene ring to which it is attached, forms a benzo 5- to 6-membered nitrogen-containing heterocyclic group, said 5- to 6-membered nitrogen-containing heterocyclic group being optionally substituted with one or more methyl groups.

In certain embodiments, -NR³R⁴, together with the benzene ring to which it is attached, forms a benzo 5- to 6-membered nitrogen-containing heterocyclic group, said 5- to 6-membered nitrogen-containing heterocyclic group being optionally substituted with one or more methyl groups.

In certain embodiments, R⁷ or R⁸ is carboxyl, said carboxyl being connected with a cleavable linker, such as a cleavable linker having a structure as shown below:

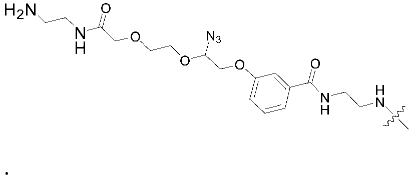

In certain embodiments, the compound of the present invention has a structure represented by formula (II): 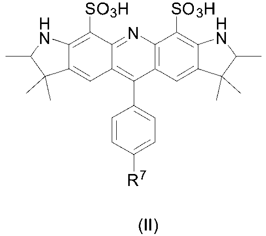 wherein, R⁷ is as defined above.

In certain embodiments, the compound of the present invention has a structure represented by formula (III): 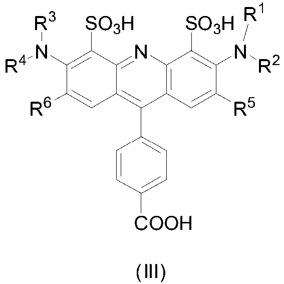 wherein, R¹, R², R³, R⁴, R⁵, R⁶ and R⁷ are as defined above.

In certain embodiments, the compound of the present invention has a structure represented by formula (IV): 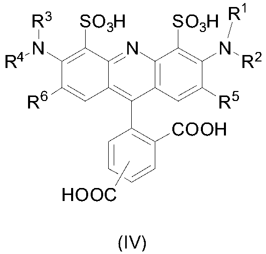 wherein, R¹, R², R³, R⁴, R⁵ and R⁶ are as defined above.

The compound of the present invention may have a structure selected from the following: 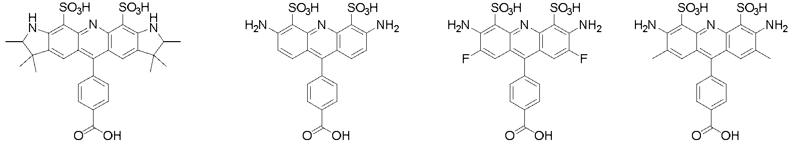 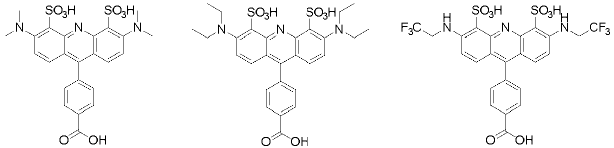 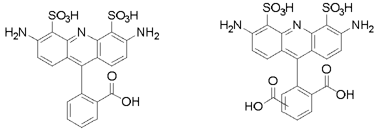 and

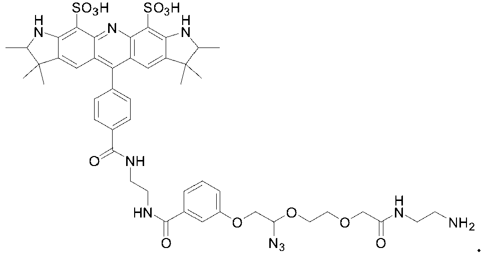

In one aspect, the present application provides a compound represented by formula (I'), an ester thereof, or a salt thereof, 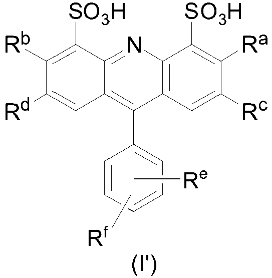
wherein, R^{a}, R^{b}, R^{c} and R^{d}, the same or different from each other, are each independently selected from H, C₁-C₆ alkyl, halo-C₁-C₆ alkyl, -NR^{1'}R^{2'}, hydroxy, hydroxy-substituted C₁-C₆ alkyl, and halogen; R^{1'} and R^{2'}, the same or different from each other, are each independently selected from H, C₁-C₆ alkyl and halo-C₁-C₆ alkyl;
R^{e} and R^{f}, the same or different, are each independently selected from H, -COOH and - C(O)NR^{3'}R^{4'}, and R^{e} and R^{f} are not simultaneously H;
R^{3'} and R^{4'}, the same or different from each other, are each independently selected from H and C₁-C₆ alkyl, said C₁-C₆ alkyl being optionally substituted with carboxyl, -C(O)NH₂ or sulfonic group;
optionally, when R^{a} is -NR^{1'}R^{2'}, -NR^{1'}R^{2'}, together with the benzene ring to which it is attached, forms a benzo 5- to 6-membered nitrogen-containing heterocyclic group, said 5- to 6-membered nitrogen-containing heterocyclic group being optionally substituted with one or more groups selected from C₁-C₆ alkyl, halogen and halo-C₁-C₆ alkyl;
optionally, when R^{b} is -NR^{1'}R^{2'}, -NR^{1'}R^{2'}, together with the benzene ring to which it is attached, forms a benzo 5- to 6-membered nitrogen-containing heterocyclic group, said 5- to 6-membered nitrogen-containing heterocyclic group being optionally substituted with one or more groups selected from C₁-C₆ alkyl, halogen and halo-C₁-C₆ alkyl.

In certain embodiments, R^{a} and R^{b} are the same.

In certain embodiments, R^{a} and R^{b} are the same, each being -NR^{1'}R^{2'} or each being hydroxy .

In certain embodiments, R^{c} and R^{d} are the same.

In certain embodiments, R^{c} and R^{d} are the same, each being H or each being halogen.

In certain embodiments, R^{1'} and R^{2'} are each H.

In certain embodiments, one of R^{1'} and R^{2'} is H, and the other is selected from C₁-C₆ alkyl (such as methyl, ethyl).

In certain embodiments, R^{e} and R^{f} are different, each being independently selected from H, - COOH and -C(O)NR^{3'}R^{4'}.

In certain embodiments, R^{e} and R^{f} are different, each being independently selected from H and -COOH.

In certain embodiments, R^{3'} and R^{4'}, the same or different from each other, are each independently selected from H and C₁-C₆ alkyl, said C₁-C₆ alkyl being optionally substituted with carboxyl or sulfonic group.

In certain embodiments, R^{a} is -NR^{1'}R^{2'}; -NR^{1'}R^{2'}, together with the benzene ring to which it is attached, forms a benzo 5- to 6-membered nitrogen-containing heterocyclic group, said 5- to 6-membered nitrogen-containing heterocyclic group being optionally substituted with one or more methyl groups.

In certain embodiments, R^{b} is -NR^{1'}R^{2'}; -NR^{1'}R^{2'}, together with the benzene ring to which it is attached, forms a benzo 5- to 6-membered nitrogen-containing heterocyclic group, said 5- to 6-membered nitrogen-containing heterocyclic group being optionally substituted with one or more methyl groups.

In certain embodiments, the compound of the present invention has a structure represented by formula (II'): 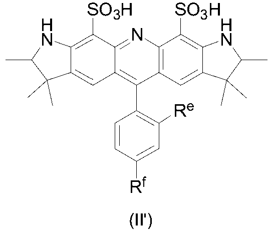 wherein, R^{e} and R^{f} are as defined above.

In certain embodiments, the compound of the present invention has a structure represented by formula (III'): 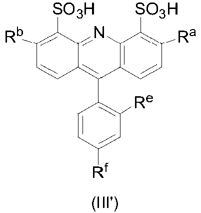 wherein, R^{a}, R^{e} and R^{f} are as defined above.

The compound of the present invention may have a structure selected from the following:

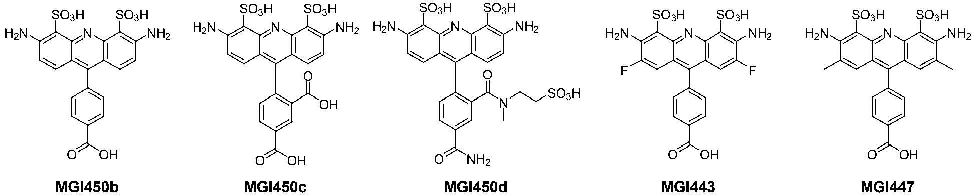

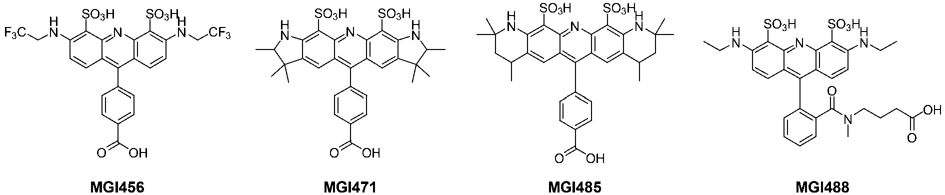

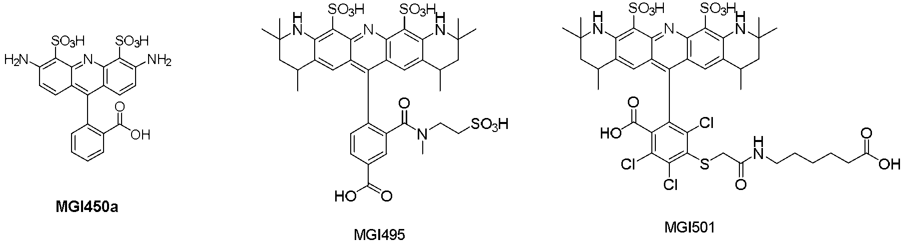

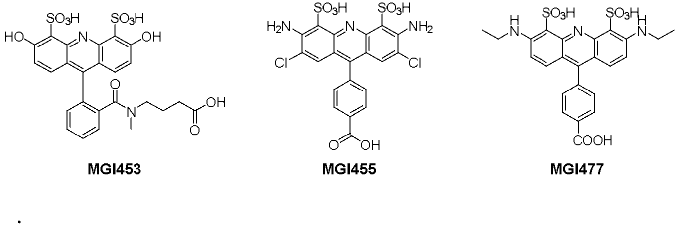

The compounds of the present invention can be conjugated to nucleotides or oligonucleotides as fluorescent dyes. Therefore, in certain embodiments, the compound represented by formula (I) of the present invention is covalently attached to a nucleotide or oligonucleotide via R⁷, R⁷ being selected from -COOH, -C(O)NH-(C₁-C₆ alkyl) and -C(O)NH₂. In certain embodiments, the compound represented by formula (I') of the present invention is covalently attached to a nucleotide or oligonucleotide via R^{e} or R^{f}.

In certain embodiments, the compound represented by formula (I) or formula (I') of the present invention can be covalently attached to a nucleotide or oligonucleotide via a cleavable linker. Therefore, the present invention also provides the compound represented by formula (i): 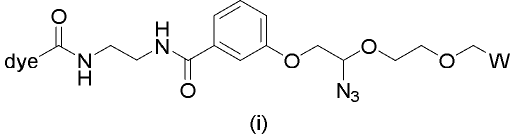 wherein, w is carboxyl or ester group, the dye is the dye compound mentioned above, including the compound represented by formula (I) or formula (I'), an ester thereof, or a salt thereof. In certain embodiments, the dye is the compound represented by formula (I'), which is connected to the rest moiety 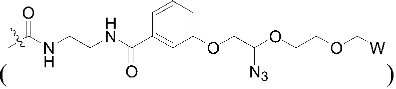 via R^{e} or R^{f}.

The ester described in the present invention is preferably an activated ester of carboxyl group. As used herein, the term "activated ester" refers to a carboxyl group derivative that can react with a compound containing amino group, for example, under mild conditions. Non-limiting examples of activated esters include, but are not limited to, p-nitrophenyl ester, pentafluorophenyl ester, and succinimidyl ester ( 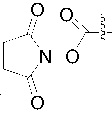 )

The salts of the compounds of the present invention are preferably salts formed by the sulfonic group on an acridine ring, such as salts formed by the sulfonic group with alkali metal ions, alkaline earth metal ions, or ammonium ions.

In certain embodiments, the compound represented by formula (i) has the following structure: 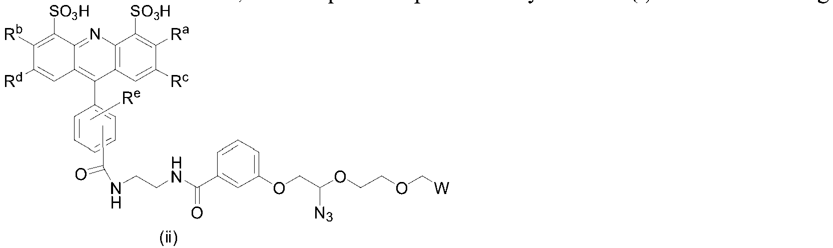 wherein, R^{a}, R^{b}, R^{c}, R^{d} and R^{e} are as defined above.

In certain embodiments, the compound represented by formula (i) has the following structure: 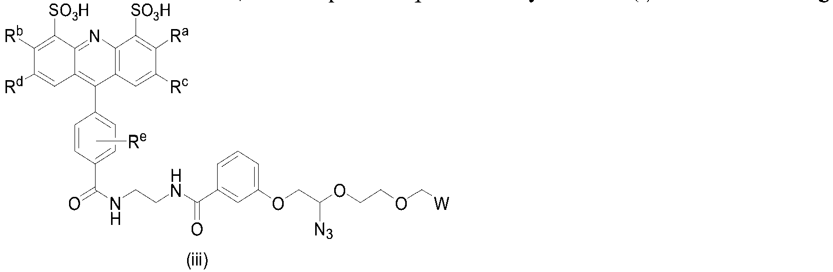 wherein, R^{a}, R^{b}, R^{c}, R^{d} and R^{e} are as defined above.

In certain embodiments, the compound represented by formula (i) has the following structure: 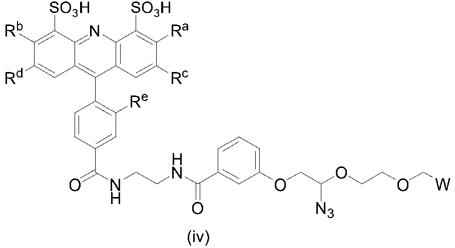 wherein, R^{a}, R^{b}, R^{c}, R^{d} and R^{e} are as defined above.

The compound represented by formula (i) can be used as an intermediate for synthesizing nucleotides or oligonucleotides labeled with dyes according to the present invention.

### Terms

As used herein, the term "C₁-C₆ alkyl" refers to a group obtained by removing one hydrogen atom from a straight-chain or branched-chain alkane containing 1 to 6 carbon atoms. Specific examples thereof include, but are not limited to: methyl, ethyl, propyl, n-butyl, isobutyl, isopropyl, tert-butyl, n-pentyl, and n-hexyl. In the present invention, the preferred C₁-C₆ alkyl is C₁-C₄ alkyl.

As used herein, the term "halogen" includes fluorine, chlorine, bromine, and iodine.

As used herein, the term "halo" means that hydrogen atom(s) on a group or compound are substituted with one or more halogen atoms, including perhalogenation and partial halogenation.

As used herein, the term "heterocyclic group" refers to a group obtained by removing one hydrogen atom from a saturated or partially saturated monocyclic or fused-ring compound containing 3 to 14 ring atoms and at least one heteroatom (e.g., 1, 2, 3, 4, or 5 heteroatoms). The term "5- to 6-membered nitrogen-containing heterocyclic group" refers to a heterocyclic group containing 5 or 6 ring atoms, among which 1, 2, or 3 ring atoms are nitrogen atoms; optionally, the heterocyclic group further contains 1 or 2 oxygen atoms or sulfur atoms.

As used herein, the term "salt" refers to: (i) salts formed by acidic functional groups (e.g., - COOH) present in the compounds provided by the present invention with appropriate inorganic or organic cations (bases), including but not limited to alkali metal salts such as sodium salts, potassium salts, lithium salts, etc.; alkaline earth metal salts such as calcium salts, magnesium salts, etc.; other metal salts such as aluminum salts, iron salts, zinc salts, copper salts, nickel salts, cobalt salts, etc.; inorganic base salts such as ammonium salts; organic base salts such as tert-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylamine salts, and tris(hydroxymethyl)aminomethane salts. And, (ii) salts formed by basic functional groups (e.g., -NH₂) present in the compounds provided by the present invention with appropriate inorganic or organic anions (acids), including but not limited to hydrohalide salts such as hydrofluoride salts, hydrochloride salts, hydrobromide salts, hydroiodide salts, etc.; inorganic acid salts such as nitrate salts, perchlorate salts, sulfate salts, phosphate salts, etc.; lower alkanesulfonate salts such as methanesulfonate salts, trifluoromethanesulfonate salts, ethanesulfonate salts, etc.; arylsulfonate salts such as benzenesulfonate salts, p-toluenesulfonate salts, etc.; organic acid salts such as acetate salts, malate salts, fumarate salts, succinate salts, citrate salts, tartrate salts, oxalate salts, maleate salts, etc.; and amino acid salts such as glycine salts, trimethylglycine salts, arginine salts, ornithine salts, glutamate salts, aspartate salts, etc.

As used herein, the term "ester" refers to esters formed by -COOH groups present in the compounds provided by the present invention with appropriate alcohols, or esters formed by -OH groups present in the compounds provided by the present invention with appropriate acids (e.g., carboxylic acids or oxygen-containing inorganic acids). Suitable ester groups include, but are not limited to, formate, acetate, propionate, butyrate, acrylate, ethyl succinate, stearate, or palmitate. Esters can undergo hydrolysis reactions in the presence of acids or bases to form the corresponding acids or alcohols.

### Labeled nucleotides

The dye compounds of the present invention are suitable for attachment to a substrate moiety. The substrate moiety can actually be any molecule or substance to which the fluorescent dyes described herein can be conjugated, and the dyes can be attached to the substrate by way of non-limiting examples. Substrate moieties can include nucleosides, nucleotides, polynucleotides, carbohydrates, proteins, antibodies, ligands, particles or solid surfaces, organic polymers and inorganic polymers, and combinations or assemblages thereof, such as chromosomes, cell nuclei, living cells, and the like. In some cases, such labeled nucleotides are also referred to as "modified nucleotides".

A particularly useful application of the fluorescent dyes of the present invention is for labeling biomolecules, such as nucleotides or oligonucleotides. Thus, in one aspect, the present application relates to nucleotides or oligonucleotides labeled with the fluorescent compounds of the present invention.

Attachment to biomolecules can be accomplished via the -C(=O)R⁷ moiety of the compound of formula (I). In certain embodiments, R is a substituted alkoxy group, which can be used to attach to amino groups of biomolecules. In one embodiment, the -C(=O)R⁷ moiety can be an activated ester residue most suitable for additional amide bond/peptide bond formation.

In certain embodiments, the dye compound can be covalently attached to an oligonucleotide or nucleotide via a nucleoside base. For example, a labeled nucleotide or oligonucleotide can have a label attached via a linker moiety to a C5 position of a pyrimidine base or a C7 position of a 7-deazapurine base. Labeled nucleotides or oligonucleotides can also have a 3'OH blocking group covalently attached to the ribose or deoxyribose of the nucleotide.

Nucleosides and nucleotides can be labeled at sites on the sugar or nucleobase. As understood by one of ordinary skill in the art, a "nucleotide" consists of a nitrogen-containing base, a sugar, and one or more phosphate groups. In RNA, the sugar is ribose and in DNA the sugar is deoxyribose, a sugar lacking the hydroxyl group present in ribose. The nitrogen-containing bases are derivatives of purines or pyrimidines. Purines are adenine (A) and guanine (G), and pyrimidines are cytosine (C) and thymine (T) or, in the context of RNA, uracil (U). The C-1 atom of deoxyribose is bound to the N-1 of pyrimidines or N-9 of purines. Nucleotides are also phosphates of nucleosides, where esterification occurs on the hydroxyl groups attached to the C-3 or C-5 of the sugar. Nucleotides are typically monophosphates, diphosphates, or triphosphates.

A "nucleoside" is structurally similar to a nucleotide but lacks the phosphate moiety. An example of a nucleoside analog will be one where a label is attached to the base and there is no phosphate group attached to the sugar molecule.

Although the bases are commonly referred to as purines or pyrimidines, the skilled artisan will understand that derivatives and analogs are available that do not alter the ability of the nucleotide or nucleoside to undergo Watson-Crick base pairing. "Derivatives" or "analogs" mean compounds or molecules whose core structure is the same or very similar to that of the parent compound but which have chemical or physical modifications, such as different or additional side groups, which allow the derived nucleotides or nucleosides to be linked to another molecule. For example, the base can be a deazapurine. Derivatives should be capable of undergoing Watson-Crick pairing. "Derivatives" and "analogs" also mean synthetic nucleotide or nucleoside derivatives having modified base portions and/or modified sugar portions. Such derivatives and analogs are discussed in, for example, Scheit, Nucleotide analogs (John Wiley & Son, 1980) and Uhlman et al., Chemical Reviews 90:543-584, 1990. Nucleotide analogs can also contain modified phosphodiester bonds, including phosphorothioate bonds, dithiophosphorate bonds, alkyl phosphonate bonds, phosphoranilidate bonds, phosphoramidate bonds, and the like.

The dye can be attached via a linker to any position on the nucleoside base, provided that Watson-Crick base pairing can still occur. Specific nucleobase labeling sites include the C5 position of pyrimidine bases or the C7 position of 7-deazapurine bases. As described above, linker groups can be used to covalently attach the dye to nucleosides or nucleotides.

In certain embodiments, labeled nucleosides or nucleotides can be enzymatically incorporable and enzymatically extendable. Thus, the linker moiety can have sufficient length to connect the nucleotide to the compound such that the compound does not significantly interfere with the overall binding and recognition of the nucleotide by nucleic acid replicases. Therefore, the linker can also contain spacer units. For example, spacers keep the nucleoside base away from the cleavage site or label.

Nucleosides or nucleotides labeled with the dye compounds of the present invention can have the following structure: 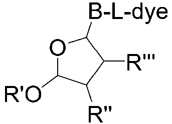 wherein the dye is a dye compound, B is a nucleobase such as uracil, thymine, cytosine, adenine, guanine, and the like, and L is an optional linker group that may or may not be present. R' can be H, monophosphate, diphosphate, triphosphate, phosphorothioate, phosphate analog, -O-attached to a reactive phosphorus-containing group, or -O- protected by a blocking group. R" can be H, OH, phosphoramidite, or a 3'-OH blocking group, and R‴ is H or OH; wherein R" is phosphoramidite, and R is an acid-cleavable hydroxyl protecting group, which allows subsequent monomer coupling under automatic synthesis conditions.

The present application also relates to polynucleotides incorporating the dye compounds of the present invention. Such polynucleotides may be DNA or RNA, which respectively comprise deoxyribonucleotides or ribonucleotides linked by phosphodiester bonds. The polynucleotides may comprise naturally occurring nucleotides, non-naturally occurring (or modified) nucleotides other than the labeled nucleotides described herein, or any combination thereof, provided that there is at least one nucleotide labeled with a dye compound according to the present application. Polynucleotides may also include non-natural backbone linkages and/or non-nucleotide chemical modifications. Chimeric structures comprising a mixture of ribonucleotides and deoxyribonucleotides that contain at least one labeled nucleotide are also contemplated.

In certain embodiments, the labeled nucleotides described in the present invention have a structure represented by formula (1): 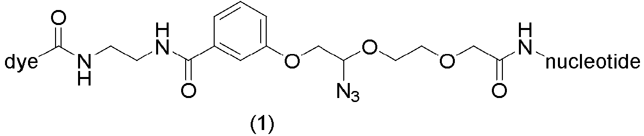 wherein, the dye is the dye compound mentioned above, including the compound represented by formula (I) or formula (I'), or a salt thereof. In certain embodiments, the dye is the compound represented by formula (I'), which is connected to the rest moiety ( 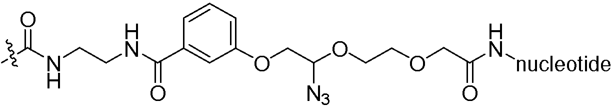 ) via R^{e} or R^{f}.

In certain embodiments, the labeled nucleotide represented by formula (1) has the following structure: 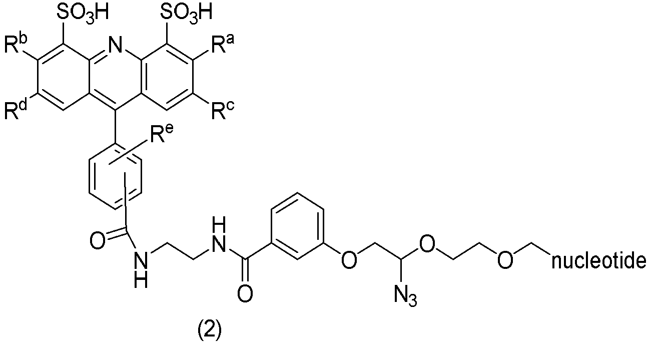 wherein, R^{a}, R^{b}, R^{c}, R^{d} and R^{e} are as defined above.

In certain embodiments, the labeled nucleotide represented by formula (1) has the following structure: 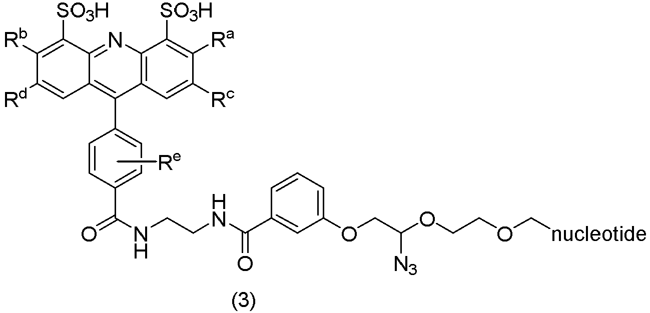 wherein, R^{a}, R^{b}, R^{c}, R^{d} and R^{e} are as defined above.

In certain embodiments, the labeled nucleotide represented by formula (1) has the following structure: 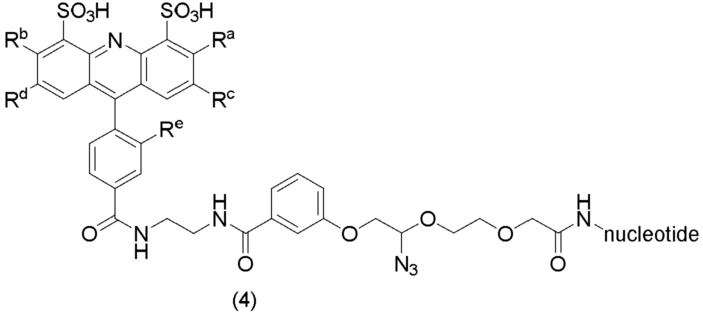 wherein, R^{a}, R^{b}, R^{c}, R^{d} and R^{e} are as defined above.

The nucleotides in the above formulas may be selected from dATP, dGTP, dCTP, and dTTP. Exemplary labeled dATP includes, but is not limited to, MGI471-V1-dATP

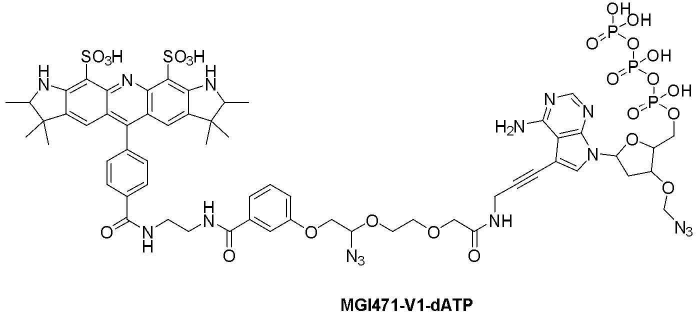

It also includes labeled dATP obtained by replacing MGI471 with another dye compound of the present invention.

Exemplary labeled dGTP includes, but is not limited to, MGI471-V1-dGTP

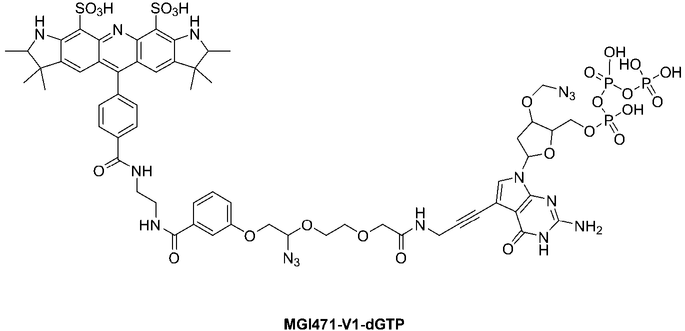

It also includes labeled dGTP obtained by replacing MGI471 with another dye compound of the present invention.

Exemplary labeled dCTP includes, but is not limited to, MGI471-V1-dCTP

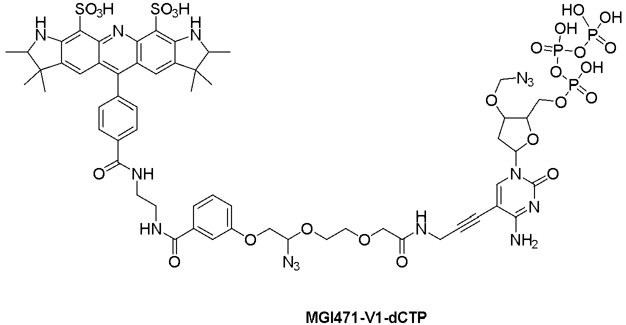

It also includes labeled dCTP obtained by replacing MGI471 with another dye compound of the present invention.

Exemplary labeled dTTP includes, but is not limited to, MGI471-V1-dTTP

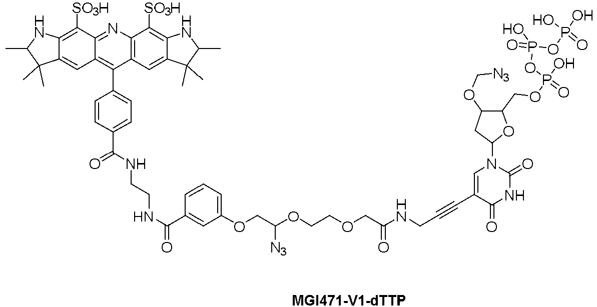

It also includes labeled dTTP obtained by replacing MGI471 with another dye compound of the present invention.

The labeled nucleotides of the present invention also include: 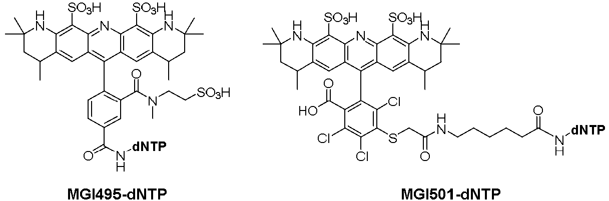 wherein, dNTP is selected from dATP, dGTP, dCTP and dTTP, with the following exemplary structures:

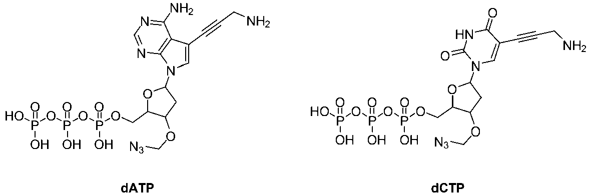

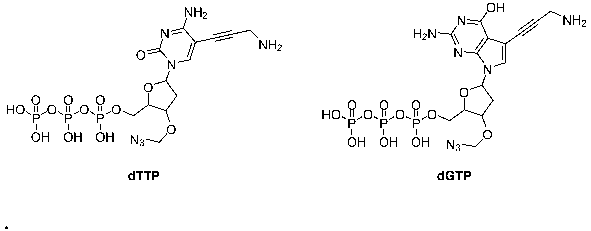

### Sequencing methods

Nucleotides (or nucleosides) containing the fluorescent dyes of the present invention can be used in any analytical method that requires the detection of fluorescent labels attached to nucleotides or nucleosides, whether by themselves, or incorporated into larger molecular structures or conjugates, or associated with larger molecular structures or conjugates. Certain embodiments of the present application relate to sequencing methods, which comprises (a) incorporating at least one labeled nucleotide as described herein into a polynucleotide; and (b) detecting the labeled nucleotide incorporated into the polynucleotide by detecting the fluorescent signal from the novel fluorescent dye attached to the modified nucleotide.

In certain embodiments, during the synthesis step, at least one labeled nucleotide is incorporated into the polynucleotide by the action of a polymerase. However, other methods of incorporating labeled nucleotides into polynucleotides are not excluded, such as chemical oligonucleotide synthesis or ligation of labeled oligonucleotides to unlabeled oligonucleotides. Thus, the term "incorporating" nucleotides into a polynucleotide encompasses polynucleotide synthesis by both chemical and enzymatic methods.

In specific non-limiting embodiments, modified nucleotides or nucleosides labeled with the fluorescent dyes according to the present invention can be used in methods for nucleic acid sequencing, resequencing, whole genome sequencing, single nucleotide polymorphism score, any other application involving the detection of modified nucleotides or nucleosides upon their incorporation into polynucleotides, or any other application that requires the use of polynucleotides labeled with modified nucleotides containing the fluorescent dyes of the present invention.

In certain embodiments, the present application provides the use of modified nucleotides containing the dye compounds of the present invention in "sequencing by synthesis" reactions of polynucleotides. Sequencing by synthesis typically involves the sequential addition of one or more nucleotides or oligonucleotides to a growing polynucleotide chain in the 5' to 3' direction using a polymerase or ligase, so as to form an extended polynucleotide chain complementary to the template nucleic acid to be sequenced. The identity of the base(s) present in one or more of the added nucleotides is determined in a detection step or "imaging" step. The identity of the added base can be determined after each nucleotide incorporation step. The sequence of the template can then be inferred using conventional Watson-Crick base pairing rules. The use of modified nucleotides labeled with dyes according to the present disclosure for determining the identity of a single base can be useful, such as in single nucleotide polymorphism score, and such single-base extension reactions are within the scope of the present application.

In an embodiment, the sequence of a template polynucleotide is determined through the detection of the incorporation of one or more nucleotides into a nascent strand complementary to the template polynucleotide to be sequenced, by detecting the fluorescent labels attached to the incorporated nucleotides. The nucleic acid template to be sequenced can be DNA or RNA, or even a hybrid molecule containing both deoxynucleotides and ribonucleotides. The nucleic acid template can comprise naturally occurring nucleotides and/or non-naturally occurring nucleotides, as well as natural or non-natural backbone linkages, provided that these do not prevent the replication of the template in the sequencing reaction.

While one application of the modified nucleotides of the present disclosure is in sequencing by synthesis reactions, the utility of such labeled nucleotides is not limited to such methods. In fact, the nucleotides can be advantageously used in any sequencing method that requires the detection of fluorescent labels attached to nucleotides incorporated into polynucleotides.

In certain embodiments, the present invention provides a method for determining the sequence of a target single-stranded polynucleotide, comprising the following steps:
(a) providing a duplex, nucleotides, a polymerase, and an excision reagent, wherein the duplex comprises a growing nucleic acid strand and the nucleic acid molecule to be sequenced;
(b) performing a reaction cycle comprising the following steps (i), (ii), and (iii):
   Step (i): using a polymerase to incorporate nucleotides into a growing nucleic acid strand, forming a nucleic acid intermediate comprising a blocking group and a detectable label;
   Step (ii): detecting the detectable label on the nucleic acid intermediate;
   Step (iii): using the excision reagent to remove the blocking group on the nucleic acid intermediate.

In certain embodiments, the reaction cycle further comprises Step (iv): using the excision reagent to remove the detectable label on the nucleic acid intermediate.

In the present invention, nucleic acids may include nucleotides or nucleotide analogs. A nucleotide typically comprises a sugar, a nucleobase, and at least one phosphate group. Nucleotides include deoxyribonucleotides, modified deoxyribonucleotides, ribonucleotides, modified ribonucleotides, peptide nucleotides, modified peptide nucleotides, modified phosphate-sugar backbone nucleotides, and mixtures thereof. Examples of nucleotides include, for instance, adenosine monophosphate (AMP), adenosine diphosphate (ADP), adenosine triphosphate (ATP), thymidine monophosphate (TMP), thymidine diphosphate (TDP), thymidine triphosphate (TTP), cytidine monophosphate (CMP), cytidine diphosphate (CDP), cytidine triphosphate (CTP), guanosine monophosphate (GMP), guanosine diphosphate (GDP), guanosine triphosphate (GTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxythymidine monophosphate (dTMP), deoxythymidine diphosphate (dTDP), deoxythymidine triphosphate (dTTP), deoxycytidine diphosphate (dCDP), deoxycytidine triphosphate (dCTP), deoxyguanosine monophosphate (dGMP), deoxyguanosine diphosphate (dGDP), deoxyguanosine triphosphate (dGTP), deoxyuridine monophosphate (dUMP), deoxyuridine diphosphate (dUDP), and deoxyuridine triphosphate (dUTP). Nucleotide analogs containing modified nucleobases can also be used in the methods described herein. Exemplary modified nucleobases that can be included in polynucleotides, whether with natural backbones or analogous structures, include, for example, inosine, xanthine, hypoxanthine, isocytosine, isoguanine, 2-aminopurine, 5-methylcytosine, 5-hydroxymethylcytosine, 2-aminoadenine, 6-methyladenine, 6-methylguanine, 2-propylguanine, 2-propyladenine, 2-thiouracil, 2-thiothymine, 2-thiocytosine, 15-halouracil, 15-halocytosine, 5-propynyluracil, 5-propynylcytosine, 6-azouracil, 6-azocytosine, 6-azothymine, 5-uracil, 4-thiouracil, 8-haloadenine or guanine, 8-aminoadenine or guanine, 8-thioadenine or guanine, 8-thioalkyladenine or guanine, 8-hydroxyadenine or guanine, 5-halogen-substituted uracil or cytosine, 7-methylguanine, 7-methyladenine, 8-azaguanine, 8-azaadenine, 7-deazaguanine, 7-deazaadenine, 3-deazaguanine, 3-deazaadenine, etc. As is known in the art, certain nucleotide analogs cannot be incorporated into polynucleotides, such as nucleotide analogs like adenosine 5'-phosphosulfate.

In the methods of the present invention, the nucleic acid molecule to be sequenced is not limited by its length. In certain preferred embodiments, the length of the nucleic acid molecule to be sequenced can be at least 10 bp, at least 20 bp, at least 30 bp, at least 40 bp, at least 50 bp, at least 100 bp, at least 200 bp, at least 300 bp, at least 400 bp, at least 500 bp, at least 1000 bp, or at least 2000 bp. In certain preferred embodiments, the length of the nucleic acid molecule to be sequenced can be 10-20 bp, 20-30 bp, 30-40 bp, 40-50 bp, 50-100 bp, 100-200 bp, 200-300 bp, 300-400 bp, 400-500 bp, 500-1000 bp, 1000-2000 bp, or more than 2000 bp. In certain preferred embodiments, the nucleic acid molecule to be sequenced can have a length of 10-1000 bp to facilitate high-throughput sequencing.

In certain preferred embodiments, the nucleic acid molecule may be pretreated before being immobilized on a support. Such pretreatment includes, but is not limited to, fragmentation of the nucleic acid molecule, end-blunting, adapter ligation, tag addition, amplification of the nucleic acid molecule, isolation and purification of the nucleic acid molecule, and any combination thereof.

In certain embodiments, the surface of the solid support may have reactive functional groups that react with complementary functional groups on the polynucleotide molecule to form covalent bonds, for example, in the same manner as the technique used to attach cDNA to microarrays, see, e.g., Smirnov et al. (2004), Genes, Chromosomes & Cancer, 40: 72-77 and Beaucage (2001), Current Medicinal Chemistry, 8: 1213-1244, both of which are incorporated herein by reference. DNB can also be effectively attached to hydrophobic surfaces, such as clean glass surfaces with low concentrations of various reactive functional groups (e.g., -OH groups). Attachment via covalent bonds formed between the polynucleotide molecule and reactive functional groups on the surface is also referred to herein as "chemical attachment."

In other embodiments, the polynucleotide molecule may be adsorbed onto the surface. In such embodiments, the polynucleotide is immobilized through non-specific interactions with the surface, or through non-covalent interactions such as hydrogen bonding, van der Waals forces, and the like.

In other embodiments, the nucleic acid library may be double-stranded nucleic acid fragments, which are immobilized on the surface of a solid support by ligation reaction with oligonucleotides immobilized on the surface of the solid support, followed by rolling circle amplification to prepare the sequencing library.

### Kit

In another aspect, the present application provides a kit comprising a nucleoside and/or a nucleotide labeled with a dye compound of the present invention. In certain embodiments, the kit comprises one or more nucleotides, wherein at least one nucleotide is a nucleotide labeled with a dye compound of the present invention. In certain embodiments, the kit may comprise two or more labeled nucleotides. The fluorescent dye compounds, labeled nucleotides, or kits of the present invention can be used in sequencing, expression analysis, hybridization analysis, genetic analysis, RNA analysis, or protein-binding assays. Such use can be performed on an automated sequencer. The sequencer may include two lasers operating at different wavelengths.

In cases where the kit comprises multiple nucleotides labeled with dye compounds, particularly two or four nucleotides, different nucleotides may be labeled with the same or different dye compounds, or one nucleotide may be unlabeled with any dye compound. When different nucleotides are labeled with the same or different dye compounds, the kit is characterized in that the nucleotides labeled with the dye compounds can be distinguished by fluorescence spectroscopy and algorithms. When two nucleotides labeled with fluorescent dye compounds are provided in the form of a kit, in certain embodiments, the spectrally distinguishable fluorescent dyes can be excited at the same wavelength (e.g., by the same laser). When four nucleotides labeled with fluorescent dye compounds are provided in the form of a kit, in certain embodiments, two of the spectrally distinguishable fluorescent dyes can both be excited at one wavelength, and the other two spectrally distinguishable dyes can both be excited at another wavelength.

The dye compounds of the present invention can be excited by blue light and have an emission wavelength falling in the cyan light region. Therefore, in certain embodiments, the kit of the present invention may comprise at least one fluorescent dye that can be excited by blue light (with an emission wavelength in the cyan light region), and in addition, two spectrally distinguishable dyes that are excited at another wavelength.

In the present invention, blue light refers to light with a wavelength in the range of approximately 450 nm to 480 nm, and cyan light refers to light with a wavelength in the range of approximately 480 nm to 490 nm. In certain embodiments, the kit of the present invention may further comprise: a reagent for immobilizing the nucleic acid molecule to be sequenced on a support (e.g., immobilization via covalent or non-covalent attachment); a primer for initiating the nucleotide polymerization reaction; a polymerase for performing the nucleotide polymerization reaction; one or more buffer solutions; one or more washing solutions; or any combination thereof.

In certain embodiments, the kit of the present invention may further comprise reagents and/or devices for extracting nucleic acid molecules from a sample. Methods for extracting nucleic acid molecules from samples are well-known in the art. Therefore, various reagents and/or devices for extracting nucleic acid molecules can be configured in the kit of the present invention as needed, such as reagents for cell lysis, reagents for DNA precipitation, reagents for DNA washing, reagents for DNA dissolution, reagents for RNA precipitation, reagents for RNA washing, reagents for RNA dissolution, reagents for protein removal, reagents for DNA removal (e.g., when the target nucleic acid molecule is RNA), reagents for RNA removal (e.g., when the target nucleic acid molecule is DNA), and any combination thereof.

In certain embodiments, the kit of the present invention further comprises a reagent for pretreating nucleic acid molecules. In the kit of the present invention, the reagent for pretreating nucleic acid molecules is not further limited and can be selected according to actual needs. The reagents for pretreating nucleic acid molecules include, for example, reagents for nucleic acid molecule fragmentation (e.g., DNase I), reagents for end-blunting of nucleic acid molecules (e.g., DNA polymerases such as T4 DNA polymerase, Pfu DNA polymerase, Klenow DNA polymerase), adapter molecules, tag molecules, reagents for ligating adapter molecules to target nucleic acid molecules (e.g., ligases such as T4 DNA ligase), reagents for nucleic acid end repair (e.g., DNA polymerases lacking 3'-5' exonuclease activity but exhibiting 5'-3' exonuclease activity), reagents for amplifying nucleic acid molecules (e.g., DNA polymerases, primers, dNTPs), reagents for isolating and purifying nucleic acid molecules (e.g., chromatography columns), and any combination thereof.

In certain embodiments, the kit of the present invention further comprises a support for immobilizing the nucleic acid molecule to be sequenced. Typically, the support for immobilizing the nucleic acid molecule to be sequenced is in a solid phase for ease of handling. Therefore, in the present disclosure, a "support" is sometimes also referred to as a "solid support" or "solid-phase support". However, it should be understood that the "support" mentioned herein is not limited to solids and may also be semi-solids (e.g., gels).

As used herein, the terms "loading", "immobilization", and "attachment" when referring to nucleic acids mean direct or indirect attachment to a solid support via covalent or non-covalent bonds. In certain embodiments of the present disclosure, the methods of the present invention include immobilizing nucleic acids on a solid support via covalent attachment. Generally, however, it is only required that the nucleic acid remains immobilized or attached to the solid support under the conditions in which the solid support is intended to be used (e.g., in applications requiring nucleic acid amplification and/or sequencing). In certain embodiments, immobilizing nucleic acids on a solid support may involve immobilizing oligonucleotides to be used as capture primers or amplification primers on the solid support, such that the 3' end is available for enzymatic extension and at least a portion of the primer sequence is capable of hybridizing to a complementary nucleic acid sequence; the nucleic acid to be immobilized is then hybridized to the oligonucleotide, in which case the immobilized oligonucleotide or polynucleotide may be in the 3'-5' direction. In certain embodiments, immobilizing nucleic acids on a solid support may involve attaching nucleic acid-binding proteins to the solid support via amination modification and capturing nucleic acid molecules via the nucleic acid-binding proteins. Alternatively, loading may occur via means other than base-pairing hybridization, such as the covalent attachment described above. Non-limiting examples of methods for attaching nucleic acids to a solid support include nucleic acid hybridization, biotin-streptavidin binding, thiol binding, photoactivated binding, covalent binding, antibody-antigen binding, physical confinement via hydrogels or other porous polymers, and the like. Various exemplary methods for immobilizing nucleic acids on solid supports can be found in, for example, G. Steinberg-Tatman et al., Bioconjugate Chemistry 2006, 17, 841-848; Xu X. et al., Journal of the American Chemical Society 128 (2006) 9286-9287; U.S. Patent Applications US 5639603, US 5641658, US 2010248991; International Patent Applications WO 2001062982, WO 2001012862, WO 2007111937, WO 0006770. For all purposes, and particularly for all teachings related to the preparation of solid supports with nucleic acids immobilized thereon, the above documents are incorporated herein by reference in their entireties.

In the present invention, the support can be made of various suitable materials. Such materials include, for example: inorganic materials, natural polymers, synthetic polymers, and any combination thereof. Specific examples include, but are not limited to: cellulose, cellulose derivatives (e.g., nitrocellulose), acrylic resins, glass, silica gel, silica, polystyrene, gelatin, polyvinylpyrrolidone, copolymers of vinyl and acrylamide, polystyrene cross-linked with divinylbenzene, etc. (see, e.g., Merrifield, Biochemistry 1964, 3, 1385-1390), polyacrylamide, latex, dextran, rubber, silicon, plastics, natural sponges, metal-plastics, cross-linked dextran (e.g., Sephadex^{™}), agarose gels (Sepharose^{™}), and other supports known to those skilled in the art.

In certain preferred embodiments, the support for immobilizing the nucleic acid molecule to be sequenced may be a solid support including an inert substrate or matrix (e.g., slides, polymer beads, etc.), which has been functionalized, for example, by applying an intermediate material containing reactive groups that allow covalent attachment of biomolecules such as polynucleotides. Examples of such supports include, but are not limited to, polyacrylamide hydrogels supported on an inert substrate such as glass, particularly the polyacrylamide hydrogels described in WO 2005/065814 and US 2008/0280773, the contents of which are incorporated herein by reference in their entireties. In such embodiments, biomolecules (e.g., polynucleotides) can be directly covalently attached to the intermediate material (e.g., hydrogel), which itself can be non-covalently attached to the substrate or matrix (e.g., glass substrate). In certain preferred embodiments, the support is a glass slide or silicon wafer whose surface is modified with a layer of chemical groups such as avidin, amino, acrylamide silane, or aldehyde.

In the present invention, the support or solid support is not limited by its size, shape, or structure. In some embodiments, the support or solid support is a planar structure, such as a slide, chip, microchip, and/or array. The surface of such a support may be in the form of a planar layer.

In certain preferred embodiments, the support for immobilizing the nucleic acid molecule to be sequenced is an array of beads or wells (also referred to as a chip). The array can be prepared using any of the materials outlined herein for preparing solid supports, and preferably, the surfaces of the beads or wells on the array are functionalized to facilitate the immobilization of nucleic acid molecules. The number of beads or wells on the array is not limited. For example, each array may contain 10-10², 10²-10³, 10³-10⁴, 10⁴-10⁵, 10⁵-10⁶, 10⁶-10⁷, 10⁷-10⁸, 10⁸-10⁹, 10¹⁰-10¹¹, 10¹¹-10¹², or more beads or wells. In certain exemplary embodiments, the surface of each bead or well may immobilize one or more nucleic acid molecules. Correspondingly, each array may immobilize 10-10², 10²-10³, 10³-10⁴, 10⁴-10⁵, 10⁵-10⁶, 10⁶-10⁷, 10⁷-10⁸, 10⁸-10⁹, 10¹⁰-10¹¹, 10¹¹-10¹², or more nucleic acid molecules. Therefore, such arrays can be particularly advantageously used for high-throughput sequencing of nucleic acid molecules.

In certain preferred embodiments, the kit of the present invention further comprises a reagent for immobilizing (e.g., immobilization via covalent or non-covalent attachment)a nucleic acid molecule to be sequenced on a support. Such reagents include, for example, reagents for activating or modifying nucleic acid molecules (e.g., at their 5' ends) such as phosphates, thiols, amines, carboxylic acids, or aldehydes; reagents for activating or modifying the surface of the support such as amino-alkoxysilanes (e.g., aminopropyltrimethoxysilane, aminopropyltriethoxysilane, 4-aminobutyltriethoxysilane, etc.); cross-linking agents such as succinic anhydride, phenyldiisothiocyanate (Guo et al., 1994), maleic anhydride (Yang et al., 1998), 1-ethyl-3-(3-dimethylaminopropyl)-carbodiimide hydrochloride (EDC), m-maleimidobenzoyl-N-hydroxysuccinimide ester (MBS), N-succinimidyl [4-iodoacetyl]aminobenzoic acid (SIAB), succinimidyl 4-(N-maleimidomethyl)cyclohexane-1-carboxylate (SMCC), N-γ-maleimidobutyryloxy-succinimide ester (GMBS), succinimidyl 4-(p-maleimidophenyl)butyrate (SMPB); and any combination thereof.

In certain preferred embodiments, the kit of the present invention further comprises a primer for initiating a nucleotide polymerization reaction. In the present invention, the primer is not further limited as long as it can specifically anneal to a region of the target nucleic acid molecule. In some exemplary embodiments, the length of the primer may be 5-50 bp, such as 5-10, 10-15, 15-20, 20-25, 25-30, 30-35, 35-40, 40-45, or 45-50 bp. In some exemplary embodiments, the primer may comprise naturally occurring or non-naturally occurring nucleotides. In some exemplary embodiments, the primer comprises or consists of naturally occurring nucleotides. In some exemplary embodiments, the primer comprises modified nucleotides such as locked nucleic acids (LNAs). In certain preferred embodiments, the primer comprises a universal primer sequence.

In certain preferred embodiments, the kit of the present invention further comprises a polymerase for performing a nucleotide polymerization reaction. In the present invention, various suitable polymerases can be used for the polymerization reaction. In some exemplary embodiments, the polymerase (e.g., a DNA polymerase) is capable of synthesizing a new DNA strand using DNA as a template. In some exemplary embodiments, the polymerase (e.g., a reverse transcriptase) is capable of synthesizing a new DNA strand using RNA as a template. In some exemplary embodiments, the polymerase (e.g., an RNA polymerase) is capable of synthesizing a new RNA strand using DNA or RNA as a template. Therefore, in certain preferred embodiments, the polymerase is selected from DNA polymerases, RNA polymerases, and reverse transcriptases.

In certain preferred embodiments, the kit of the present invention further comprises one or more excision reagents. In certain embodiments, the excision reagent is selected from endonuclease IV and alkaline phosphatase.

In certain preferred embodiments, the kit of the present invention further comprises one or more buffer solutions. Such buffers include, but are not limited to, buffers for DNase I, buffers for DNA polymerases, buffers for ligases, buffers for eluting nucleic acid molecules, buffers for dissolving nucleic acid molecules, buffers for performing nucleotide polymerization reactions (e.g., PCR), and buffers for performing ligation reactions. The kit of the present invention may comprise any one or more of the above buffer solutions.

In certain embodiments, the buffer for DNA polymerase comprises monovalent salt ions (e.g., sodium ions, chloride ions) and/or divalent salt ions (e.g., magnesium ions, sulfate ions, manganese ions). In certain embodiments, the concentration of the monovalent or divalent salt ions in the buffer solution is 10 µM - 200 mM, such as 10 µM, 50 µM, 100 µM, 200 µM, 500 µM, 1 mM, 3 mM, 10 mM, 20 mM, 50 mM, 100 mM, 150 mM, or 200 mM.

In certain embodiments, the buffer for DNA polymerase comprises tris(hydroxymethyl)aminomethane (Tris). In certain embodiments, the concentration of Tris in the buffer solution is 10 mM - 200 mM, such as 10 mM, 20 mM, 50 mM, 100 mM, 150 mM, or 200 mM.

In certain embodiments, the buffer for DNA polymerase comprises an organic solvent such as DMSO or glycerol. In certain embodiments, the mass content of the organic solvent in the buffer solution is 0.01% - 10%, such as 0.01%, 0.02%, 0.05%, 1%, 2%, 5%, or 10%.

In certain embodiments, the pH of the buffer for DNA polymerase is 7.0 - 9.0, such as 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, or 9.0.

In certain embodiments, the buffer for DNA polymerase comprises: monovalent salt ions (e.g., sodium ions, chloride ions), divalent salt ions (e.g., magnesium ions, sulfate ions, manganese ions), Tris, and an organic solvent (e.g., DMSO or glycerol). In certain embodiments, the pH of the buffer solution is 8.8.

In certain preferred embodiments, the kit of the present invention further comprises one or more washing solutions. Examples of such washing solutions include, but are not limited to, phosphate buffer, citrate buffer, Tris-HCl buffer, acetate buffer, carbonate buffer, and the like. The kit of the present invention may comprise any one or more of the above washing solutions.

In another aspect, the present application provides the use of the fluorescent dye, labeled nucleotide or oligonucleotide, or kit of the present invention for determining the sequence of a target polynucleotide.

The present invention also provides the use of the fluorescent dye in the fields of sequencing, expression analysis, hybridization analysis, genetic analysis, RNA analysis, protein-binding assays, in vitro diagnostics, immunoassays, and molecular labeling. In certain embodiments, the molecular labeling is used for cell imaging, tissue imaging, or in vivo biological imaging.

The present invention further provides the use of the fluorescent dye in the fluorescent labeling, quantification, or detection of proteins, enzymes, or nucleic acids.

### Method for preparing the dye compound

The present application also provides a method for preparing the dye compound of the present invention, which comprises: subjecting a xanthene structure to a substitution reaction in the presence of ammonia and methanol to convert it into an acridine structure.

The reaction route for synthesizing the compound of formula (I) is as follows:

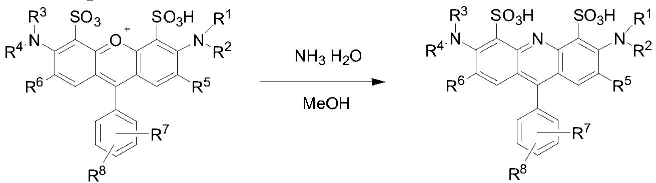

R¹-R⁷ are as defined above.

The reaction route for synthesizing the compound of formula (I') is as follows:

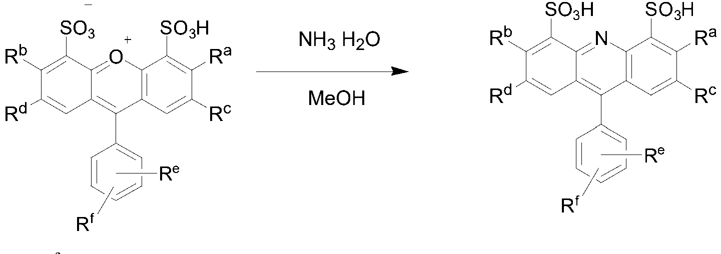

R^{a}-R^{f} are as defined above.

The method can be carried out at room temperature or under heating (such as 50-60°C).

### Methods for preparing dye-labeled nucleotides

The present application also provides methods for preparing the labeled nucleotides of the present invention, which can be carried out according to any one of Method A, Method B, or Method C.

### Method A:

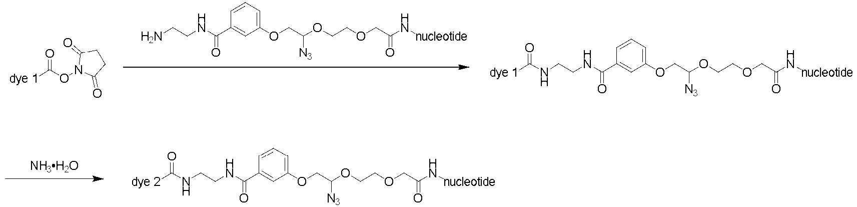

wherein, dye 2 is a dye compound of the present invention and has an acridine structure, while dye 1 is the precursor of dye 2 and has a xanthene structure.

### Method B:

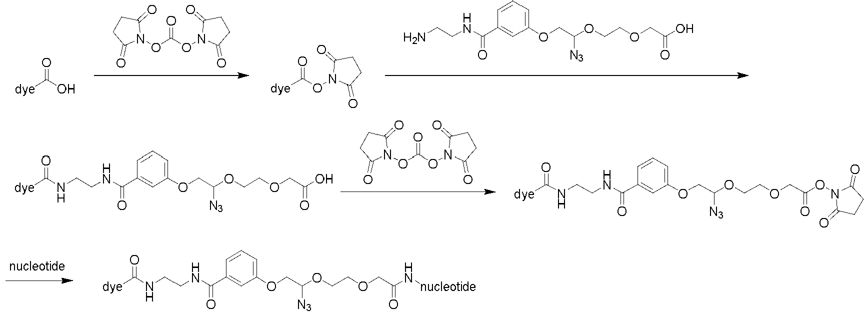

wherein, the dye is a dye compound of the present invention.

### Method C:

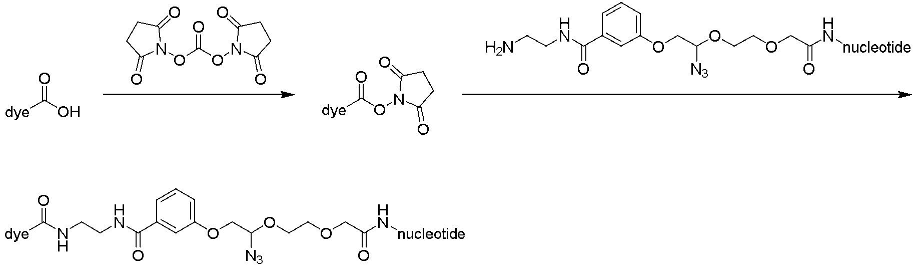

wherein, the dye is a dye compound of the present invention.

### Method D:

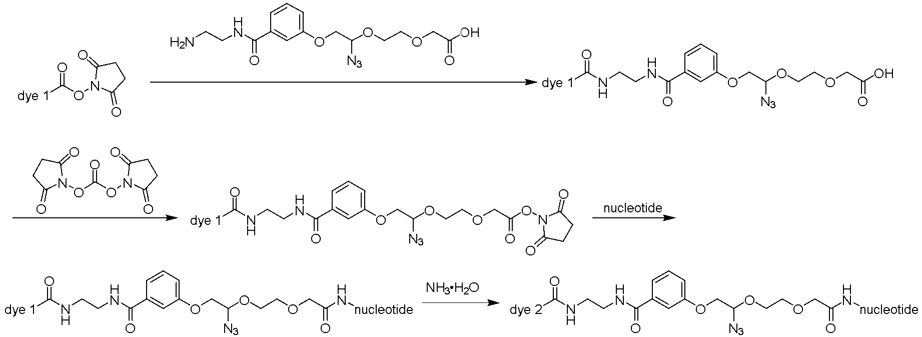

wherein, dye 2 is a dye compound of the present invention and has an acridine structure, while dye 1 is the precursor of dye 2 and has a xanthene structure.

In the above methods, the nucleotide may have a structure selected from the following:

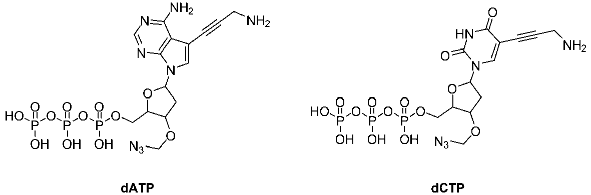

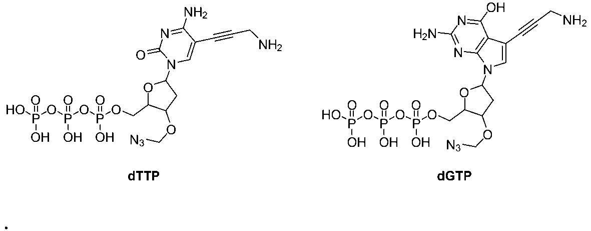

### Advantageous effects

The present invention uses substituted xanthenes as raw materials to synthesize acridine dyes under ammonia/methanol reaction conditions. The corresponding maximum excitation wavelength and emission wavelength of the synthesized dyes undergo a blue shift; under the excitation of blue light, their emission wavelength falls in the cyan light region.

The present invention develops new dyes in the blue light region or cyan light region and uses them for labeling bases, which helps improve sequencing accuracy in the field of gene sequencing.

The preparation method of the dye of the present invention has the advantages of simple steps and mild conditions.

The embodiments of the present invention will be described in detail below with reference to the accompanying figures and examples. However, those skilled in the art will understand that the following figures and examples are only used to illustrate the present invention and are not intended to limit the scope of the present invention. Various objects and advantageous aspects of the present invention will become apparent to those skilled in the art from the following detailed description of the figures and preferred embodiments.

### Brief description of the figures

Figure 1 is ¹H NMR spectrum of MGI450b.
Figure 2A is ¹H NMR spectrum of MGI443.
Figure 2B is F NMR spectrum of MGI443.
Figure 3 is ¹H NMR spectrum of MGI447.
Figure 4 is ¹H NMR spectrum of MGI477.
Figure 5 is ¹H NMR spectrum of MGI456.
Figure 6 is ¹H NMR spectrum of MGI485.
Figure 7A is ³¹P NMR spectrum of MGI501-V1-dATP.
Figure 7B is ³¹P NMR spectrum of MGI501-V1-dCTP.
Figure 8A is ³¹P NMR spectrum of MGI495-V1-dATP.
Figure 8B is ³¹P NMR spectrum of MGI495-V1-dCTP.
Figure 9A is ³¹P NMR spectrum of MGI450c-V1-dATP.
Figure 9B is ³¹P NMR spectrum of MGI450c-V1-dCTP.
Figure 9C is ³¹P NMR spectrum of MGI450c-V 1-dGTP.
Figure 10A is ³¹P NMR spectrum of MGI450b-V1-dATP.
Figure 10B is ³¹P NMR spectrum of MGI450b-V1-dTTP.
Figure 10C is ³¹P NMR spectrum of MGI450b-V1-dGTP.
Figure 11A is ³¹P NMR spectrum of MGI443-V1-dATP.
Figure 11B is ³¹P NMR spectrum of MGI443-V1-dCTP.
Figure 11C is ³¹P NMR spectrum of MGI443-V1-dTTP.
Figure 11D is ³¹P NMR spectrum of MGI443-V1-dGTP.
Figure 12A is ³¹P NMR spectrum of MGI488-V1-dATP.
Figure 12B is ³¹P NMR spectrum of MGI488-V1-dCTP.
Figure 13A is ³¹P NMR spectrum of MGI447-V 1-dATP.
Figure 13B is ³¹P NMR spectrum of MGI447-V1-dCTP.
Figure 13C is ³¹P NMR spectrum of MGI447-V1-dTTP.
Figure 13D is ³¹P NMR spectrum of MGI447-V1-dGTP.
Figure 14A is ¹⁹F NMR spectrum of MGI456-V1-dATP.
Figure 14B is ³¹P NMR spectrum of MGI456-V1-dATP.
Figure 14C is ¹⁹F NMR spectrum of MGI456-V1-dCTP.
Figure 14D is ³¹P NMR spectrum of MGI456-V1-dCTP.
Figure 14E is ¹⁹F NMR spectrum of MGI456-V1-dTTP.
Figure 14F is ¹⁹F NMR spectrum of MGI456-V1-dGTP.
Figure 14G is ³¹P NMR spectrum of MGI456-V1-dGTP.
Figure 15A is ³¹P NMR spectrum of MGI485-V1-dATP.
Figure 15B is ³¹P NMR spectrum of MGI485-V1-dCTP.
Figure 16 is ³¹P NMR spectrum of MGI450d-V1-dATP.
Figure 17A is ¹H NMR spectrum of MGI471-V1-dATP.
Figure 17B is ³¹P NMR spectrum of MGI471-V1-dATP.
Figure 17C is ³¹P NMR spectrum of MGI471-V1-dCTP.
Figure 17D is ³¹P NMR spectrum of MGI471-V1-dTTP.
Figure 17E is ³¹P NMR spectrum of MGI471-V1-dGTP.
Figure 18A is excitation and emission spectra of MGI471-V1.
Figure 18B is excitation and emission spectra of MGI471.
Figure 18C is excitation and emission spectra of MGI471-V1-dATP.
Figure 18D is excitation and emission spectra of MGI471-V1-dGTP.
Figure 18E is excitation and emission spectra of MGI471-V1-dCTP.
Figure 18F is excitation and emission spectra of MGI471-V1-dTTP.
Figure 18G is excitation and emission spectra of MGI495-V1-dATP.
Figure 18H is excitation and emission spectra of MGI495-V1-dCTP.
Figure 18I is excitation and emission spectra of MGI501-V1-dATP.
Figure 18J is excitation and emission spectra of MGI501-V1-dCTP.
Figure 18K is excitation and emission spectra of MGI447.
Figure 18L is excitation and emission spectra of MGI450b.
Figure 18M is excitation and emission spectra of MGI456.
Figure 18N is excitation and emission spectra of MGI477.
Figure 18O is excitation and emission spectra of MGI485.
Figure 18P is excitation and emission spectra of MGI450d.
Figure 18Q is excitation and emission spectra of MGI450d-V1-dATP.
Figure 18R is excitation and emission spectra of MGI450d-V1-dCTP.
Figure 18S is excitation and emission spectra of MGI450d-V1-dTTP.
Figure 18T is excitation and emission spectra of MGI485-V1-dATP.
Figure 18U is excitation and emission spectra of MGI485-V1-dCTP.
Figure 18V is excitation and emission spectra of MGI447-V1-dATP.
Figure 18W is excitation and emission spectra of MGI447-V1-dCTP.
Figure 18X is excitation and emission spectra of MGI447-V1-dTTP.
Figure 18Y is excitation and emission spectra of MGI447-V1-dGTP.
Figure 18Z is excitation and emission spectra of MGI453.

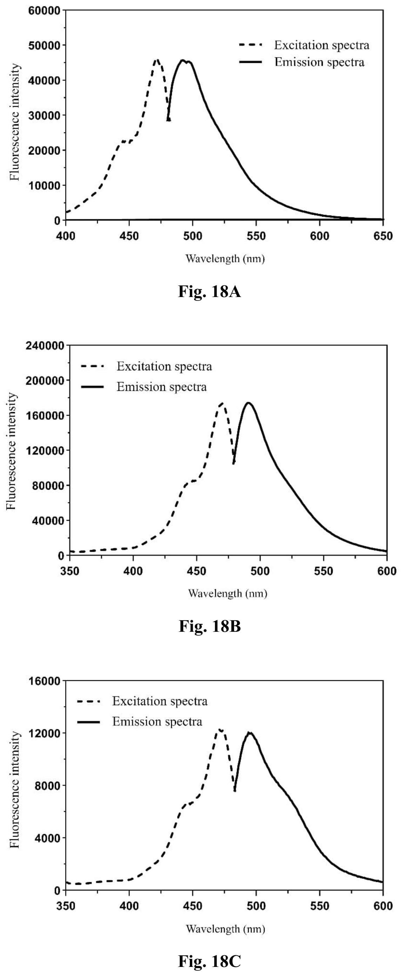

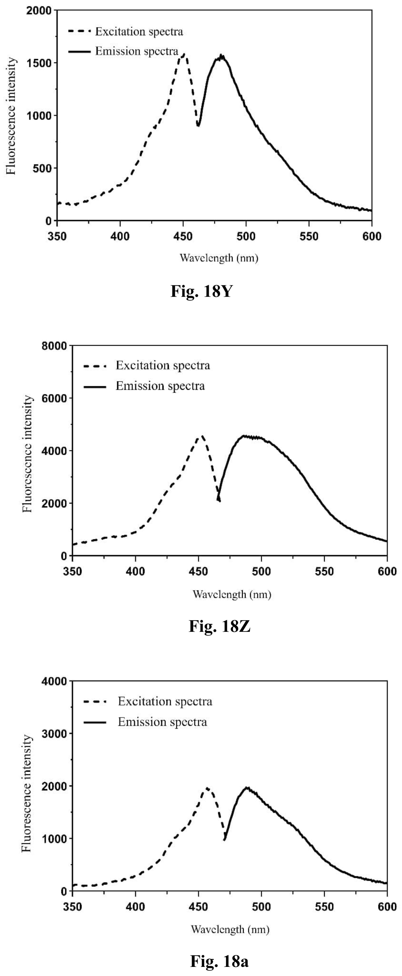

Figure 18a is excitation and emission spectra of MGI456-V1-dATP.

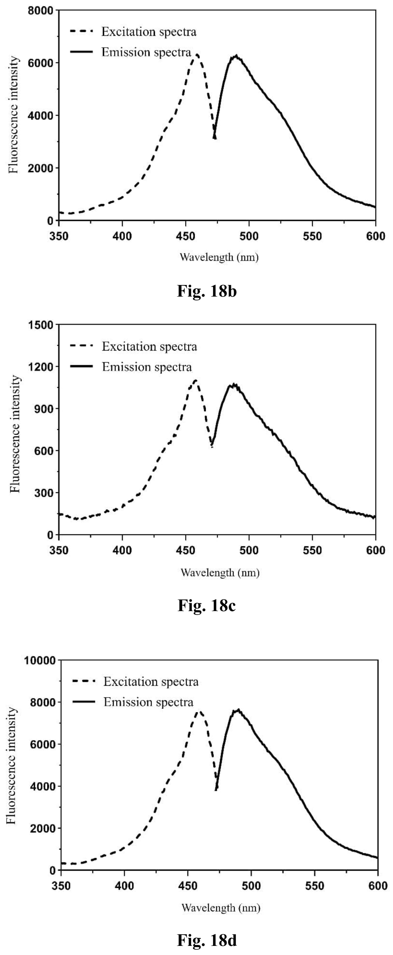

Figure 18b is excitation and emission spectra of MGI456-V1-dCTP.
Figure 18c is excitation and emission spectra of MGI456-V1-dGTP.

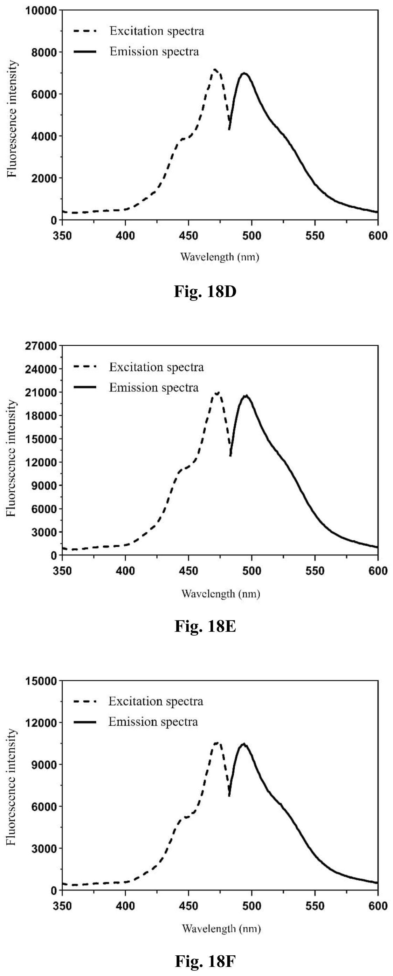

Figure 18d is excitation and emission spectra of MGI456-V1-dTTP.

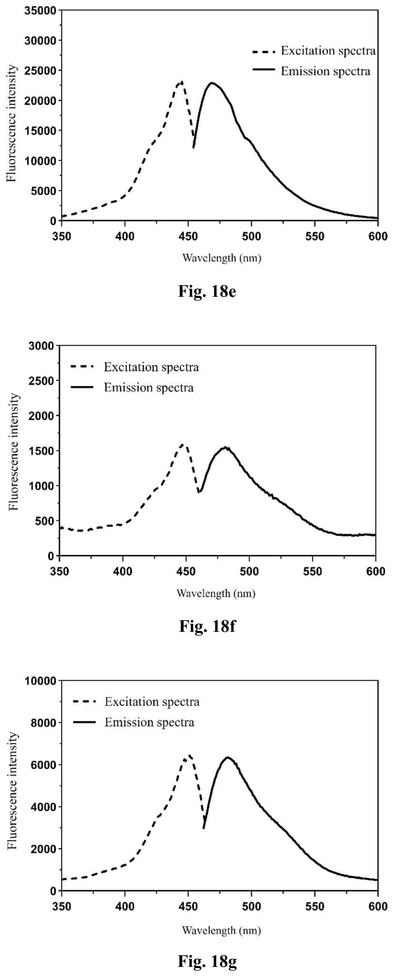

Figure 18e is excitation and emission spectra of MGI450a.
Figure 18f is excitation and emission spectra of MGI450b-V1-dATP.

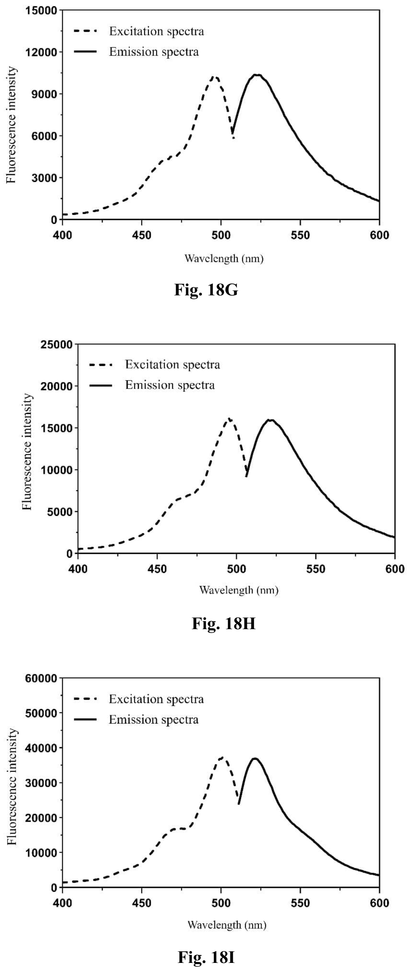

Figure 18g is excitation and emission spectra of MGI450b-V1-dCTP.

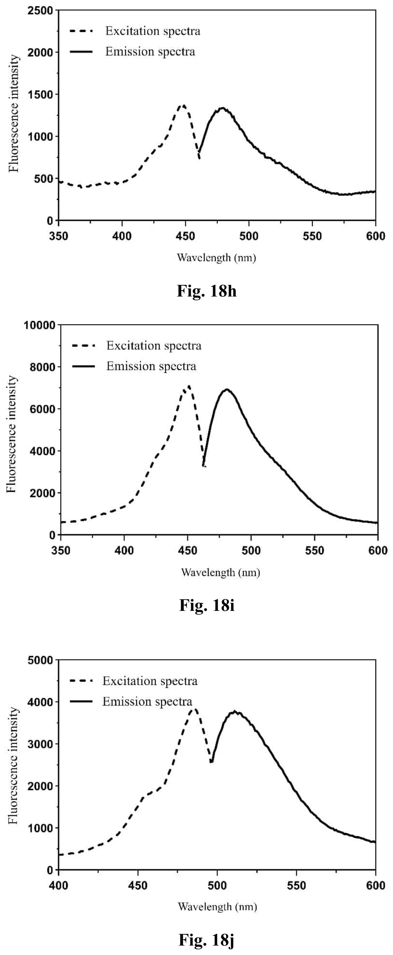

Figure 18h is excitation and emission spectra of MGI450b-V1-dGTP.
Figure 18i is excitation and emission spectra of MGI450b-V1-dTTP.

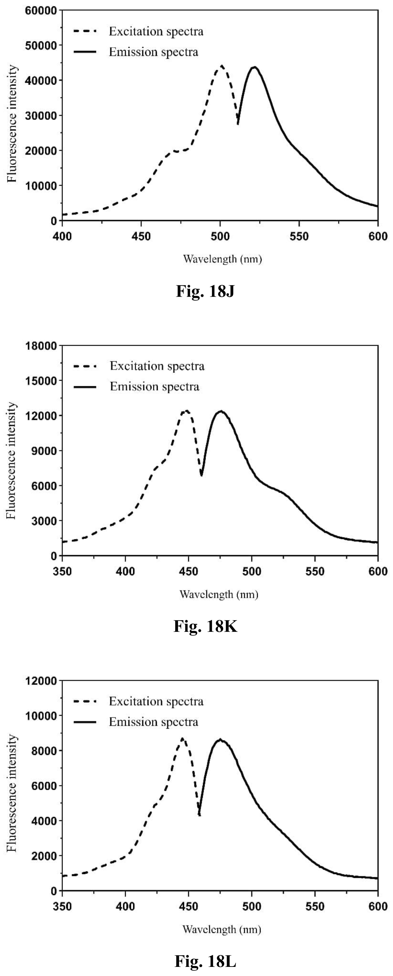

Figure 18j is excitation and emission spectra of MGI488-V1-dATP.

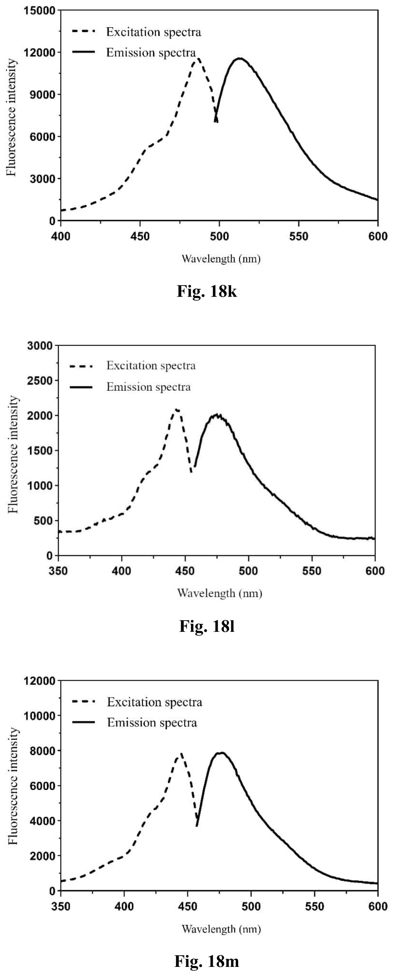

Figure 18k is excitation and emission spectra of MGI488-V1-dCTP.
Figure 18l is excitation and emission spectra of MGI443-V1-dATP.

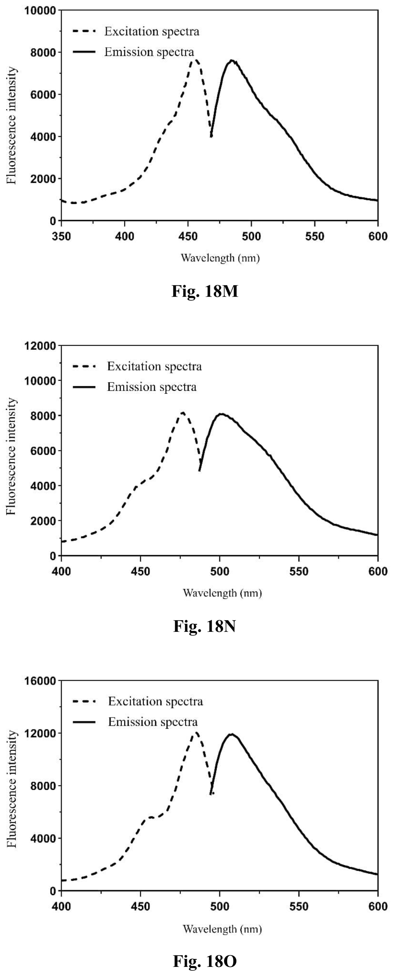

Figure 18m is excitation and emission spectra of MGI443-V1-dCTP.

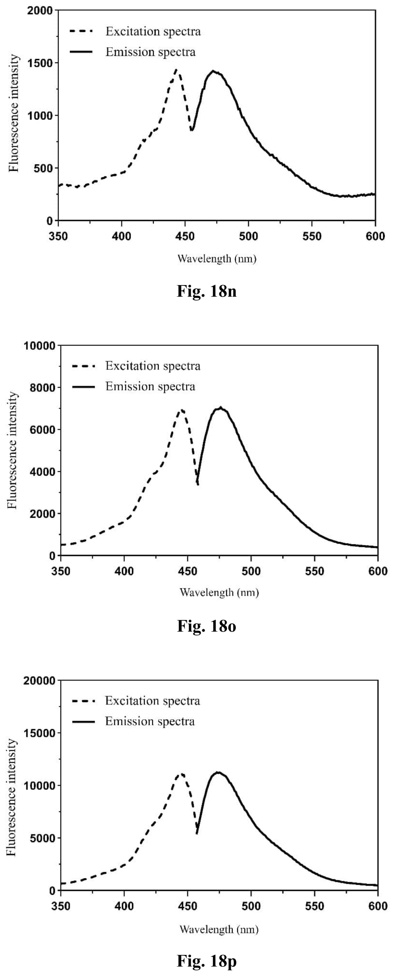

Figure 18n is excitation and emission spectra of MGI443-V1-dGTP.
Figure 18o is excitation and emission spectra of MGI443-V1-dTTP.

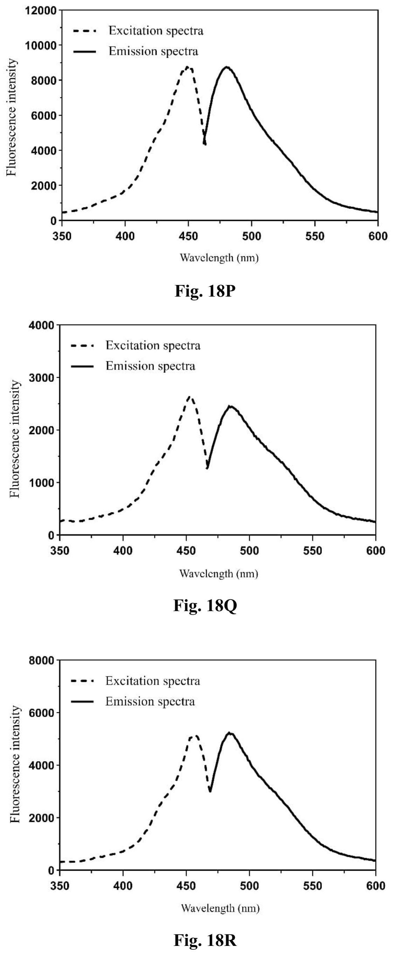

Figure 18p is excitation and emission spectra of MGI450c.

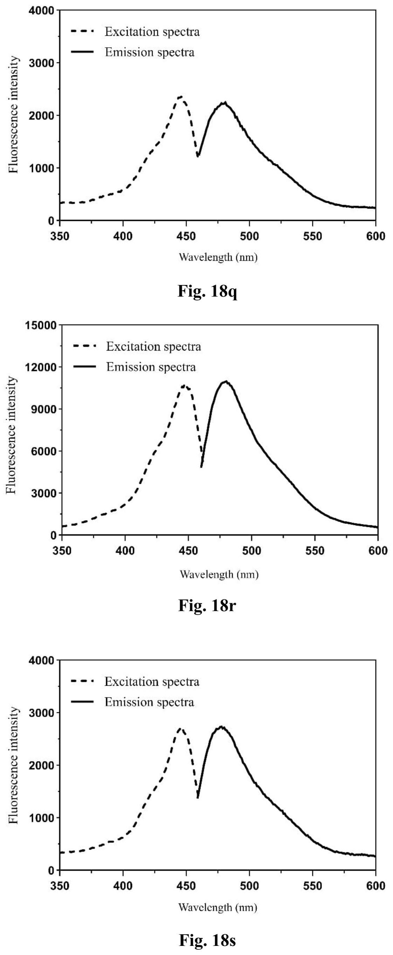

Figure 18q is excitation and emission spectra of MGI450c-V1-dATP.
Figure 18r is excitation and emission spectra of MGI450c-V1-dCTP.

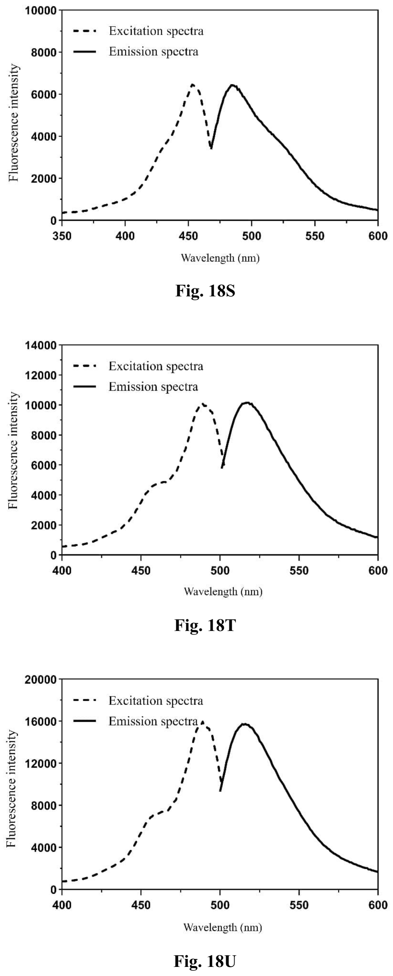

Figure 18s is excitation and emission spectra of MGI450c-V1-dGTP.

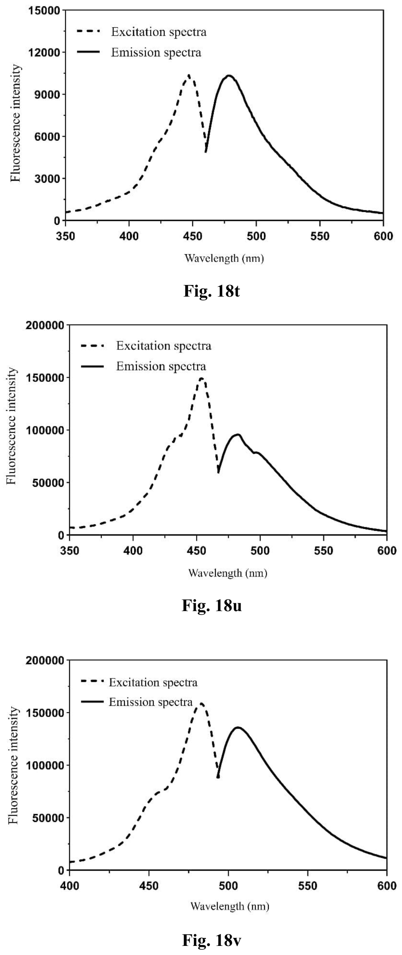

Figure 18t is excitation and emission spectra of MGI450c-V1-dTTP.
Figure 18u is excitation and emission spectra of MGI455.

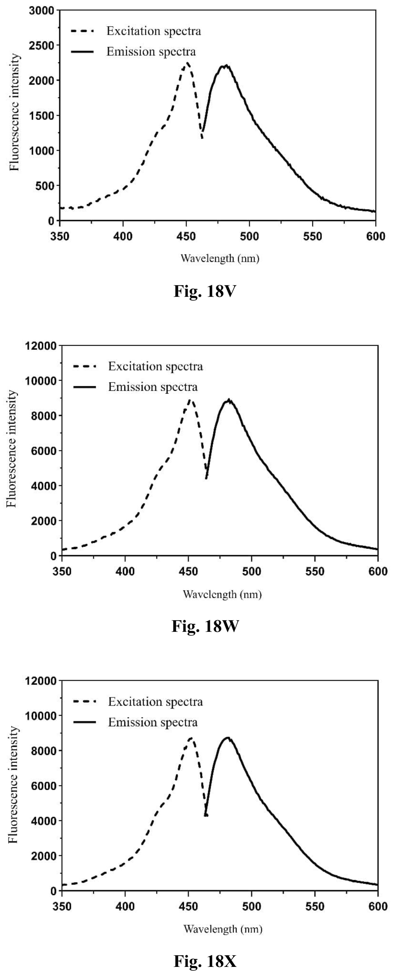

Figure 18v is excitation and emission spectra of MGI488.

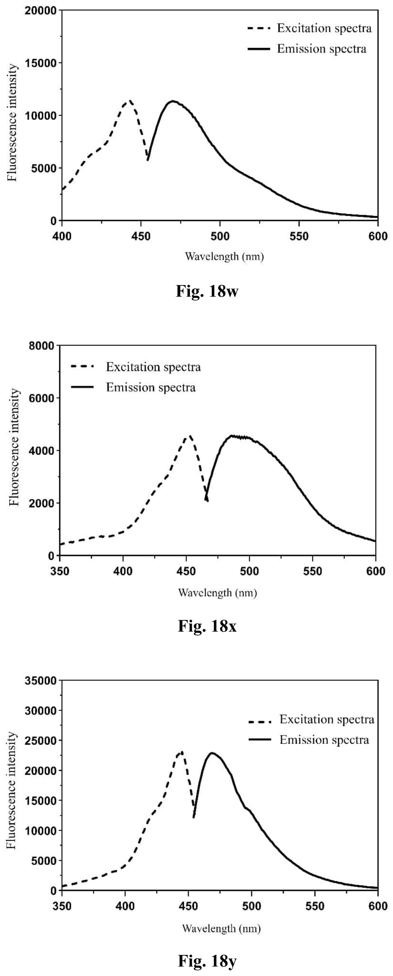

Figure 18w is excitation and emission spectra of MGI443.
Figure 18x is excitation and emission spectra of MGI453.
Figure 18y is excitation and emission spectra of MGI450a.

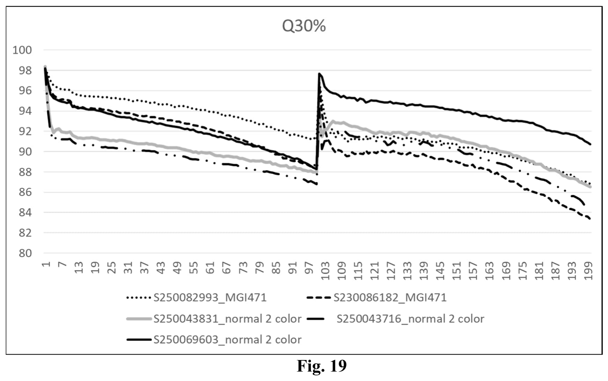

Figure 19 is Q30 data of MGI471 dNTP on a sequencer.

### Detailed description of specific embodiments

The embodiments of the present invention will be described in detail below with reference to examples. However, those skilled in the art will understand that the following examples are only used to illustrate the present invention and should not be regarded as limiting the scope of the present invention. For those not specified in the examples, the experiments are conducted under conventional conditions or conditions recommended by the manufacturer. The reagents or instruments without indicated manufacturers are conventional products available on the market.

As used herein, common organic abbreviations are defined as follows:
Boc: tert-Butoxycarbonyl
°C: Degree Celsius
dATP: Deoxyadenosine Triphosphate
dTTP: Deoxythymidine Triphosphate
dCTP: Deoxycytidine Triphosphate
dGTP: Deoxyguanosine Triphosphate
DIPEA: N,N-Diisopropylethylamine
DMAP: 4-Dimethylaminopyridine
DMF: N,N-Dimethylformamide
DMSO-d₆: Deuterated Dimethyl Sulfoxide
D₂O: Deuterated Water
DSC: N,N'-Disuccinimidyl Carbonate
FA: Formic Acid
g: Gram
LCMS: Liquid Chromatography-Mass Spectrometry
mg: Milligram
mL: Milliliter
MeOH: Methanol
m/z: Mass-to-Charge Ratio
NH₃·H₂O: Ammonia
TEAB: Triethylamine-Bicarbonate Buffer Solution
THF: Tetrahydrofuran

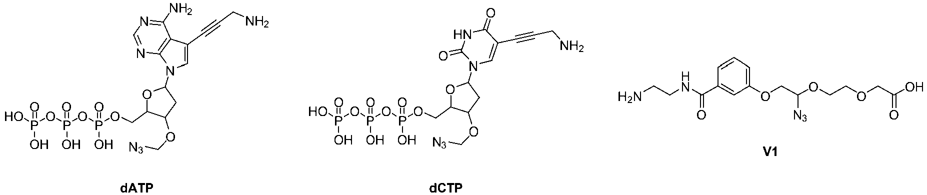

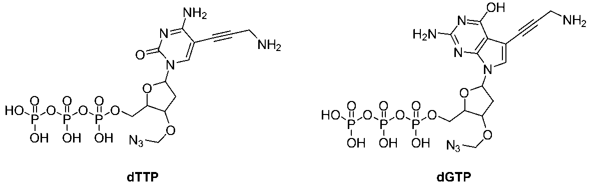

### Example 1 Synthesis of MGI453

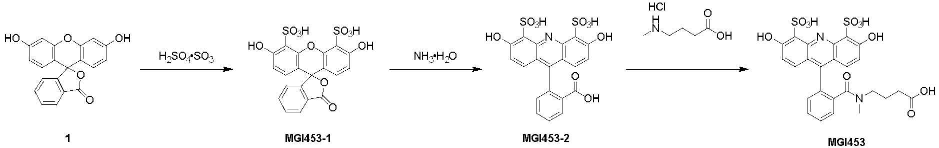

### (1) Synthesis of 3',6'-dihydroxy-3-carbonyl-3H-spiro[isobenzofuran-1,9'-xanthene]-4',5'-disulfonic acid (MGI453-1)

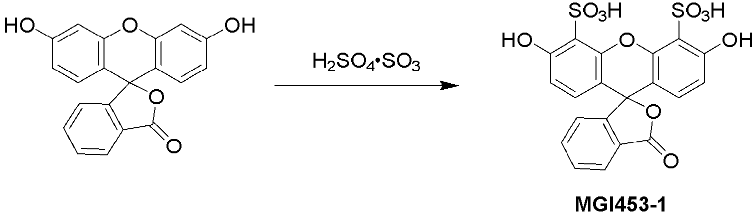

In a 100 mL flask, 30% fuming sulfuric acid (30 mL), and then fluorescein (3 g, 9.03 mmol) were added, heated up to 90°C, and stirred magnetically for 2 hours. The reaction solution was slowly added dropwise into crushed ice to quench the reaction, followed by filtering, the filtrate was purified by flash preparative liquid chromatography (water/acetonitrile), and the prepared solution was concentrated under reduced pressure to obtain MGI453-1. LCMS: calcd for C₂₀H₁₂O₁₁S₂ [M+H]⁺: 492.98. Found, m/z, [M+H]⁺: 493.10.

### (2) Synthesis of 2-(3,6-dihydroxy-4,5-disulfoacridin-9-yl)benzoic acid (MGI453-2)

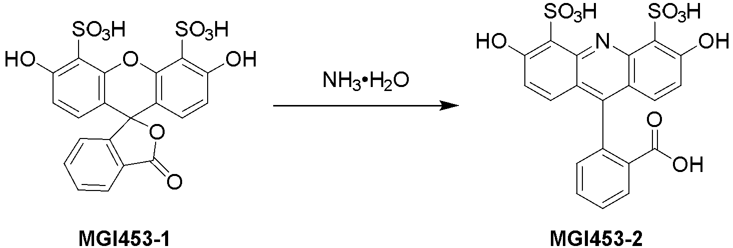

In a 100 mL flask, MGI453-1 (200 mg, 406.15 µmol) was dissolved with ammonia solution (25%-28%, 20 mL) and methanol (20 mL), heated up to 50°C, and stirred magnetically for 15 hours. The reaction solution was concentrated under reduced pressure to obtain a crude product, the crude product was purified by flash preparative liquid chromatography (0.1% trifluoroacetic acid/acetonitrile), and the prepared solution was concentrated under reduced pressure, and freeze-dried to obtain MGI453-2. LCMS: calcd for C₂₀H₁₃NO₁₀S₂ [M+H]⁺: 492.00. Found, m/z, [M+H]⁺: 492.00.

¹H NMR (600 MHz, dmso) δ = 12.70 (s, 1H), 8.28 (d, *J*=7.8, 1H), 7.86 (dt, *J*=30.8, 7.6, 2H), 7.51 (d, *J*=7.5, 1H), 7.43 (d, *J*=9.4, 2H), 7.30 (d, *J*=9.4, 2H).

### (3) Synthesis of 4-(2-(3,6-dihydroxy-4,5-disulfoacridin-9-yl)-N-methylbenzamido)butyric acid (MGI453)

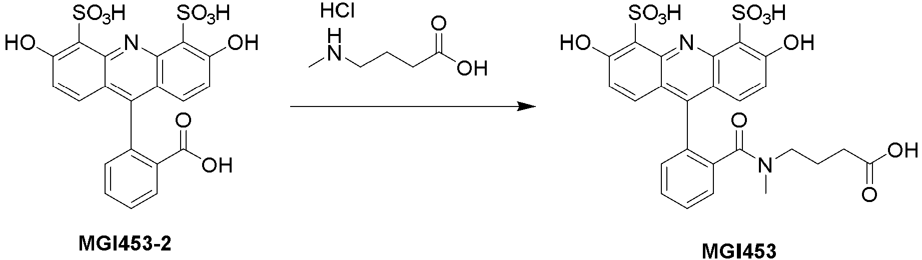

In a 15 mL sample vial, MGI453-2 (50 mg, 101.74 µmol) was dissolved with anhydrous N,N-dimethylformamide (2 mL), then N,N'-disuccinimidyl carbonate (52 mg, 202.99 µmol) and 4-dimethylaminopyridine (13 mg, 106.41 µmol) were added, followed by stirring magnetically at 20°C for 3 hours. Then 4-methylaminobutyric acid hydrochloride (78 mg, 507.79 µmol) and triethylamine (103 mg, 1.02 mmol) were added, followed by further stirring magnetically at 20 °C for 15 hours. The reaction solution was filtered, first the filtrate was purified by flash preparative liquid chromatography (0.1% trifluoroacetic acid/acetonitrile), and the prepared solution was concentrated under reduced pressure, and freeze-dried to obtain a yellow solid. After further purification by preparative liquid chromatography (0.1% trifluoroacetic acid/acetonitrile), the prepared solution was concentrated under reduced pressure, and freeze-dried to obtain MGI453. LCMS: calcd for C₂₅H₂₂N₂O₁₁S₂ [M+H]⁺: 590.07. Found, m/z, [M+H]⁺: 591.04.

¹H NMR (600 MHz, dmso) δ = 12.73 (s, 1H), 7.78 - 7.67 (m, 3H), 7.61 - 7.51 (m, 3H), 7.31 (dd, *J*=18.5, 9.4, 2H), 3.21 - 3.13 (m, 1H), 2.95 (t, *J*=7.2, 1H), 2.90 (s, 3H), 2.16 (t, *J*=7.0, 1H), 1.89 (t, *J*=7.1, 1H), 1.63 - 1.57 (m, 1H), 1.25 (p, *J*=7.1, 1H).

### Example 2 Synthesis of MGI450a

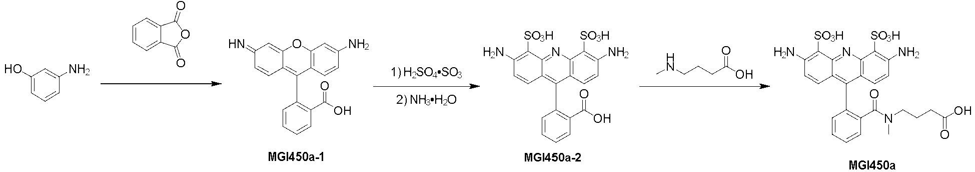

### (1) Synthesis of 2-(6-amino-3-imino-3H-xanthen-9-yl)benzoic acid (MGI450a-1)

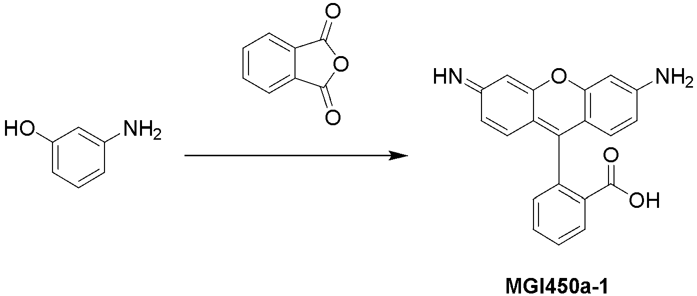

In a 40 mL sample vial, 3-aminophenol (2.95 g, 27.01 mmol), methanesulfonic acid (15 mL), and then phthalic anhydride (2 g, 13.50 mmol) were added, heated up to 180°C, and stirred magnetically for 6 hours. The reaction solution was slowly poured into ice water to quench the reaction, followed by filtering, and the filter cake was washed with a small amount of water to obtain a red solid. The red solid was slurried with dichloromethane/methanol = 10/1 (50 mL), filtered, and dried to obtain MGI450a-1. LCMS: calcd for C₂₀H₁₄N₂O₃ [M+H]⁺: 331.10. Found, m/z, [M+H]⁺: 331.18.

### (2) Synthesis of 2-(3,6-diamino-4,5-disulfoacridin-9-yl) benzoic acid (MGI450a-2)

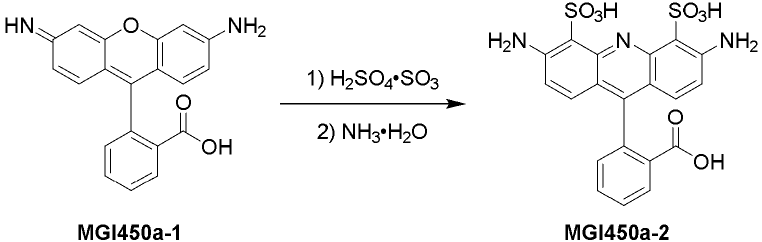

In a 250 mL flask, 50% fuming sulfuric acid (100 mL) was added for dissolution, then MGI450a-1 (2.2 g, 6.66 mmol) was added in batches, heated up to 60°C, and stirred magnetically for 15 hours. The reaction solution was slowly added dropwise into ice water to quench the reaction, then sodium carbonate was added to adjust the solution to neutral. An equal volume of methanol and ammonia solution (25%-28%, 100 mL) was added, followed by stirring magnetically at 22°C for 15 hours. The reaction solution was concentrated under reduced pressure to remove most of the solvent, followed by filtering, the filtrate was purified by flash preparative liquid chromatography (0.1% trifluoroacetic acid/acetonitrile), and the prepared solution was concentrated under reduced pressure to obtain MGI450a-2. LCMS: calcd for C₂₀H₁₅N₃O₈S₂ [M+H]⁺:490.03. Found, m/z, [M+H]⁺: 490.13.

### (3) Synthesis of 4-(2-(3,6-diamino-4,5-disulfoacridin-9-yl)-N-methylbenzamido)butyric acid (MGI450a)

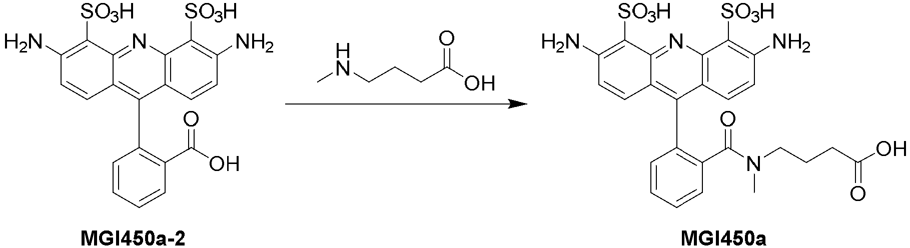

In a 50 mL flask, MGI450a-2 (200 mg, 408.60 µmol) was dissolved with anhydrous N,N-dimethylformamide (10 mL), then N,N'-disuccinimidyl carbonate (209 mg, 815.86 µmol) and 4-dimethylaminopyridine (50 mg, 409.26 µmol) were added, followed by stirring magnetically at 22°C for 15 hours. Then 4-methylaminobutyric acid (96 mg, 819.48 µmol) and triethylamine (165 mg, 1.63 mmol) were added, followed by further stirring magnetically at 20°C for 15 hours. The reaction solution was filtered, first the filtrate was purified by flash preparative liquid chromatography (0.1% trifluoroacetic acid/acetonitrile), then purified by preparative liquid chromatography (0.1% trifluoroacetic acid/acetonitrile), and the prepared solution was concentrated under reduced pressure to obtain MGI450a. LCMS: calcd for C₂₅H₂₄NO₉S₂ [M+H]⁺: 589.10. Found, m/z, [M+H]⁺: 589.28.

¹H NMR (600 MHz, DMSO) δ = 14.62 (s, 1H), 8.13 (d, *J*=7.3, 1H), 7.67 (d, *J*=7.0, 2H), 7.23 (d, *J*=6.3, 1H), 6.98 (d, *J*=9.3, 2H), 6.91 (d, *J*=9.3, 2H).

### Example 3 Synthesis of MGI450b

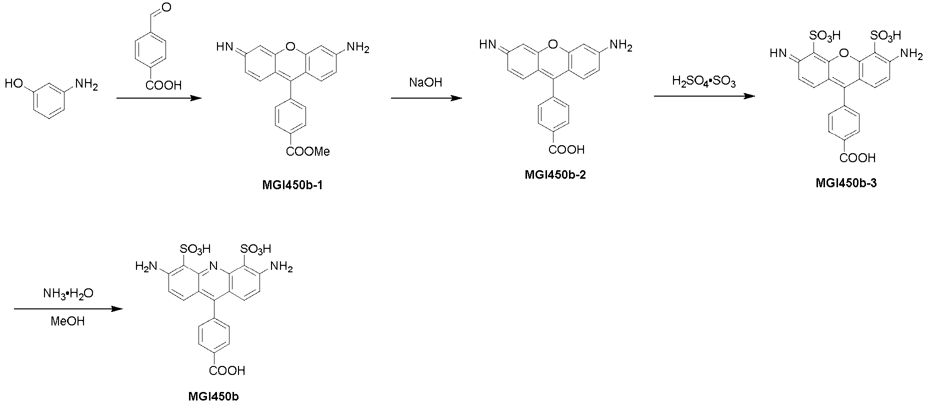

### (1) Synthesis of methyl 4-(6-amino-3-imino-3H-xanthen-9-yl)benzoate (MGI450b-1)

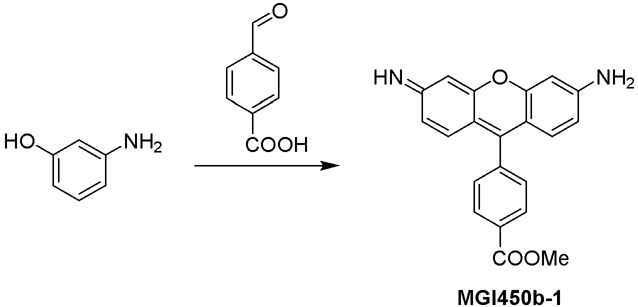

In a 40 mL sample vial, 3-aminophenol (2.91 g, 26.64 mmol), methanesulfonic acid (20 mL), and then 4-formylbenzoic acid (2 g, 13.32 mmol) were added, heated up to 180°C, and stirred magnetically for 5 hours. The reaction solution was transferred to a 250 mL flask, methanol (50 mL) and dichloromethane (50 mL), and then 2,3-dichloro-5,6-dicyano-1,4-benzoquinone (3.02 g, 13.32 mmol) were added, followed by stirring magnetically at 18°C for 2 hours. The reaction solution was concentrated under reduced pressure to remove methanol and dichloromethane, then the concentrated solution was poured into water (500 mL), stirred, and filtered to obtain a crude product, and the crude product was purified by column chromatography to obtain MGI450b-1. LCMS: calcd for C₂₁H₁₆N₂O₃ [M+H]⁺:345.12. Found, m/z, [M+H]⁺: 345.17.

### (2) Synthesis of 4-(6-amino-3-imino-3H-xanthen-9-yl)benzoic acid (MGI450b-2)

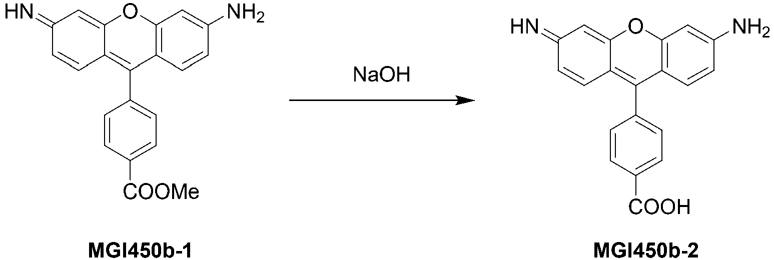

In a 100 mL flask, MGI450b-1 (3.9 g, 11.33 mmol) was dissolved with methanol (90 mL), and an aqueous solution (30 mL) of sodium hydroxide (906 mg, 22.65 mmol), and stirred magnetically at 19°C for 5 hours. The reaction solution was concentrated under reduced pressure to remove methanol, adjusted to pH = 5-6 with 1 M aqueous hydrochloric acid solution, filtered, washed with a small amount of water, and dried to obtain MGI450b-2.

### (3) Synthesis of 4-(6-amino-3-imino-4,5-disulfo-3H-xanthen-9-yl)benzoic acid (MGI450b-3)

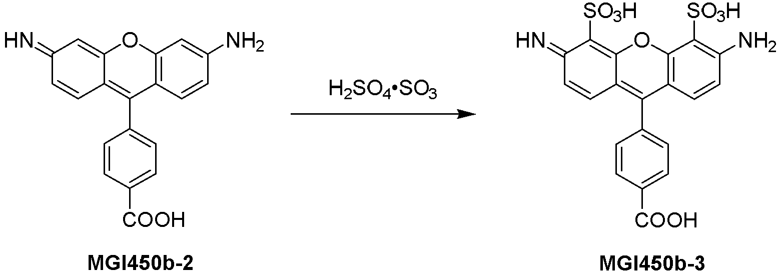

In a 250 mL flask, 50% fuming sulfuric acid (100 mL) was added for dissolution, then MGI450b-2 (2 g, 6.05 mmol) was added, heated up to 60°C, and stirred magnetically for 20 hours. The reaction solution was slowly added dropwise into crushed ice to quench the reaction, followed by filtering, the filtrate was purified by flash preparative liquid chromatography (water/acetonitrile), and the prepared solution was concentrated under reduced pressure to obtain MGI450b-3. LCMS: calcd for C₂₀H₁₄N₂O₉S₂ [M-H]⁻:489.01. Found, m/z, [M-H]⁻: 489.11.

### (4) Synthesis of 4-(3,6-diamino-4,5-disulfoacridin-9-yl)benzoic acid (MGI450b)

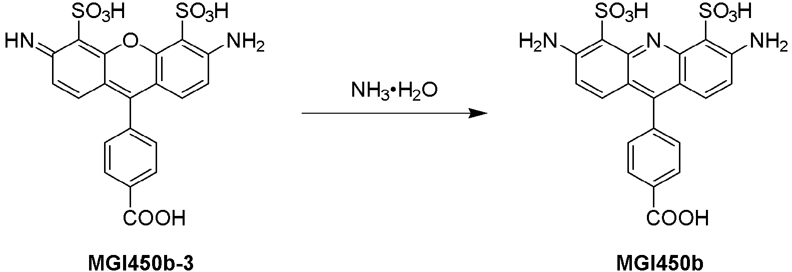

In a 40 mL sample vial, MGI450b-3 (100 mg, 203.89 µmol) was dissolved with ammonia solution (25%-28%, 5 mL) and methanol (5 mL), and stirred magnetically at 25°C for 15 hours. The reaction solution was concentrated under reduced pressure to obtain a crude product, the crude product was purified by preparative liquid chromatography (0.1 M TEAB/acetonitrile), and the prepared solution was concentrated under reduced pressure, and freeze-dried to obtain MGI450b. LCMS: calcd for C₂₀H₁₅N₃O₈S₂ [M-H]⁻: 488.03. Found, m/z, [M-H]⁻: 488.19.

¹H NMR (600 MHz, dmso) δ = 8.14 (d, *J*=8.2, 2H), 7.47 (d, *J*=8.0, 2H), 7.07 (d, *J*=9.3, 2H), 6.96 (d, *J*=9.4, 2H).

### Example 4

Fluorescent dye MGI450c was synthesized in accordance with the method for synthesis of MGI450b.

LCMS: calcd for C₂₁H₁₅N₃O₁₀S₂ [M-H]⁻: 532.02. Found, m/z, [M-H]⁻: 532.18.

### Example 5

Fluorescent dye MGI450d was synthesized in accordance with the method for synthesis of MGI450b.

LCMS: calcd for C₂₄H₂₂N₄O₁₂S₃ [M-H]⁻: 653.04. Found, m/z, [M-H]⁻: 653.11.

### Example 6

Fluorescent dye MGI443 was synthesized in accordance with the method for synthesis of MGI450b.

LCMS: calcd for C₂₀H₁₃F₂N₃O₈S₂ [M-H]⁻:524.01. Found, m/z, [M-H]⁻: 524.17.

¹H NMR (400 MHz, dmso) δ = 8.16 (d, *J*=8.2, 2H), 7.51 (d, *J*=8.1, 2H), 6.95 (d, *J=*11.6, 2H).

### Example 7

Fluorescent dye MGI455 was synthesized in accordance with the method for synthesis of MGI450b.

LCMS: calcd for C₂₀H₁₃Cl₂N₃O₈S₂ [M+H]⁺:557.95. Found, m/z, [M+H]⁺: 558.90.

¹H NMR (600 MHz, DMSO) δ = 14.97 (s, 1H), 8.73 (s, 2H), 8.21 - 8.16 (m, 2H), 7.56 (d, *J*=8.1, 2H), 7.28 (s, 2H).

### Example 8

Fluorescent dye MGI447 was synthesized in accordance with the method for synthesis of MGI450b.

LCMS: calcd for C₂₂H₁₉N₃O₈S₂ [M-H]⁻:516.06. Found, m/z, [M-H]⁻: 516.12.

¹H NMR (600 MHz, dmso) δ = 8.12 (d, *J*=7.6, 2H), 7.36 (d, *J*=7.7, 2H), 6.98 (s, 2H), 2.11 (s, 6H).

### Example 9

Fluorescent dye MGI477 was synthesized in accordance with the method for synthesis of MGI450b.

LCMS: calcd for C₂₄H₂₃N₃O₈S₂ [M-H]⁻:544.09. Found, m/z, [M-H]⁻: 544.18.

¹H NMR (600 MHz, dmso) δ = 8.94 (s, 2H), 8.16 (d, *J*=7.4, 2H), 7.51 (d, *J*=7.3, 2H), 7.17 (d, *J*=9.5, 2H), 7.09 (d, *J*=9.6, 2H), 3.41 - 3.35 (m, 4H), 1.21 (t, *J*=7.1, 6H).

### Example 10

Fluorescent dye MGI456 was synthesized in accordance with the method for synthesis of MGI450b.

LCMS: calcd for C₂₄H₁₇F₆N₃O₈S₂ [M+H]⁺:654.04. Found, m/z, [M+H]⁺: 654.20.

¹H NMR (600 MHz, dmso) δ = 9.39 (t, *J*=6.7, 2H), 8.17 (d, *J*=7.5, 2H), 7.55 (d, *J*=7.0, 2H), 7.40 (d, *J*=9.7, 2H), 7.26 (d, *J*=9.6, 2H), 4.47 (p, *J*=8.9, 4H).

### Example 11

Fluorescent dye MGI471 was synthesized in accordance with the method for synthesis of MGI450b.

LCMS: calcd for C₃₀H₃₁N₃O₈S₂ [M-H]⁻: 624.16. Found, m/z, [M-H]⁻: 624.23.

¹H NMR (600 MHz, DMSO-d₆): δ 14.37 (s, 1H), 8.22 - 8.18 (m, 2H), 7.83 (s, 2H), 7.52 (dt, *J =* 8.6, 4.5 Hz, 2H), 6.76 (s, 2H), 3.77 - 3.71 (m, 2H), 1.18 - 1.14 (m, 12H), 0.99 (d, *J =* 4.3 Hz, 6H).

### Example 12

Fluorescent dye MGI485 was synthesized in accordance with the method for synthesis of MGI450b.

LCMS: calcd for C₃₂H₃₅N₃O₈S₂ [M-H]⁻:652.19. Found, m/z, [M-H]⁻: 652.45.

¹H NMR (600 MHz, dmso) δ = 9.28 (s, 2H), 8.17 (d, *J*=6.4, 2H), 7.49 (d, *J*=5.4, 2H), 6.98 (s, 2H), 1.81 (d, *J*=9.0, 2H), 1.30 -1.24 (m, 16H), 1.04 (dd, *J*=14.6, 8.5, 6H).

### Example 13

Fluorescent dye MGI488 was synthesized in accordance with the method for synthesis of MGI450b.

LCMS: calcd for C₂₉H₃₂N₄O₉S₂ [M-H]⁻:643.16. Found, m/z, [M-H]⁻: 643.22.

¹H NMR (400 MHz, dmso) δ = 8.88 (t, *J*=5.2, 2H), 7.72 - 7.63 (m, 2H), 7.62 - 7.53 (m, 1H), 7.43 - 7.31 (m, 1H), 7.21 - 7.00 (m, 4H), 3.35 (dt, *J*=12.5, 6.8, 4H), 3.15 - 3.06 (m, 1H), 3.05 - 2.98 (m, 1H), 2.83 (s, 3H), 2.12 (t, *J*=6.9, 1H), 1.95 (t, *J*=7.1, 1H), 1.56 (q, *J*=7.3,1H), 1.34 (q, *J*=7.3, 1H), 1.21 (t, *J*=7.2, 6H).

### Example 14 Synthesis of MGI501 modified nucleotide

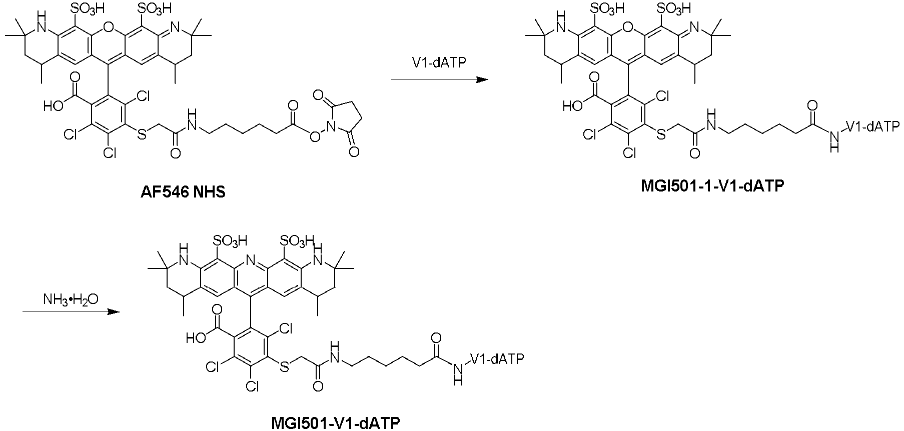

### (1) Synthesis of MGI501-1-V1-dATP or MGI501-1-V1-dCTP

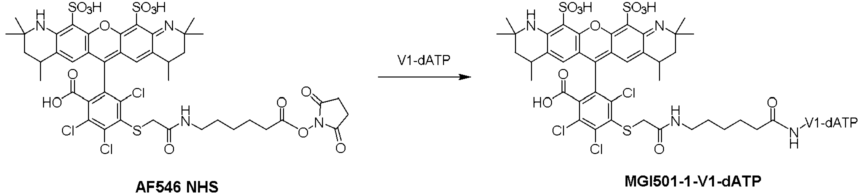

In a 4 mL sample vial, AF546 NHS (10 mg, 9.45 µmol) was dissolved with N,N-dimethylformamide (1 mL), then V1-dATP (18 mg, 18.99 µmol) and N,N-diisopropylaminoethylamine (12 mg, 92.85 µmol) were added, followed by stirring magnetically at 22°C for 15 hours. The reaction solution was filtered, the filtrate was purified by flash preparative chromatography (0.1 M TEAB/acetonitrile), and the prepared solution was concentrated under reduced pressure to obtain MGI501-1-V1-dATP. LCMS: calcd for C₇₀H₈₂Cl₃N₁₆O₂₈P₃S₃ [M-H]: 1889.29. Found, m/z, [(M-2)/2]⁻: 944.45.

MGI501-1-V1-dCTP was synthesized in accordance with the same method as described above.

LCMS: calcd for C₆₈H₈₀Cl₃N₁₄O₃₀P₃S₃ [M-H]⁻: 1865.26. Found, m/z, [(M-2)/2]⁻: 932.87.

### (2) Synthesis of MGI501-V1-dATP or MGI501-V1-dCTP

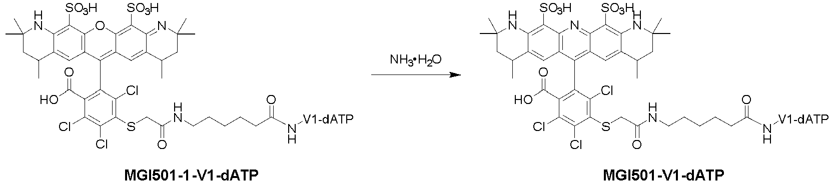

In a 15 mL sample vial, MGI501-1-V1-dATP (10 mg, 5.29 µmol) was dissolved with a methanol solution of ammonia (7 M, 2 mL), and stirred magnetically at 23°C for 15 hours. The reaction solution was filtered, the filtrate was purified by preparative liquid chromatography (0.1% ammonia/acetonitrile), and the prepared solution was concentrated under reduced pressure, and freeze-dried to obtain MGI501-V1-dATP. LCMS: calcd for C₇₀H₈₃Cl₃N₁₇O₂₇P₃S₃ [M-H]⁻:1888.31. Found, m/z, [(M-2)/2]⁻: 943.87.

¹H NMR (400 MHz, DMSO-*d*₆) δ 9.24 (t, *J =* 6.1 Hz, 2H), 8.44 (s, 1H), 8.10 (s, 1H), 8.01 (s, 2H), 7.70 (s, 1H), 7.48 - 7.41 (m, 2H), 7.35 - 7.27 (m, 2H), 7.08 (d, *J =* 14.6 Hz, 3H), 6.99 (d, *J= 15.8* Hz, 2H), 6.49 (dd, *J* = 8.9, 5.8 Hz, 1H), 5.14 (s, 1H), 4.93 (d, *J* = 9.0 Hz, 1H), 4.86 (d, *J* = 8.8 Hz, 1H), 4.57 (s, 1H), 4.25 - 4.20 (m, 1H), 4.14 (d, *J* = 5.5 Hz, 4H), 3.98 (s, 7H), 3.68 (s, 2H), 2.86 (d, *J =* 62.4 Hz, 16H), 2.01 (s, 2H), 1.82 (d, *J =* 12.8 Hz, 2H), 1.43 (s, 2H), 1.31 (s, 5H), 1.25 (d, *J* = 5.5 Hz, 3H), 1.20 (t, *J =* 6.3 Hz, 3H), 1.12 (dd, *J =* 6.5, 3.4 Hz, 2H), 1.07 - 1.00 (m, 3H).

³¹P NMR (162 MHz, dmso) δ = -9.98 (d, *J*=18.5), -13.44, -22.65.

MGI501-V1-dCTP was synthesized in accordance with the same method as described above.

LCMS: calcd for C₆₈H₈₁Cl₃N₁₅O₂₉P₃S₃ [M-H]⁻: 1864.28. Found, m/z, [M-H]⁻: [(M-2)/2]⁻: 932.30.

¹H NMR (400 MHz, dmso) δ 9.24 (d, *J =* 5.9 Hz, 2H), 8.67 (s, 2H), 8.03 (s, 3H), 7.75 (s, 1H), 7.45 (d, *J* = 7.6 Hz, 2H), 7.35 - 7.29 (m, 1H), 7.11 (s, 2H), 7.07 (s, 1H), 7.00 (d, *J =* 15.7 Hz, 2H), 6.88 (s, 1H), 6.15 - 6.07 (m, 1H), 5.16 (s, 1H), 4.92 (d, *J* = 9.1 Hz, 1H), 4.81 (d, *J* = 8.9 Hz, 1H), 4.41 (s, 1H), 4.27 - 4.21 (m, 2H), 4.15 (dd, *J =* 17.8, 8.4 Hz, 4H), 4.01 - 3.90 (m, 5H), 3.89 - 3.82 (m, 2H), 3.70 - 3.65 (m, 2H), 3.49 (d, *J* = 8.2 Hz, 2H), 3.19 (s, 2H), 2.93 (d, *J =* 6.6 Hz, 3H), 2.85 (s, 2H), 2.71 (d, *J =* 17.9 Hz, 1H), 2.31 - 2.30 (m, 1H), 2.15 (s, 2H), 2.01 (s, 2H), 1.82 (d, *J* = 12.4 Hz, 2H), 1.42 (d, *J =* 8.2 Hz, 3H), 1.36 - 1.23 (m, 12H), 1.22 - 1.11 (m, 6H).

³¹P NMR (162 MHz, dmso) δ 74.99 (s), -10.17 - -10.42 (m), -12.28 - -12.91 (m), -22.73 (s).

### Example 15 Synthesis of MGI495 modified nucleotide

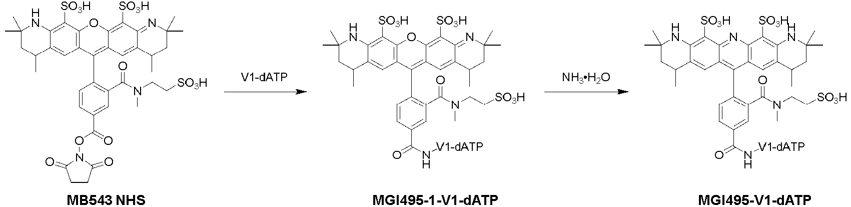

### (1) Synthesis of MGI495-1-V1-dATP, MGI495-1-V1-dCTP

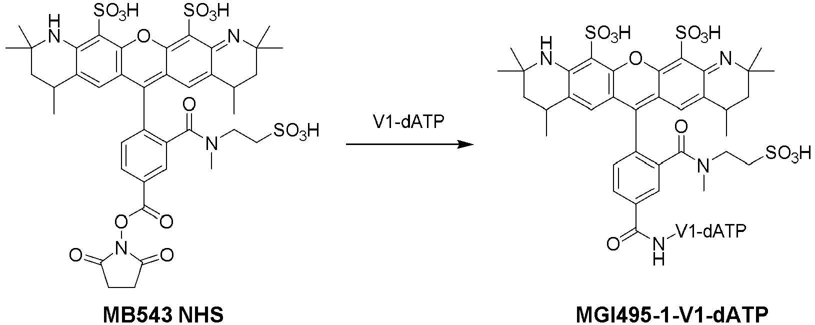

In a 15 mL sample vial, MB543 NHS (15 mg, 16.36 µmol) was dissolved with anhydrous N,N-dimethylformamide (2 mL), then V1-dATP (18 mg, 18.99 µmol) and N,N-diisopropylethylamine (19 mg, 139.27 µmol) were added, followed by stirring magnetically at 22°C for 15 hours. The reaction solution was filtered, the filtrate was purified by flash preparative liquid chromatography (0.1 M TEAB/acetonitrile), and the prepared solution was concentrated under reduced pressure to obtain MGI495-1-V1-dATP. LCMS: calcd for C₆₆H₇₉N₁₆O₂₉P₃S₃ [M-H]⁻: 1747.36 Found, m/z, [(M-2)/2]⁻: 873.35.

MGI495-1-V1-dCTP was synthesized in accordance with the same method as described above.

LCMS: calcd for C₆₄H₇₇N₁₄O₃₁P₃S₃ [M-H]⁻: 1725.33 Found, m/z, [(M-2)/2]⁻: 862.03.

### (2) Synthesis of MGI495-V1-dATP, MGI495-V1-dCTP

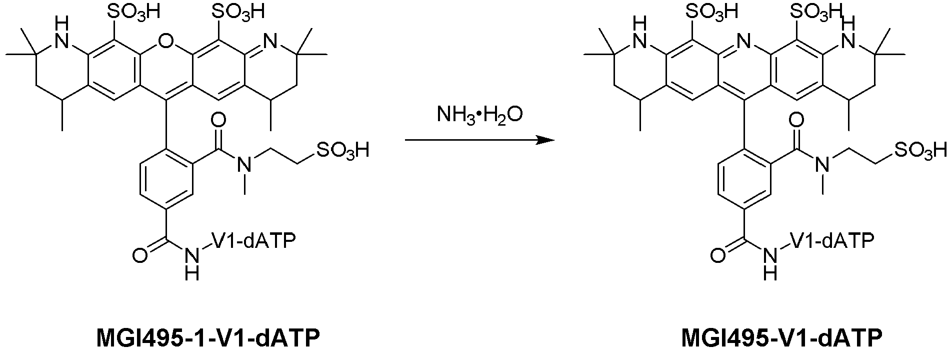

In a 15 mL sample vial, MGI495-1-V1-dATP (25 mg, 14.29 µmol) was dissolved with methanol (2 mL), then ammonia (2 mL) was added, followed by stirring magnetically at 25°C for 15 hours. The reaction solution was concentrated under reduced pressure to obtain a crude product, the crude product was purified by preparative HPLC (0.1% NH₃·H₂O/acetonitrile), and the prepared solution was concentrated under reduced pressure, and freeze-dried to obtain the compound MGI495-V1-dATP.LCMS: calcd for C₆₆H₈₀N₁₇O₂₈P₃S₃ [M-H]⁻: 1746.37 Found, m/z, [(M-2)/2]⁻: 837.09.

¹H NMR (400 MHz, dmso) δ 9.26 (d, *J =* 7.8 Hz, 2H), 9.04 (d, *J =* 42.3 Hz, 2H), 8.76 (s, 1H), 8.43 (t, *J =* 5.5 Hz, 1H), 8.24 (t, *J =* 11.7 Hz, 1H), 8.16 - 8.08 (m, 1H), 8.07 - 7.97 (m, 1H), 7.70 (s, 1H), 7.57 - 7.47 (m, 2H), 7.46 - 7.34 (m, 2H), 7.12 (d, *J =* 8.3 Hz, 2H), 6.94 (d, *J =* 5.3 Hz, 2H), 6.49 (dd, *J =* 8.7, 5.9 Hz, 1H), 5.15 (t, *J =* 4.6 Hz, 1H), 4.90 (dd, *J =* 31.3, 8.9 Hz, 2H), 4.56 (d, *J* = 4.9 Hz, 1H), 4.25 (dd, *J =* 10.4, 4.0 Hz, 1H), 4.16 (dd, *J =* 16.6, 5.3 Hz, 3H), 3.99 - 3.83 (m, 5H), 3.72 - 3.67 (m, 2H), 3.31 (s, 3H), 3.06 (dd, *J =* 15.8, 8.6 Hz, 1H), 2.99 - 2.73 (m, 3H), 2.65 - 2.57 (m, 2H), 2.42 - 2.37 (m, 1H), 1.81 (d, *J =* 13.6 Hz, 2H), 1.45 - 1.12 (m, 12H), 1.04 (dd, *J =* 6.0, 2.6 Hz, 6H).

³¹P NMR (162 MHz, dmso) δ -10.51 (d, *J =* 18.9 Hz), -12.94 (d, *J =* 24.0 Hz), -23.00 - -23.49 (m).

MGI495-V1-dCTP was synthesized in accordance with the same method as described above.

LCMS: calcd for C₆₄H₇₇N₁₅O₃₀P₃S₃ [M-H]⁻: 1724.34 Found, m/z, [(M-2)/2]⁻: 861.35.

¹H NMR (400 MHz, dmso) δ 9.26 (d, *J =* 10.1 Hz, 2H), 9.06 (d, *J=* 32.6 Hz, 2H), 8.83 - 8.75 (m, 1H), 8.58 (dd, *J =* 12.2, 7.8 Hz, 1H), 8.24 (t, *J =* 11.5 Hz, 1H), 8.16 - 8.01 (m, 2H), 7.98 (s, 1H), 7.76 (s, 1H), 7.57 - 7.48 (m, 2H), 7.45 - 7.35 (m, 2H), 7.14 (d, *J =* 8.2 Hz, 2H), 6.94 (d, *J =* 5.2 Hz, 2H), 6.89 (s, 1H), 6.11 (dd, *J =* 7.8, 6.0 Hz, 1H), 5.19 - 5.13 (m, 1H), 4.91 (d, *J =* 8.8 Hz, 1H), 4.80 (d, *J =* 8.9 Hz, 1H), 4.44 - 4.39 (m, 1H), 4.26 (dd, *J =* 10.4, 4.3 Hz, 1H), 4.20 - 4.06 (m, 4H), 3.99 - 3.90 (m, 4H), 3.86 (d, *J =* 4.5 Hz, 2H), 3.68 (s, 2H), 3.03 (dd, *J =* 14.2, 6.9 Hz, 1H), 2.83 (dd, *J =* 28.6, 23.2 Hz, 3H), 2.58 (t, *J =* 5.4 Hz, 2H), 2.40 (s, 1H), 2.32 (d, *J =* 8.0 Hz, 1H), 2.17 (dd, *J =* 14.7, 7.6 Hz, 1H), 1.81 (d, *J =* 12.1 Hz, 2H), 1.45 - 1.12 (m, 12H), 1.05 (d, *J =* 6.4 Hz, 6H).

³¹P NMR (162 MHz, dmso) δ -10.44 (d, *J =* 18.9 Hz), -12.86 (d, *J =* 27.2 Hz), -23.09 (t, *J =* 20.3 Hz).

### Example 16 Synthesis of MGI450c modified nucleotide

(1) The following compounds were synthesized in accordance with the method as described in Example 15:
   MGI450c-1-V1-dATP
   LCMS: calcd for C₅₁H₅₂N₁₅O₂₇P₃S₂ [M-H]⁻: 1462.18. Found, m/z, [(M-2)/2]⁻: 730.68.
   MGI450c-1-V1-dCTP
   LCMS: calcd for C₄₉H₅₀N₁₃O₂₉P₃S₂ [M-H]⁻: 1440.15. Found, m/z, [(M-2)/2]⁻: 719.20.
   MGI450c-1-V1-dTTP
   LCMS: calcd for C₄₉H₅₁N₁₄O₂₈P₃S₂ [M-H]⁻: 1439.17. Found, m/z, [(M-2)/2]⁻: 719.73.
   MGI450c-1-V1-dGTP
   LCMS: calcd for C₅₁H₅₂N₁₅O₂₈P₃S₂ [M-H]⁻: 1478.18. Found, m/z, [(M-2)/2]⁻: 738.80.
(2) The following compounds were synthesized in accordance with the method as described in Example 15:
   MGI450c-V1-dATP
   LCMS: calcd for C₅₁H₅₃N₁₆O₂₆P₃S₂ [M-H]⁻: 1461.20. Found, m/z, [(M-2)/2]⁻: 730.27.
   ¹H NMR (400 MHz, dmso) δ 9.07 (s, 1H), 8.76 (s, 1H), 8.67 (d, *J =* 1.4 Hz, 1H), 8.43 (t, *J =* 5.4 Hz, 1H), 8.20 (d, *J =* 8.2 Hz, 1H), 8.10 (s, 1H), 7.85 (s, 3H), 7.70 (s, 1H), 7.51 (d, *J =* 8.2 Hz, 2H), 7.42 - 7.34 (m, 2H), 7.12 (d, *J =* 8.3 Hz, 1H), 6.94 (dd, *J =* 18.2, 9.3 Hz, 3H), 6.49 (dd, *J =* 8.8, 5.8 Hz, 1H), 5.15 (t, *J* = 4.8 Hz, 1H), 4.89 (dd, *J =* 29.8, 8.9 Hz, 2H), 4.56 (d, *J* = 4.6 Hz, 1H), 4.25 (dd, *J =* 10.5, 4.4 Hz, 1H), 4.16 (dd, J= 17.2, 4.8 Hz, 3H), 3.98 (s, 1H), 3.96 - 3.81 (m, 4H), 3.68 (d, *J=* 6.1 Hz, 3H), 2.95 (dd, *J =* 14.4, 7.1 Hz, 2H), 2.85 (d, *J =* 7.2 Hz, 1H), 2.69 - 2.58 (m, 1H), 2.39 (dd, *J =* 13.3, 5.9 Hz, 1H).
   ³¹P NMR (162 MHz, dmso) δ -10.47 (d, *J =* 19.4 Hz), -12.76 (d, *J =* 20.9 Hz), -22.81 - -23.33 (m).
   MGI450c-V1-dCTP
   LCMS: calcd for C₄₉H₅₁N₁₄O₂₈P₃S₂ [M-H]⁻: 1439.17. Found, m/z, [(M-2)/2]⁻: 718.74.
   ¹H NMR (400 MHz, dmso) δ 9.09 (s, 1H), 8.80 (s, 1H), 8.68 (d, *J =* 0.8 Hz, 1H), 8.22 (d, *J =* 7.4 Hz, 1H), 8.02 (s, 1H), 7.85 (s, 3H), 7.72 (s, 1H), 7.58 - 7.45 (m, 1H), 7.42 - 7.34 (m, 1H), 7.14 (d, *J* = 8.9 Hz, 1H), 6.92 (dt, *J =* 16.5, 8.3 Hz, 3H), 6.17 - 6.06 (m, 1H), 5.17 (dd, *J =* 6.0, 4.3 Hz, 1H), 4.90 (d, *J* = 8.8 Hz, 1H), 4.79 (d, *J =* 8.9 Hz, 1H), 4.41 (s, 1H), 4.27 (dd, *J* = 9.6, 6.0 Hz, 1H), 4.22 - 4.06 (m, 3H), 4.03 - 3.82 (m, 4H), 3.67 (dd, *J =* 7.9, 2.9 Hz, 2H), 3.52 (s, 2H), 2.88 (s, 3H), 2.31- 2.29 (m, 1H), 2.19 - 2.12 (m, 1H).
   ³¹P NMR (162 MHz, dmso) δ -9.64 - -10.54 (m), -12.89 - -13.79 (m), -22.30 - -23.25 (m).
   MGI450c-V1-dTTP
   LCMS: calcd for C₄₉H₅₂N₁₅O₂₇P₃S₂ [M-H]⁻: 1438.18. Found, m/z, [(M-2)/2]⁻: 719.24.
   MGI450c-V1-dGTP
   LCMS: calcd for C₅₁H₅₃N₁₆O₂₇P₃S₂ [M-H]⁻: 1477.19. Found, m/z, [(M-2)/2]⁻: 738.15.
   ¹H NMR (400 MHz, dmso) δ 9.07 (s, 1H), 8.82 (s, 1H), 8.65 (s, 1H), 8.35 - 8.25 (m, 1H), 8.09 (d, *J* = 7.6 Hz, 1H), 7.80 (s, 3H), 7.51 (d, *J =* 8.0 Hz, 1H), 7.43 - 7.36 (m, 1H), 7.25 (d, *J =* 8.1 Hz, 1H), 7.19 (s, 1H), 7.12 (d, *J =* 8.2 Hz, 1H), 6.97 (d, *J =* 9.3 Hz, 1H), 6.89 (d, *J* = 9.3 Hz, 1H), 6.54 (s, 1H), 6.20 (dd, *J =* 8.8, 5.8 Hz, 1H), 5.21 - 5.14 (m, 1H), 4.87 (dd, *J =* 25.6, 8.8 Hz, 2H), 4.53 (d, *J =* 4.0 Hz, 1H), 4.27 (dd, *J* = 10.3, 4.4 Hz, 1H), 4.20 (dd, *J =* 10.1, 5.8 Hz, 1H), 4.12 (d, *J* = 5.1 Hz, 1H), 4.06 (d, *J* = 4.7 Hz, 1H), 3.98 - 3.83 (m, 4H), 3.68 (d, *J =* 8.8 Hz, 2H), 3.49 (s, 2H), 2.73 (d, *J* = 7.0 Hz, 1H), 2.64 - 2.56 (m, 1H), 2.30 - 2.23 (m, 1H).
   ³¹P NMR (162 MHz, dmso) δ -10.50 (d, *J =* 20.0 Hz), -11.96 - -12.59 (m), -22.86 (dd, *J =* 24.2, 20.9 Hz).

### Example 17 Synthesis of MGI450b modified nucleotide

### (1) Synthesis of MGI450b-V1

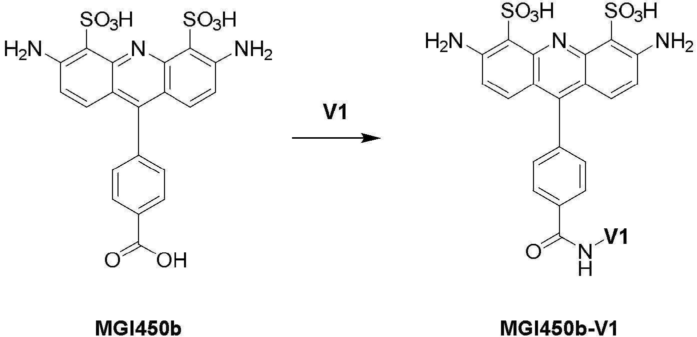

In a 15 mL sample vial, MGI450b (20 mg, 40.86 µmol) was dissolved with anhydrous N,N-dimethylformamide (2 mL), then N,N'-disuccinimidyl carbonate (21 mg, 81.98 µmol) and 4-dimethylaminopyridine (5 mg, 40.93 µmol) were added, followed by stirring magnetically at 25°C for 2 hours. Then V1 (30 mg, 81.66 µmol) and triethylamine (21 mg, 207.52 µmol) were added, followed by further stirring magnetically at 25°C for 4 hours. The reaction solution was filtered, first the filtrate was purified by flash preparative liquid chromatography (0.1 M TEAB/acetonitrile), and the prepared solution was concentrated under reduced pressure to obtain a crude product. Then the crude product was purified by preparative liquid chromatography (0.1 M TEAB/acetonitrile), and the prepared solution was concentrated under reduced pressure, and freeze-dried to obtain MGI450b-V1. LCMS: calcd for C₃₅H₃₄N₈O₁₃S₂ [M-H]⁻:837.17. Found, m/z, [M-H]⁻: 837.36.

### (2) Synthesis of MGI450b-V1-dATP

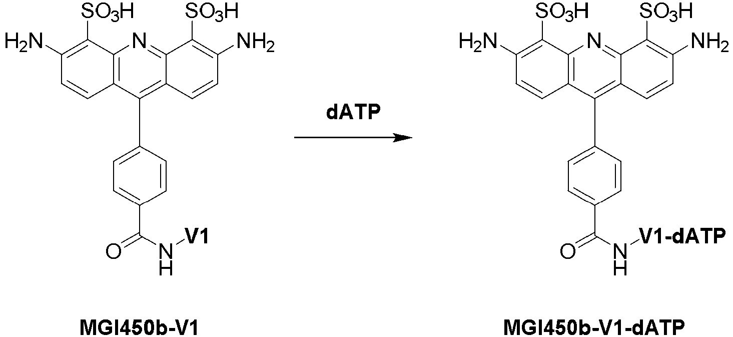

In a 15 mL sample vial, MGI450b-V1 (10 mg, 11.92 µmol) was dissolved with anhydrous N,N-dimethylformamide (1 mL), then N,N'-disuccinimidyl carbonate (6 mg, 23.42 µmol) and 4-dimethylaminopyridine (3 mg, 24.56 µmol) were added, followed by stirring magnetically at 25°C for 2 hours. Then dATP (14 mg, 23.40 µmol) and triethylamine (6 mg, 59.29 µmol) were added, followed by further stirring magnetically at 25°C for 4 hours. The reaction solution was filtered, first the filtrate was purified by flash preparative liquid chromatography (0.1 M TEAB/acetonitrile), and the prepared solution was concentrated under reduced pressure to obtain a crude product. Then the crude product was purified by preparative liquid chromatography (0.1 M TEAB/acetonitrile), and the prepared solution was concentrated under reduced pressure, and freeze-dried to obtain MGI450b-V1-dATP. LCMS: calcd for C₅₀H₅₃N₁₆O₂₄P₃S₂ [M-H]⁻:1417.21. Found, m/z, [(M-2)/2]⁻: 708.36.

¹H NMR (400 MHz, dmso) δ 8.97 (s, 1H), 8.79 (s, 1H), 8.45 - 8.38 (m, 1H), 8.15 - 8.09 (m, 2H), 7.95 (s, 3H), 7.70 (d, *J =* 1.6 Hz, 1H), 7.50 (t, *J =* 8.3 Hz, 2H), 7.42 - 7.35 (m, 1H), 7.30 - 7.24 (m, 1H), 7.16 - 7.05 (m, 2H), 6.98 (d, *J =* 9.4 Hz, 1H), 6.65 (d, *J =* 4.7 Hz, 1H), 6.49 (dd, *J =* 8.7, 5.8 Hz, 1H), 5.18 - 5.13 (m, 1H), 4.92 (d, *J =* 8.8 Hz, 1H), 4.85 (d, *J =* 8.9 Hz, 1H), 4.56 (d, *J* = 5.1 Hz, 1H), 4.25 (dd, *J =* 10.6, 4.4 Hz, 1H), 4.22 - 4.11 (m, 2H), 3.98 (s, 1H), 3.96 - 3.82 (m, 3H), 3.68 (dd, *J =* 6.5, 4.2 Hz, 2H), 3.51 (s, 4H), 2.95 (s, 10H), 2.70 - 2.58 (m, 1H), 2.39 (dd, *J =* 14.0, 5.9 Hz, 1H).

³¹P NMR (162 MHz, dmso) δ -10.28 (d, *J =* 18.7 Hz), -13.08 (d, *J =* 25.2 Hz), -22.76 (dd, *J* = 25.0, 18.9 Hz).

The following compounds were synthesized in accordance with the same method as described above:
MGI450b-V1-dCTP

LCMS: calcd for C₄₈H₅₂N₁₅O₂₅P₃S₂ [M-H]⁻:1394.19. Found, m/z, [(M-2)/2]⁻: 696.72.

¹H NMR (400 MHz, dmso) δ 9.10 (s, 1H), 8.96 (s, 1H), 8.78 (d, J = 23.3 Hz, 1H), 8.52 (s, 1H), 8.19 - 8.01 (m, 2H), 7.95 (s, 1H), 7.72 (s, 1H), 7.58 (s, 1H), 7.53 - 7.44 (m, 1H), 7.42 - 7.34 (m, 1H), 7.26 (d, J = 7.5 Hz, 1H), 7.15 - 7.06 (m, 1H), 6.98 (dd, J = 9.4, 4.0 Hz, 1H), 6.90 (d, J = 8.3 Hz, 1H), 6.12 (d, J = 7.4 Hz, 1H), 5.20 (d, J = 19.1 Hz, 1H), 4.94 - 4.75 (m, 2H), 4.43 (s, 1H), 4.19 (dd, J = 49.6, 15.5 Hz, 3H), 4.04 - 3.75 (m, 4H), 3.67 (s, 2H), 3.07 - 2.95 (m, 1H), 2.90 (s, 2H), 2.77 (S, 3H), 2.31 - 2.28 (m, 1H), 2.02 - 1.97(m, 1H).

³¹P NMR (162 MHz, dmso) δ -10.09 - -10.84 (m), -12.05 - -13.06 (m), -22.42 - -23.22 (m).

MGI450b-V1-dTTP
LCMS: calcd for C₄₈H₅₁N₁₄O₂₆P₃S₂ [M-H]⁻:1395.18. Found, m/z, [(M-2)/2]⁻: 697.42.

¹H NMR (400 MHz, dmso) δ 9.14 (s, 1H), 9.01 (s, 1H), 8.96 - 8.89 (m, 1H), 8.15 (dd, *J* = 8.5, 5.9 Hz, 2H), 7.96 (s, 3H), 7.61 (s, 1H), 7.49 (t, *J =* 9.0 Hz, 2H), 7.38 (t, *J =* 7.9 Hz, 1H), 7.15 - 7.06 (m, 2H), 7.02 - 6.98 (m, 1H), 6.10 (t, *J =* 6.8 Hz, 1H), 5.22 - 5.14 (m, 1H), 4.91 - 4.85 (m, 1H), 4.80 (d, *J =* 8.9 Hz, 1H), 4.47 (s, 1H), 4.26 (ddd, *J =* 15.7, 10.7, 4.6 Hz, 2H), 4.11 - 4.00 (m, 4H), 3.97 (d, *J =* 2.4 Hz, 1H), 3.92 - 3.83 (m, 4H), 3.67 (s, 8H), 2.31 (dd, *J =* 11.1, 6.8 Hz, 2H).

³¹P NMR (162 MHz, dmso) δ -10.84 (d, *J =* 21.3 Hz), -12.60 (d, *J =* 23.6 Hz), -23.29 (t, *J =* 22.4 Hz).

MGI450b-V1-dGTP
LCMS: calcd for C₅₀H₅₃N₁₆O₂₅P₃S₂ [M-H]⁻:1433.20. Found, m/z, [(M-2)/2]⁻: 716.34.

¹H NMR (400 MHz, dmso) δ 10.46 (s, 1H), 8.96 (s, 1H), 8.79 (s, 1H), 8.29 (s, 1H), 8.13 (d, *J* = 8.2 Hz, 1H), 7.96 (s, 3H), 7.53 - 7.46 (m, 2H), 7.41 - 7.35 (m, 1H), 7.19 (s, 1H), 7.13 (d, *J =* 7.6 Hz, 1H), 7.09 (d, *J =* 9.4 Hz, 1H), 6.98 (d, *J* = 9.4 Hz, 1H), 6.39 (s, 1H), 6.24 - 6.18 (m, 1H), 5.19 - 5.14 (m, 1H), 4.87 (dd, *J =* 26.2, 8.8 Hz, 2H), 4.52 (s, 1H), 4.27 (dd, *J =* 10.6, 4.0 Hz, 1H), 4.21 - 4.15 (m, 1H), 4.09 (dd, *J =* 15.8, 5.3 Hz, 2H), 3.99 - 3.81 (m, 4H), 3.72 - 3.63 (m, 2H), 3.51 (s, 2H), 2.90 (s, 8H), 2.68 - 2.56 (m, 1H), 2.35 - 2.22 (m, 1H).

³¹P NMR (162 MHz, dmso) δ -10.18 (d, *J =* 18.8 Hz), -13.20 (dd, *J =* 15.3, 13.5 Hz), -22.61 - -22.73 (m).

### Example 18 Synthesis of MGI443 modified nucleotide

The following compounds were synthesized in accordance with the method as described in Example 17:
MGI443-V1

LCMS: calcd for C₃₅H₃₂F₂N₈O₁₃S₂ [M-H]⁻: 873.15. Found, m/z, [M-H]⁻: 873.30.

MGI443-V1-dATP
LCMS: calcd for C₅₀H₅₁F₂N₁₆O₂₄P₃S₂ [M-H]⁻:1453.19. Found, m/z, [(M-2)/2]⁻: 726.60.

¹H NMR (400 MHz, dmso) δ 9.01 (s, 1H), 8.82 (s, 1H), 8.46 - 8.37 (m, 1H), 8.20 - 8.07 (m, 2H), 8.03 - 7.76 (m, 3H), 7.71 (d, *J* = 1.6 Hz, 1H), 7.54 (dd, *J* = 15.5, 8.1 Hz, 2H), 7.42 - 7.35 (m, 1H), 7.12 (d, *J =* 7.7 Hz, 1H), 6.95 (d, *J =* 11.6 Hz, 1H), 6.49 (dd, *J =* 8.8, 5.9 Hz, 1H), 5.15 (t, *J* = 4.8 Hz, 1H), 4.89 (dd, *J* = 25.8, 8.9 Hz, 2H), 4.57 (d, *J* = 4.9 Hz, 1H), 4.25 (dd, *J* = 10.5, 4.5 Hz, 1H), 4.20 - 4.12 (m, 2H), 4.01 - 3.83 (m, 4H), 3.68 (dd, *J* = 6.7, 4.1 Hz, 2H), 2.94 (d, *J=* 6.0 Hz, 10H), 2.69 - 2.59 (m, 1H), 2.39 (dd, *J =* 13.8, 6.2 Hz, 1H).

³¹P NMR (162 MHz, dmso) δ -10.49 (d, *J =* 19.5 Hz), -12.86 (d, *J* = 24.3 Hz), -22.97 (dd, *J* = 24.9, 19.7 Hz).

MGI443-V1-dCTP
LCMS: calcd for C₄₈H₅₀F₂N₁₅O₂₅P₃S₂ [M-H]⁻:1430.17. Found, m/z, [(M-2)/2]⁻: 714.95.

¹H NMR (400 MHz, dmso) δ 8.97 (s, 1H), 8.79 (s, 1H), 8.65 - 8.58 (m, 1H), 8.15 (d, *J =* 8.0 Hz, 1H), 8.00 (s, 1H), 7.73 (s, 1H), 7.58 - 7.48 (m, 2H), 7.42 - 7.36 (m, 1H), 7.14 (dd, *J* = 8.2, 1.7 Hz, 1H), 6.96 (d, *J* = 11.6 Hz, 1H), 6.88 (s, 1H), 6.76 (d, *J* = 4.0 Hz, 1H), 6.14 - 6.09 (m, 1H), 5.19 - 5.13 (m, 1H), 4.84 (dd, *J =* 40.5, 8.8 Hz, 2H), 4.44 - 4.38 (m, 1H), 4.22 (ddd, *J =* 15.4, 10.4, 4.6 Hz, 2H), 4.12 (d, *J* = 4.9 Hz, 1H), 4.04 - 3.83 (m, 4H), 3.72 - 3.63 (m, 2H), 3.51 (s, 2H), 3.04 (d, *J* = 6.9 Hz, 6H), 2.91 (d, *J =* 6.9 Hz, 2H), 2.31 (ddd, *J =* 8.1, 6.3, 2.6 Hz, 1H), 2.19 - 2.13 (m, 1H).

³¹P NMR (162 MHz, dmso) δ -10.38 (d, *J =* 18.7 Hz), -13.29 (d, *J* = 25.4 Hz), -22.94 (dd, *J* = 25.0, 18.7 Hz).

MGI443-V1-dTTP
LCMS: calcd for C₄₈H₄₉F₂N₁₄O₂₆P₃S₂ [M-H]⁻:1431.16. Found, m/z, [(M-2)/2]⁻: 715.23.

¹H NMR (400 MHz, dmso) δ 8.98 (s, 1H), 8.82 (s, 1H), 8.77 - 8.70 (m, 1H), 8.16 (d, *J* = 8.2 Hz, 1H), 8.08 (s, 1H), 7.97 - 7.77 (m, 3H), 7.56 (d, *J =* 8.4 Hz, 1H), 7.50 (d, *J =* 7.8 Hz, 1H), 7.41 - 7.36 (m, 1H), 7.14 (d, *J* = 8.2 Hz, 1H), 6.96 (d, *J =* 11.7 Hz, 1H), 6.13 - 6.07 (m, 1H), 5.20 - 5.15 (m, 1H), 4.90 (d, *J* = 8.9 Hz, 1H), 4.80 (d, *J* = 8.9 Hz, 1H), 4.44 (d, *J* = 2.5 Hz, 1H), 4.27 (dd, *J* = 10.6, 4.1 Hz, 1H), 4.19 (dd, *J =* 10.6, 5.2 Hz, 1H), 4.13 - 4.04 (m, 2H), 4.02 - 3.83 (m, 4H), 3.67 (dd, *J* = 7.0, 5.4 Hz, 2H), 3.51 (s, 2H), 2.97 (dd, *J =* 45.2, 6.5 Hz, 8H), 2.34 - 2.28 (m, 1H).

³¹P NMR (162 MHz, dmso) δ -10.35 (d, *J* = 18.5 Hz), -13.41 (d, *J =* 25.1 Hz), -22.70 - -23.05 (m).

MGI443-V1-dGTP
LCMS: calcd for C₅₀H₅₁F₂N₁₆O₂₅P₃S₂ [M-H]⁻:1469.18. Found, m/z, [(M-2)/2]⁻: 734.27.

¹H NMR (400 MHz, dmso) δ 10.45 (s, 1H), 9.00 (s, 1H), 8.80 (d, *J* = 4.2 Hz, 1H), 8.26 (dd, *J =* 12.0, 6.5 Hz, 1H), 8.14 (d, *J =* 8.1 Hz, 1H), 7.97 - 7.67 (m, 3H), 7.56 - 7.45 (m, 2H), 7.35 (dd, *J =* 13.7, 5.7 Hz, 1H), 7.17 (d, *J* = 3.4 Hz, 1H), 7.13 - 7.04 (m, 1H), 6.93 (d, *J* = 11.6 Hz, 1H), 6.37 (s, 2H), 6.19 (dd, *J* = 8.9, 5.8 Hz, 1H), 5.16 - 5.10 (m, 1H), 4.84 (dd, *J* = 23.9, 8.8 Hz, 2H), 4.50 (d, *J* = 4.8 Hz, 1H), 4.25 (dd, *J =* 10.4, 4.4 Hz, 1H), 4.16 (dd, *J* = 8.7, 4.9 Hz, 1H), 4.12 - 4.04 (m, 2H), 3.94 - 3.83 (m, 4H), 3.66 (dd, *J =* 8.4, 3.6 Hz, 2H), 3.16 (d, *J* = 5.4 Hz, 2H), 2.93 (d, *J* = 5.5 Hz, 10H), 2.65 - 2.53 (m, 1H), 2.33 - 2.21 (m, 1H).

³¹P NMR (162 MHz, dmso) δ -10.59 (t, *J* = 21.7 Hz), -12.93 (d, *J* = 25.7 Hz), -22.85 - -23.20 (m).

### Example 19 Synthesis of MGI488 modified nucleotide

The following compounds were synthesized in accordance with the method as described in Example 17:
MGI488-V1

LCMS: calcd for C₄₄H₅₁N₉O₁₄S₂ [M-H]⁻: 992.30. Found, m/z, [M-H]⁻: 992.49.

MGI488-V1-dATP
LCMS: calcd for C₅₉H₇₀N₁₇O₂₅P₃S₂ [M-H]⁻:1572.34. Found, m/z, [(M-2)/2]⁻: 785.91.

¹H NMR (400 MHz, dmso) δ 8.95 - 8.87 (m, 1H), 8.60 (s, 1H), 8.45 (dd, *J* = 15.6, 11.0 Hz, 2H), 8.09 (s, 1H), 8.03 (s, 1H), 7.67 (dd, *J* = 10.7, 5.9 Hz, 2H), 7.59 (dd, *J* = 14.4, 8.6 Hz, 1H), 7.44 (d, *J* = 9.0 Hz, 2H), 7.35 (dd, *J* = 13.9, 6.4 Hz, 1H), 7.17 (d, *J* = 9.4 Hz, 1H), 7.09 (dd, *J* = 16.1, 9.0 Hz, 2H), 6.52 - 6.46 (m, 1H), 5.18 - 5.11 (m, 1H), 4.89 (dd, *J* = 28.8, 8.9 Hz, 2H), 4.56 (d, *J* = 5.1 Hz, 1H), 4.24 (dd, *J* = 10.0, 4.6 Hz, 1H), 4.18 - 4.11 (m, 2H), 3.98 (s, 1H), 3.95 - 3.84 (m, 2H), 3.69 (d, *J* = 4.2 Hz, 1H), 3.49 (s, 2H), 3.17 (s, 3H), 2.97 (s, 8H), 2.62 (d, *J* = 8.8 Hz, 1H), 2.39 (dd, *J =* 12.7, 6.3 Hz, 1H), 2.04 - 1.97 (m, 1H), 1.66 (dd, *J* = 22.4, 12.5 Hz, 2H), 1.28 - 1.14 (m, 6H).

³¹P NMR (162 MHz, dmso) δ -10.05 (d, *J* = 18.2 Hz), -13.38 (dd, *J* = 25.9, 2.8 Hz), -22.38 - -22.61 (m).

MGI488-V1-dCTP
LCMS: calcd for C₅₇H₆₉N₁₆O₂₆P₃S₂ [M-H]⁻:1549.32. Found, m/z, [(M-2)/2]⁻: 774.30.

¹H NMR (400 MHz, dmso) δ 8.93 - 8.87 (m, 1H), 8.67 - 8.61 (m, 1H), 8.56 - 8.48 (m, 1H), 8.11 - 7.97 (m, 2H), 7.76 - 7.65 (m, 2H), 7.63 - 7.51 (m, 2H), 7.41 (ddd, *J* = 22.8, 13.9, 5.8 Hz, 3H), 7.17 (d, *J* = 9.4 Hz, 1H), 7.13 (d, *J* = 7.8 Hz, 1H), 7.08 (d, *J* = 9.6 Hz, 1H), 6.89 (s, 1H), 6.15 - 6.10 (m, 1H), 5.16 (d, *J =* 4.2 Hz, 1H), 4.91 (d, *J =* 8.8 Hz, 1H), 4.80 (d, *J* = 8.8 Hz, 1H), 4.44 - 4.37 (m, 1H), 4.28 - 4.21 (m, 1H), 4.20 - 4.09 (m, 2H), 4.00 - 3.91 (m, 2H), 3.89 - 3.82 (m, 1H), 3.67 (d, *J =* 7.4 Hz, 1H), 3.17 (s, 3H), 3.05 - 2.87 (m, 8H), 2.79 (s, 1H), 2.29 (s, 1H), 2.01 (s, 1H), 1.71 (s, 1H), 1.62 (s, 1H), 1.23 (dd, *J* = 8.9, 5.4 Hz, 3H), 1.16 (s, 3H).

³¹P NMR (162 MHz, dmso) δ -10.15 (d, *J =* 18.6 Hz), -13.31 (d, *J* = 24.6 Hz), -22.68 (dd, *J* = 23.5, 18.8 Hz).

### Example 20 Synthesis of MGI447 modified nucleotide

### (1) Synthesis of MGI447-V1-dATP

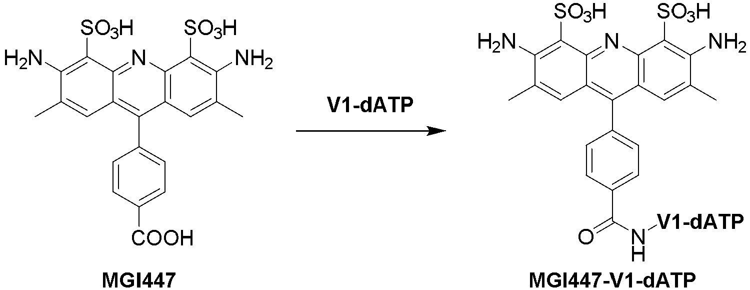

In a 4 mL sample vial, MGI447 (5 mg, 9.66 µmol) was dissolved with anhydrous N,N-dimethylformamide (1 mL), then N,N'-disuccinimidyl carbonate (5 mg, 19.52 µmol) and 4-dimethylaminopyridine (2 mg, 16.37 µmol) were added, followed by stirring magnetically at 25°C for 2 hours. Then V1-dATP (18 mg, 18.99 µmol) and triethylamine (5 mg, 49.41 µmol) were added, followed by further stirring magnetically at 25°C for 4 hours. The reaction solution was filtered, first the filtrate was purified by flash preparative liquid chromatography (0.1 M TEAB/acetonitrile), and the prepared solution was concentrated under reduced pressure to obtain a crude product. Then the crude product was purified by preparative liquid chromatography (0.1 M TEAB/acetonitrile), and the prepared solution was concentrated under reduced pressure, and freeze-dried to obtain MGI447-V1-dATP. LCMS: calcd for C₅₂H₅₇N₁₆O₂₄P₃S₂ [M-H]⁻:1445.24. Found, m/z, [(M-2)/2]⁻: 722.32.

¹H NMR (400 MHz, dmso) δ 9.02 (s, 1H), 8.82 (s, 1H), 8.45 - 8.38 (m, 1H), 8.15 (d, *J* = 8.2 Hz, 1H), 8.09 (s, 1H), 7.71 (d, *J* = 1.7 Hz, 1H), 7.50 (dd, *J* = 16.2, 8.2 Hz, 2H), 7.42 - 7.35 (m, 1H), 7.20 (s, 1H), 7.12 (d, *J =* 9.0 Hz, 1H), 6.97 (s, 1H), 6.65 (s, 1H), 6.49 (dd, *J =* 8.8, 5.8 Hz, 1H), 5.35 - 5.30 (m, 1H), 5.16 (t, *J* = 4.8 Hz, 1H), 4.89 (dd, *J* = 26.3, 8.9 Hz, 2H), 4.56 (s, 1H), 4.26 (dd, *J =* 10.4, 4.4 Hz, 1H), 4.21 - 4.12 (m, 2H), 3.98 - 3.84 (m, 4H), 3.68 (s, 2H), 3.51 (s, 2H), 2.94 (d, *J =* 6.1 Hz, 10H), 2.63 (dd, *J =* 18.3, 9.6 Hz, 1H), 2.39 (dd, *J =* 12.5, 6.1 Hz, 1H), 2.12 (s, 3H), 2.03 - 1.95 (m, 1H), 1.23 (s, 3H).

³¹P NMR (162 MHz, dmso) δ -10.49 (d, *J =* 19.2 Hz), -12.96 (d, *J* = 24.9 Hz), -22.98 (dd, *J* = 25.1, 19.5 Hz).

The following compounds were synthesized in accordance with the same method as described above:
MGI447-V1-dCTP

LCMS: calcd for C₅₀H₅₅N₁₄O₂₆P₃S₂ [M-H]⁻:1423.21. Found, m/z, [(M-2)/2]⁻: 711.21.

¹H NMR (400 MHz, dmso) δ 9.03 (s, 1H), 8.86 (s, 1H), 8.71 - 8.62 (m, 1H), 8.15 (d, *J* = 8.1 Hz, 1H), 8.02 (s, 1H), 7.72 (s, 1H), 7.54 (d, *J* = 12.9 Hz, 1H), 7.50 - 7.46 (m, 1H), 7.42 - 7.35 (m, 1H), 7.14 (d, *J =* 6.5 Hz, 1H), 6.97 (s, 1H), 6.88 (s, 1H), 6.16 - 6.08 (m, 1H), 5.17 (t, *J* = 4.6 Hz, 1H), 4.89 (d, *J* = 8.8 Hz, 1H), 4.80 (d, *J* = 8.9 Hz, 1H), 4.40 (s, 1H), 4.27 (dd, *J* = 10.6, 4.0 Hz, 1H), 4.19 (dd, *J =* 10.7, 5.0 Hz, 1H), 4.11 (d, *J* = 4.8 Hz, 1 H), 3.91 (dt, *J* = 11.6, 8.1 Hz, 4H), 3.67 (d, *J* = 9.0 Hz, 2H), 3.52 (s, 2H), 2.92 (s, 10H), 2.31 - 2.29 (m, 1H), 2.12 (s, 3H), 2.04 - 1.95 (m, 1H), 1.23 (s, 3H).

³¹P NMR (162 MHz, dmso) δ -10.33 (d, *J =* 19.1 Hz), -13.20 (d, *J* = 25.7 Hz), -22.83 (dd, *J* = 23.8, 18.4 Hz).

MGI447-V1-dTTP
LCMS: calcd for C₅₀H₅₆N₁₅O₂₅P₃S₂ [M-H]⁻:1422.22. Found, m/z, [(M-2)/2]⁻: 711.18.

¹H NMR (400 MHz, dmso) δ 9.03 (s, 1H), 8.88 (s, 1H), 8.79 (s, 1H), 8.15 (d, *J =* 8.2 Hz, 1H), 8.10 (s, 1H), 7.56 (s, 1H), 7.53 - 7.45 (m, 2H), 7.42 - 7.36 (m, 1H), 7.14 (d, *J* = 8.2 Hz, 1H), 6.97 (s, 1H), 6.14 - 6.07 (m, 1H), 5.18 (d, *J* = 4.3 Hz, 1H), 4.90 (d, *J =* 8.9 Hz, 1H), 4.80 (d, *J =* 8.9 Hz, 1H), 4.45 (s, 1H), 4.31 - 4.26 (m, 1H), 4.20 (dd, *J =* 10.5, 5.4 Hz, 1H), 4.09 (d, *J =* 18.0 Hz, 2H), 4.01 - 3.82 (m, 4H), 3.67 (s, 2H), 3.52 (s, 2H), 2.94 (s, 10H), 2.30 (d, *J =* 8.0 Hz, 1H), 2.12 (s, 3H), 2.00 (d, *J =* 8.0 Hz, 1H), 1.23 (s, 3H).

³¹P NMR (162 MHz, dmso) δ -10.30 (d, *J =* 18.7 Hz), -13.32 (d, *J* = 24.8 Hz), -22.78 (dd, *J* = 24.8, 18.8 Hz).

MGI447-V1-dGTP
LCMS: calcd for C₅₂H₅₇N₁₆O₂₅P₃S₂ [M-H]⁻:1461.23. Found, m/z, [(M-2)/2]⁻: 730.29.

¹H NMR (400 MHz, dmso) δ 10.47 (s, 1H), 9.01 (s, 1H), 8.82 (d, *J =* 4.4 Hz, 1H), 8.33 - 8.25 (m, 1H), 8.15 (d, *J =* 8.1 Hz, 1H), 7.50 (dd, *J =* 13.9, 7.3 Hz, 2H), 7.38 (t, *J* = 8.1 Hz, 1H), 7.19 (s, 1H), 7.13 (d, *J* = 6.7 Hz, 1H), 6.97 (s, 1H), 6.65 (s, 1H), 6.39 (s, 1H), 6.21 (dd, *J* = 9.1, 5.6 Hz, 1H), 5.17 (t, *J =* 4.8 Hz, 1H), 4.87 (dd, *J* = 25.1, 8.8 Hz, 2H), 4.52 (d, *J =* 5.2 Hz, 1H), 4.27 (dd, *J* = 10.4, 4.4 Hz, 1H), 4.19 (dd, *J* = 9.6, 5.8 Hz, 1H), 4.15 - 4.03 (m, 2H), 3.98 - 3.81 (m, 4H), 3.72 - 3.63 (m, 2H), 3.52 (s, 2H), 2.94 (s, 10H), 2.64 - 2.57 (m, 1H), 2.30 - 2.25 (m, 1H), 2.12 (s, 3H), 2.03 - 1.94 (m, 1H), 1.23 (s, 3H).

³¹P NMR (162 MHz, dmso) δ -10.38 (d, *J =* 18.9 Hz), -13.06 (d, *J* = 26.4 Hz), -22.90 (dd, *J* = 25.2, 18.8 Hz).

### Example 21 Synthesis of MGI456 modified nucleotide

The following compounds were synthesized in accordance with the method as described in Example 20:
MGI456-V1-dATP

LCMS: calcd for C₅₄H₅₅F₆N₁₆O₂₄P₃S₂ [M-H]⁻:1581.21. Found, m/z, [(M-2)/2]⁻: 790.71.

¹H NMR (400 MHz, dmso) δ 9.41 (t, J = 6.6 Hz, 1H), 9.07 (s, 1H), 8.86 (s, 1H), 8.45 - 8.36 (m, 1H), 8.17 (d, J = 8.1 Hz, 1H), 8.09 (s, 1H), 7.71 (d, J = 2.3 Hz, 1H), 7.57 (d, J = 8.3 Hz, 1H), 7.53 (d, J = 8.5 Hz, 1H), 7.39 (dd, J = 17.0, 8.6 Hz, 2H), 7.28 (d, J = 9.6 Hz, 1H), 7.11 (d, J = 8.5 Hz, 1H), 6.49 (dd, J = 8.5, 5.7 Hz, 1H), 5.16 (t, J = 4.8 Hz, 1H), 4.88 (dd, J = 26.5, 8.9 Hz, 2H), 4.57 (d, J = 6.0 Hz, 1H), 4.53 - 4.42 (m, 2H), 4.26 (dd, J = 10.4, 4.7 Hz, 1H), 4.21 - 4.11 (m, 2H), 4.00 - 3.80 (m, 4H), 3.68 (dd, J = 6.6, 4.4 Hz, 2H), 3.52 (s, 2H), 2.85 (s, 8H), 2.63 - 2.60 (m, 1H), 2.41 - 2.36 (m, 1H), 2.00 (dd, J = 15.2, 6.6 Hz, 1H).

¹⁹F NMR (376 MHz, dmso) δ -70.45 (t, *J =* 9.4 Hz).

³¹P NMR (162 MHz, dmso) δ -10.48 (d, *J=* 19.5 Hz), -12.83 (d, *J* = 25.1 Hz), -22.90 (dd, *J* = 25.0, 19.5 Hz).

MGI456-V1-dCTP
LCMS: calcd for C₅₂H₅₃F₆N₁₄O₂₆P₃S₂ [M-H]⁻:1559.18. Found, m/z, [(M-2)/2]⁻: 778.96.

¹H NMR (400 MHz, dmso) δ 9.43 - 9.35 (m, 1H), 9.02-8.959 (m, 1H), 8.84-8.792 (m, 1H), 8.67-8.61 (m, 1H), 8.14 (d, *J =* 8.0 Hz, 1H), 7.99 (s, 1H), 7.71-7.66 (m, 1H), 7.56-7.47 (m, 2H), 7.41 - 7.33 (m, 1H), 7.26 (d, *J* = 9.5 Hz, 1H), 7.15 (d, *J* = 13.7 Hz, 1H), 6.87-6.83 (m, 1H), 6.65-6.59 (m, 1H), 6.12-6.07 (m, 1H), 5.30 (t,, *J* = 4.0 Hz, 1H), 5.17-5.13 (m, 1H), 4.87 (d, *J =* 8.8 Hz, 1H), 4.78 (d, *J* = 8.8 Hz, 1H), 4.51-4.42 (m, 2H), 4.27 - 4.17 (m, 1H), 4.12-4.06 (m, 1H), 3.99-3.81 (s, 3H), 3.66-3.60 (m, 1H), 3.54-3.45 (m, 1H), 3.04-2.74 (m, 6H), 1.96 - 1.89 (m, 1H), 1.47-1.40 (m, 1H).

¹⁹F NMR (376 MHz, dmso) δ -70.45 (t, *J =* 9.4 Hz).

³¹P NMR (162 MHz, dmso) δ -9.67 - -10.31 (m), -13.02 - -13.53 (m), -22.26 - -22.81 (m).

MGI456-V1-dTTP
LCMS: calcd for C₅₂H₅₄F₆N₁₅O₂₅P₃S₂ [M-H]⁻:1558.20. Found, m/z, [(M-2)/2]⁻: 779.04.

¹H NMR (400 MHz, dmso) δ 9.40-9.36 (m, 1H), 9.03 - 8.94 (m, 1H), 8.85 - 8.79 (m, 1H), 8.77 - 8.70 (m, 1H), 8.14 (d, *J* = 8.4 Hz, 1H), 8.07 (s, 1H), 7.56-7.46 (m, 2H), 7.42 - 7.09 (m, 4H), 6.66 - 6.57 (m, 1H), 6.10-6.06 (m, 1H), 5.31-5.29 (m, 1H), 5.21 - 5.12 (m, 1H), 4.88 (d, *J* = 9.1 Hz, 1H), 4.77 (d, *J =* 9.1 Hz, 1H),, 4.51-4.42 (m, 2H), 4.5-3.84 (m, 6H), 3.68-3.61 (m, 1H), 3.53-3.47 (m, 1H), 3.06-2.80 (s, 6H), 1.97 - 1.93 (m, 1H), 1.45-1.40 (m, 1H).

¹⁹F NMR (376 MHz, dmso) δ -70.45 (t, *J=* 9.3 Hz).

³¹P NMR (162 MHz, dmso) δ -9.82 - -10.46 (m), -12.89 - -13.69 (m), -22.07 - -22.74 (m).

MGI456-V1-dGTP
LCMS: calcd for C₅₄H₅₅F₆N₁₆O₂₅P₃S₂ [M-H]⁻:1597.21. Found, m/z, [(M-2)/2]⁻: 798.71.

¹H NMR (400 MHz, dmso) δ 10.48 (s, 1H), 9.40 (t, *J* = 6.9 Hz, 1H), 9.04 (s, 1H), 8.85 (s, 1H), 8.33 - 8.23 (m, 1H), 8.16 (d, *J =* 8.1 Hz, 1H), 7.57 (d, *J =* 8.3 Hz, 1H), 7.52 (d, *J =* 6.4 Hz, 1H), 7.39 (dd, *J =* 18.0, 9.0 Hz, 2H), 7.28 (d, *J =* 9.6 Hz, 1H), 7.19 (s, 1H), 7.13 (d, *J =* 7.4 Hz, 1H), 6.40 (s, 1H), 6.21 (dd, *J* = 9.2, 5.7 Hz, 1H), 5.17 (t, *J* = 4.7 Hz, 1H), 4.87 (dd, *J =* 24.2, 8.8 Hz, 2H), 4.55 - 4.43 (m, 2H), 4.27 (dd, *J* = 10.4, 4.0 Hz, 1H), 4.19 (dd, *J* = 10.2, 4.5 Hz, 1H), 4.14 - 4.03 (m, 2H), 4.00 - 3.79 (m, 4H), 3.68 (dd, *J =* 8.5, 3.4 Hz, 2H), 3.52 (s, 2H), 2.93 (s, 10H), 2.66 - 2.56 (m, 1H), 2.28 (dd, *J =* 13.1, 6.3 Hz, 1H), 2.04 - 1.94 (m, 1H).

¹⁹F NMR (376 MHz, dmso) δ -70.45 (t, *J =* 9.4 Hz).

³¹P NMR (162 MHz, dmso) δ -10.48 (d, *J* = 19.0 Hz), -12.88 (d, *J =* 26.0 Hz), -22.76 - -23.21 (m).

### Example 22 Synthesis of MGI485 modified nucleotide

The following compounds were synthesized in accordance with the method as described in Example 20:
MGI485-V1-dATP

LCMS: calcd for C₆₂H₇₃N₁₆O₂₄P₃S₂ [M-H]⁻:1581.36. Found, m/z, [(M-2)/2]⁻: 790.38.

¹H NMR (400 MHz, dmso) δ 9.29 (s, 1H), 8.98 (s, 1H), 8.78 (s, 1H), 8.46 - 8.39 (m, 1H), 8.15 (d, *J* = 8.3 Hz, 1H), 8.09 (s, 1H), 7.70 (s, 1H), 7.51 (d, *J* = 8.2 Hz, 1H), 7.41 - 7.33 (m, 1H), 7.19 (s, 1H), 7.12 (d, *J =* 7.9 Hz, 1H), 7.01 (s, 1H), 6.66 (s, 1H), 6.54 - 6.46 (m, 1H), 5.18 - 5.12 (m, 1H), 4.89 (dd, *J* = 28.5, 8.8 Hz, 2H), 4.55 (s, 1H), 4.25 (dd, *J =* 10.3, 4.2 Hz, 1H), 4.16 (dd, *J* = 18.4, 5.3 Hz, 2H), 4.02 - 3.83 (m, 3H), 3.68 (s, 1H), 3.51 (s, 2H), 2.98 (s, 8H), 2.64 - 2.59 (m, 1H), 2.41 - 2.35 (m, 1H), 2.03 - 1.95 (m, 1H), 1.82 (d, *J* = 8.0 Hz, 1H), 1.52 - 1.12 (m, 12H), 1.12 - 0.93 (m, 6H).

³¹P NMR (162 MHz, dmso) δ -10.07 (d, *J =* 18.0 Hz), -13.31 (d, *J* = 26.4 Hz), -22.57 (dd, *J* = 25.7, 18.1 Hz).

MGI485-V1-dCTP
LCMS: calcd for C₆₀H₇₁N₁₄O₂₆P₃S₂ [M-H]⁻:1559.33. Found, m/z, [(M-2)/2]⁻: 778.84.

¹H NMR (400 MHz, dmso) δ 9.27 (s, 1H), 8.97 (s, 1H), 8.79 (s, 1H), 8.62 (s, 1H), 8.14 (dd, *J* = 7.8, 4.8 Hz, 1H), 7.99 (s, 1H), 7.70 (s, 1H), 7.52 - 7.45 (m, 1H), 7.40 - 7.33 (m, 1H), 7.16 - 7.08 (m, 1H), 6.99 (s, 1H), 6.84 (s, 1H), 6.62 (s, 1H), 6.14 - 6.05 (m, 1H), 5.30 (s, 1H), 5.15 (s, 1H), 4.83 (dd, *J =* 42.4, 9.1 Hz, 2H), 4.41 - 4.35 (m, 1H), 4.29 - 4.20 (m, 1H), 4.08 (s, 2H), 3.95 (s, 3H), 3.66 (s, 1H), 3.49 (s, 2H), 2.96 (s, 6H), 2.63 - 2.57 (m, 1H), 2.29 - 2.23 (m, 1H), 2.01 - 1.94 (m, 1H), 1.79 (s, 1H), 1.21 (dd, *J =* 41.8, 34.1 Hz, 12H), 1.07 - 0.67 (m, 6H).

³¹P NMR (162 MHz, dmso) δ -9.96 (d, *J* = 18.0 Hz), -13.41 (d, *J* = 4.1 Hz), -22.31 (d, *J* = 2.8 Hz).

### Example 23 Synthesis of MGI450d modified nucleotide

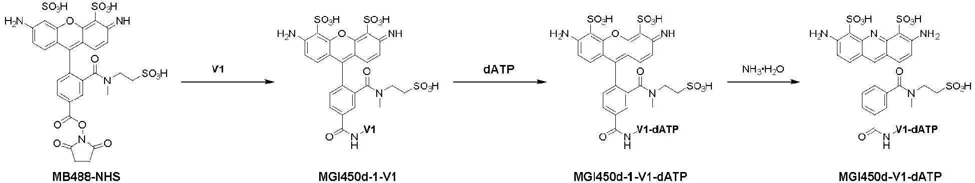

### (1) Synthesis of MGI450d-1-V1

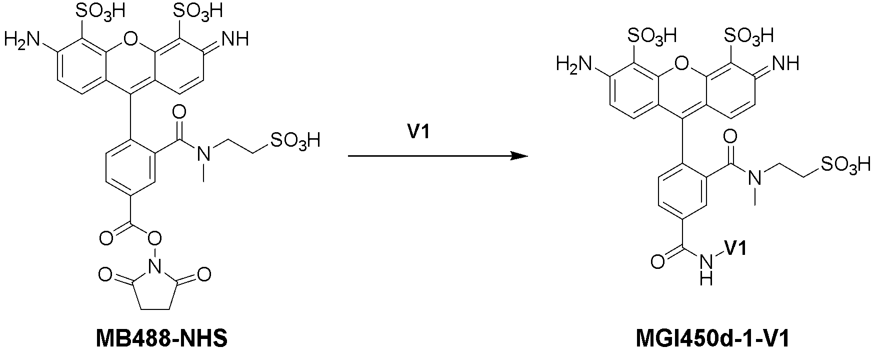

In a 15 mL sample vial, MB488 NHS (50 mg, 66.43 µmol) was dissolved with anhydrous N,N-dimethylformamide (5 mL), then V1 (49 mg, 133.38 µmol) and N,N-diisopropylethylamine (26 mg, 201.17 µmol) were added, followed by stirring magnetically at 24°C for 15 hours. The reaction solution was filtered, the filtrate was first purified by flash preparative liquid chromatography (0.1 M TEAB/acetonitrile), and then purified by flash preparative liquid chromatography (0.1% triethylamine/acetonitrile), and the prepared solution was concentrated under reduced pressure, and freeze-dried to obtain MGI450d-1-V1. LCMS: calcd for C₃₉H₄₀N₈O₁₈S₃ [M-H]⁻: 1003.16 Found, m/z, [(M-2)/2]⁻: 501.02.

### (2) Synthesis of MGI450d-1-V1-dATP

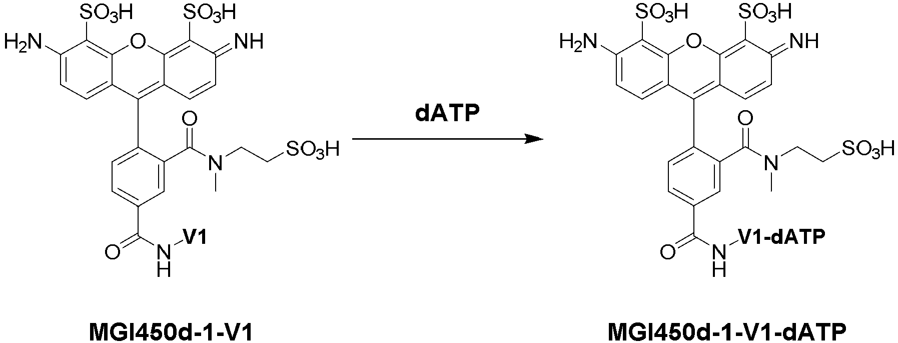

In a 4 mL sample vial, MGI450d-1-V1 (10 mg, 9.95 µmol) was dissolved with anhydrous N,N-dimethylformamide (1 mL), then N,N'-disuccinimidyl carbonate (5 mg, 19.52 µmol) and 4-dimethylaminopyridine (2 mg, 16.37 µmol) were added, followed by stirring magnetically at 25°C for 2 hours. Then dATP (12 mg, 20.06 µmol) and triethylamine (5 mg, 49.41 µmol) were added, followed by further stirring magnetically at 25°C for 4 hours. The reaction solution was filtered, the filtrate was purified by flash preparative liquid chromatography (0.1 M TEAB/acetonitrile), and the prepared solution was concentrated under reduced pressure to obtain MGI450d-1-V1-dATP. LCMS: calcd for C₅₄H₅₉N₁₆O₂₉P₃S₃ [M-H]⁻:1583.20. Found, m/z, [(M-2)/2]⁻: 791.25.

The following compounds were synthesized in accordance with the same method as described above:
MGI450d-1-V1-dTTP
LCMS: calcd for C₅₂H₅₇N₁₄O₃₁P₃S₃ [M-H]⁻:1561.17. Found, m/z, [(M-2)/2]⁻: 780.23.
MGI450d-1-V1-dCTP
LCMS: calcd for C₅₂H₅₈N₁₅O₃₀P₃S₃ [M-H]⁻:1560.18. Found, m/z, [(M-2)/2]⁻: 779.82.
MGI450d-1-V1-dGTP
LCMS: calcd for C₅₄H₅₉N₁₆O₃₀P₃S₃ [M-H]⁻:1599.20. Found, m/z, [(M-2)/2]⁻: 799.65.

### (3) Synthesis of MGI450d-V1-dATP

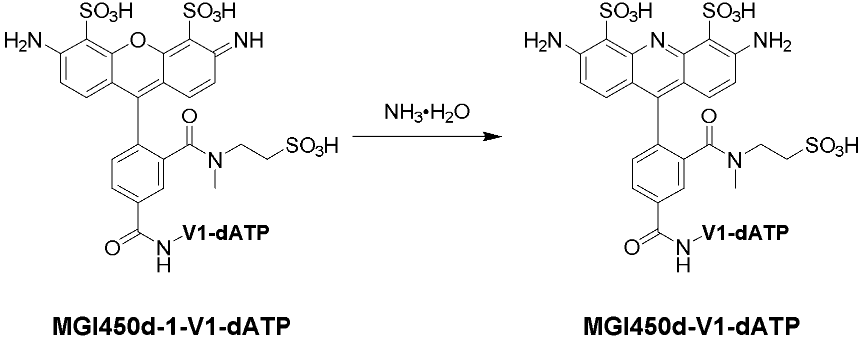

In a 15 mL sample vial, the compound MGI450d-1-V1-dATP (20 mg) was dissolved with methanol (2 mL), then ammonia (2 mL) was added, followed by stirring magnetically at 25°C for 15 hours. The reaction solution was concentrated under reduced pressure to obtain a crude product, the crude product was purified by preparative HPLC (0.1 M TEAB/acetonitrile), and the prepared solution was concentrated under reduced pressure, and freeze-dried to obtain the compound MGI450d-V1-dATP. LCMS: calcd for C₅₄H₅₉N₁₇O₂₈P₃S₃ [M-H]⁻: 1581.21. Found, m/z, [M/2-H]⁻: 790.78.

¹H NMR (400 MHz, dmso) δ 9.06 (s, 1H), 8.70 (s, 1H), 8.42 (s, 1H), 8.23 (s, 1H), 8.08 (s, 1H), 8.01 (d, *J* = 8.5 Hz, 1H), 7.97 - 7.70 (m, 3H), 7.68 (s, 1H), 7.46 (dd, *J* = 21.3, 10.6 Hz, 2H), 7.35 (s, 1H), 7.22 - 7.14 (m, 1H), 7.09 (s, 1H), 6.97 (dd, *J* = 20.9, 9.3 Hz, 2H), 6.63 (s, 1H), 6.48 (d, *J =* 5.7 Hz, 1H), 5.30 (d, *J* = 4.6 Hz, 1H), 5.13 (s, 1H), 4.87 (dd, *J =* 29.5, 8.9 Hz, 2H), 4.54 (s, 1H), 4.22 (s, 1H), 4.12 (d, *J* = 5.1 Hz, 2H), 3.96 (t, *J* = 22.4 Hz, 3H), 3.66 (s, 1H), 3.49 (s, 2H), 2.95 (d, *J =* 14.4 Hz, 6H), 2.63 - 2.56 (m, 1H), 2.38 (s, 1H), 1.97 (t, *J* = 7.0 Hz, 1H), 1.21 (s, 3H).

³¹P NMR (162 MHz, dmso) δ 74.99 (s), -10.12 (d, *J* = 18.1 Hz), -13.35 (d, *J* = 26.3 Hz), - 22.66 (dd, *J =* 26.2, 18.2 Hz).

The following compounds were synthesized in accordance with the same method as described above:
MGI450d-V1-dTTP
LCMS: calcd for C₅₂H₅₇N₁₅O₃₀P₃S₃ [M-H]⁻: 1559.18. Found, m/z, [M/2-H]⁻: 779.47.
MGI450d-V1-dCTP
LCMS: calcd for C₅₂H₅₈N₁₆O₂₉P₃S₃ [M-H]⁻: 1558.19. Found, m/z, [M/2-H]⁻: 778.76.
MGI450d-V1-dGTP
LCMS: calcd for C₅₄H₅₉N₁₇O₂₉P₃S₃ [M-H]⁻: 1598.21. Found, m/z, [M/2-H]⁻: 798.49.

### Example 24 Synthesis of MGI471 modified nucleotide

### (1) Synthesis of MGI471-V1-dATP

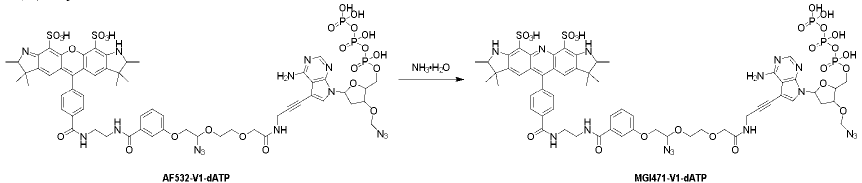

In a 4 mL sample vial, the compound AF532-V1-dATP (10 mg, 15.96 µmol) was dissolved with methanol (0.5 mL), then ammonia (0.5 mL) was added, followed by stirring magnetically at 22°C for 15 hours. The reaction solution was concentrated under reduced pressure to obtain a crude product, the crude product was purified by preparative HPLC (0.1% ammonia/acetonitrile), and the prepared solution was concentrated under reduced pressure, and freeze-dried to obtain the compound MGI471-V1-dATP.

¹H NMR (600 MHz, D₂O): δ 7.92 (s, 1H), 7.73-7.68 (m, 2H), 7.43 (d, *J* = 12.2 Hz, 1H), 7.04-6.93 (m, 4H), 6.87 (d, *J =* 13.7 Hz, 1H), 6.63-6.55 (m, 1H), 6.50 (s, 2H), 6.25-6.14 (m, 1H), 4.47-4.42 (m, 1H), 4.17 (s, 1H), 4.05-3.96 (m, 4H), 3.94-3.68 (m, 6H), 3.67-3.34 (m, 12H), 3.05 (q, *J* = 7.3 Hz, 2H), 2.36-2.27 (m, 2H), 1.11(t, *J* = 7.3 Hz, 3H), 0.94-0.91 (m, 6H), 0.79-0.76 (m, 6H), 0.66 - 0.62 (m, 6H).

³¹P NMR (243 MHz, D2O) δ -10.77 (d, *J =* 19.2 Hz), -11.36 (d, *J =* 19.5 Hz), -23.13 (t, *J =* 19.4 Hz).

LCMS: calcd for C₆₀H₆₉N₁₆O₂₄P₃S₂ [M-H]⁻: 1553.33. Found, m/z, [M-2/2]⁻: 776.31.

### (2) Synthesis of MGI471-V1-dGTP

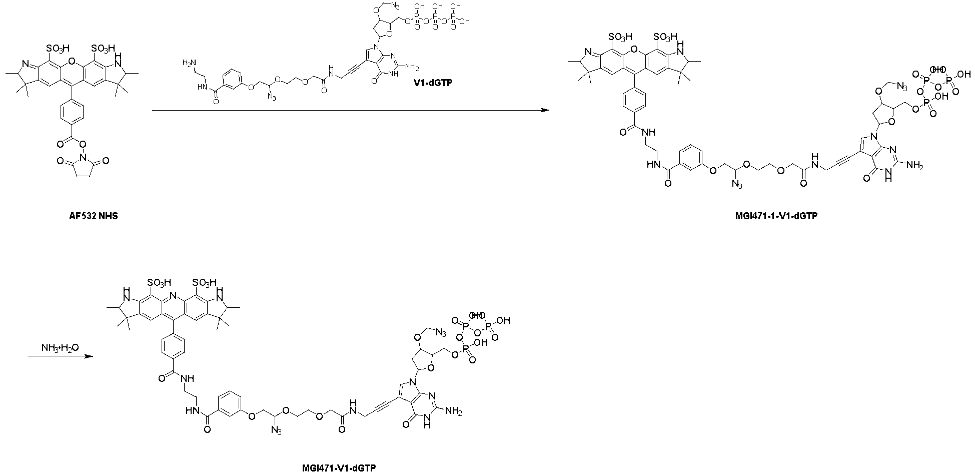

### (2-1) Synthesis of MGI471-1-V1-dGTP

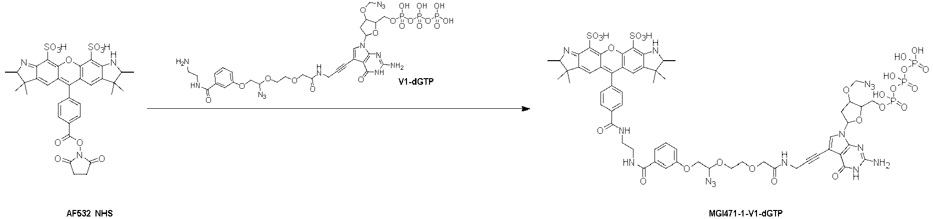

In a 15 mL sample vial, the compound V1-dGTP (40 mg, 41.51 µmol) was dissolved with anhydrous N,N-dimethylformamide (4 mL), then the compound AF532 NHS (30 mg, 41.45 µmol), N,N-diisopropylethylamine (22 mg, 170.22 µmol) and 4-dimethylaminopyridine (11 mg, 81.85 µmol) were added, followed by stirring magnetically at 25°C for 15 hours. The reaction solution was filtered, the filtrate was purified by flash preparative liquid chromatography (0.1 M TEAB/acetonitrile), and the prepared solution was concentrated under reduced pressure to obtain the compound MGI471-1-V1-dGTP. LCMS: calcd for C₆₀H₆₈N₁₅O₂₆P₃S₂ [M-H]⁻: 1570.31. Found, m/z, [M-H]⁻: 1570.24.

### (2-2) Synthesis of MGI471-V1-dGTP

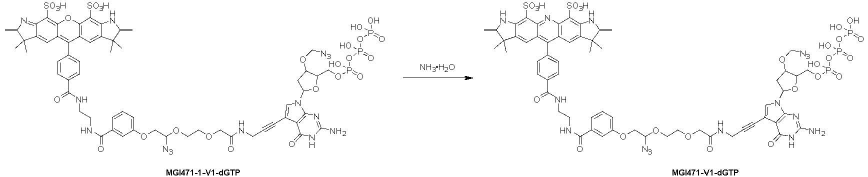

In a 15 mL sample vial, the compound MGI471-1-V1-dGTP (20 mg, 12.72 µmol) was dissolved with methanol (3 mL), then ammonia (3 mL) was added, followed by stirring magnetically at 24°C for 15 hours. The reaction solution was concentrated under reduced pressure to obtain a crude product, the crude product was purified by preparative HPLC (0.1% ammonia/acetonitrile), and the prepared solution was concentrated under reduced pressure, and freeze-dried to obtain the compound MGI471-V1-dGTP.

¹H NMR (600 MHz, D₂O) δ 7.81 (d, *J =* 7.7 Hz, 2H), 7.79-7.64 (m, 1H), 7.23 (d, *J =* 7.0 Hz, 2H), 7.18 - 7.10 (m, 2H), 6.97 - 6.94 (m, 1H), 6.90 (dt, *J* = 10.6, 2.9 Hz, 1H), 6.81 (d, *J* = 6.8 Hz, 1H), 6.69 (s, 2H), 6.57-6.44 (m, 1H), 5.92-5.81 (m, 1H), 4.84-4.71 (m, 1H), 4.37 (s, 1H), 4.12-4.07 m, 1H), 3.98 - 3.73 (m, 11H), 3.69 - 3.64 (m, 3H), 3.61 - 3.51 (m, 9H), 2.32-2.24 (m, 1H), 2.18-2.09 (m, 1H), 1.03 (d, *J =* 6.7 Hz, 6H), 0.94-0.91 (m, 6H), 0.80 (dd, *J =* 9.0, 5.0 Hz, 6H).

³¹P NMR (243 MHz, D₂O) δ -10.14, -11.41 (d, *J =* 20.2 Hz), -22.93 (t, *J* = 19.4 Hz).

LCMS: calcd for C₆₀H₆₉N₁₆O₂₅P₃S₂ [M-H]⁻: 1569.33. Found, m/z, [M-2/2]⁻: 785.10.

### (3) Synthesis of MGI471-V1-dCTP

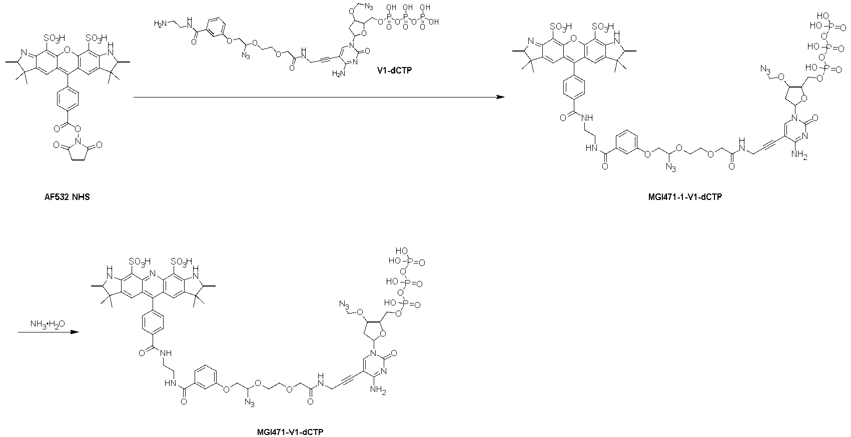

### (3-1) Synthesis of MGI471-1-V1-dCTP

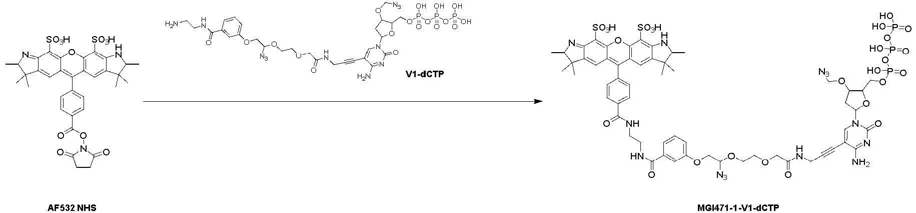

In a 15 mL sample vial, the compound V1-dCTP (40 mg, 43.26 µmol) was dissolved with anhydrous N,N-dimethylformamide (4 mL), then the compound AF532 NHS (32 mg, 44.21 µmol), N,N-diisopropylethylamine (22 mg, 170.22 µmol) and 4-dimethylaminopyridine (11 mg, 90.04 µmol) were added, followed by stirring magnetically at 25°C for 15 hours. The reaction solution was filtered, the filtrate was purified by flash preparative liquid chromatography (0.1 M TEAB/acetonitrile), and the prepared solution was concentrated under reduced pressure to obtain the compound MGI471-1-V1-dCTP. LCMS: calcd for C₅₈H₆₇N₁₄O₂₆P₃S₂ [M-H]⁻: 1531.30. Found, m/z, [M-H]⁻: 1532.24.

### (3-2) Synthesis of MGI471-V1-dCTP

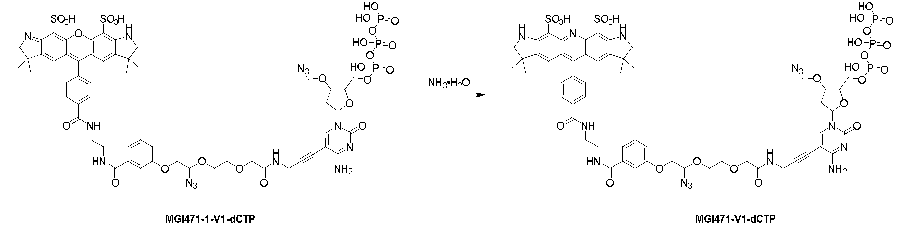

In a 15 mL sample vial, the compound MGI471-1-V1-dCTP (20 mg, 13.04 µmol) was dissolved with methanol (3 mL), then ammonia (3 mL) was added, followed by stirring magnetically at 24°C for 15 hours. The reaction solution was concentrated under reduced pressure to obtain a crude product, the crude product was purified by preparative HPLC (0.1% ammonia/acetonitrile), and the prepared solution was concentrated under reduced pressure, and freeze-dried to obtain the compound MGI471-1-V1-dCTP.

¹H NMR (600 MHz, D₂O): δ 7.82 (d, *J =* 7.9 Hz, 2H), 7.75 - 7.69 (m, 1H), 7.31 (d, *J =* 7.8 Hz, 2H), 7.23 - 7.15 (m, 2H), 6.99-7.02 (m, 1H), 6.89 (d, *J =* 8.3 Hz, 1H), 6.75 (s, 2H), 5.81 (dt, *J* = 26.2, 6.0 Hz, 1H), 4.78 (t, *J* = 4.9 Hz, 1H), 4.33 (s, 1H), 4.12 (s, 1H), 4.03 - 3.91 (m, 6H), 3.88 - 3.72 (m, 5H), 3.70 - 3.66 (m, 2H), 3.63 - 3.50 (m, 8H), 3.48 - 3.34 (m, 2H), 3.04 (q, *J =* 7.3 Hz, 3H), 2.29-2.36 (m, 1H), 2.01 - 1.94 (m, 1H), 1.12 (t, *J =* 7.3 Hz, 5H), 1.03 (d, *J =* 6.7 Hz, 6H), 0.97 - 0.94 (m, 6H), 0.83 (d, *J =* 7.1 Hz, 6H).

³¹P NMR (243 MHz, D₂O) δ -10.84 (d, *J* = 20.0 Hz), -11.63 (d, *J* = 20.1 Hz), -23.22 (t, *J* = 19.6 Hz).

LCMS: calcd for C₅₈H₆₈N₁₅O₂₅P₃S₂ [M-H]⁻: 1530.32. Found, m/z, [M-2/2]⁻: 764.85.

### (4) Synthesis of MGI471-V1-dTTP

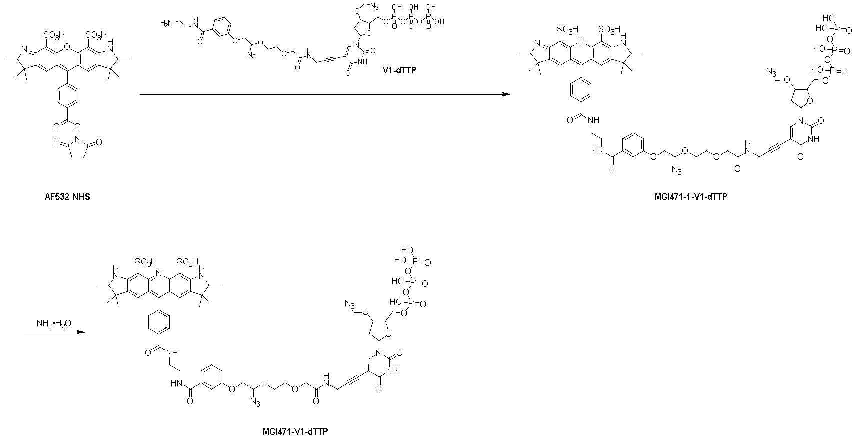

### (4-1) Synthesis of MGI471-1-V1-dTTP

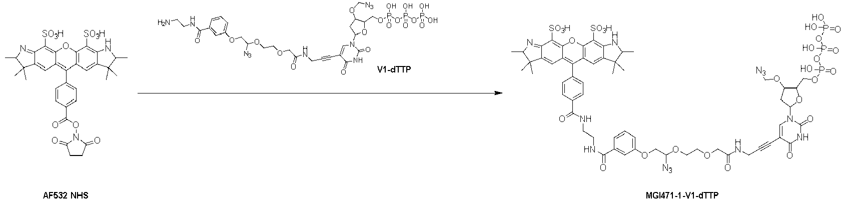

In a 15 mL sample vial, the compound V1-dTTP (40 mg, 43.22 µmol) was dissolved with anhydrous N,N-dimethylformamide (4 mL), then the compound AF532 NHS (32 mg, 22.21 µmol), N,N-diisopropylethylamine (22 mg, 170.22 µmol) and 4-dimethylaminopyridine (11 mg, 90.04 µmol) were added, followed by stirring magnetically at 25°C for 15 hours. The reaction solution was filtered, the filtrate was purified by flash preparative liquid chromatography (0.1 M TEAB/acetonitrile), and the prepared solution was concentrated under reduced pressure to obtain the compound MGI471-1-V1-dTTP.

LCMS: calcd for C₅₈H₆₆N₁₃O₂₇P₃S₂ [M-H]⁻: 1532.28. Found, m/z, [M-H]⁻: 1533.10.

### (4-2) Synthesis of MGI471-V1-dTTP

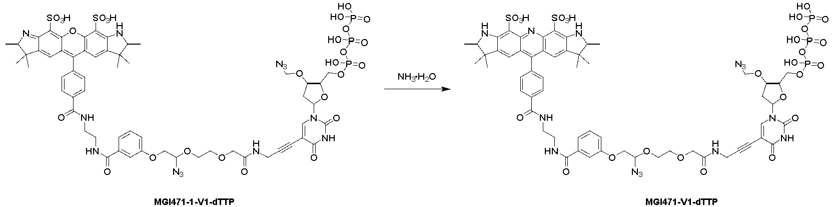

In a 15 mL sample vial, the compound MGI471-1-V1-dTTP (20 mg, 13.04 µmol) was dissolved with methanol (3 mL), then ammonia (3 mL) was added, followed by stirring magnetically at 24°C for 15 hours. The reaction solution was concentrated under reduced pressure to obtain a crude product, the crude product was purified by preparative HPLC (0.1% ammonia/acetonitrile), and the prepared solution was concentrated under reduced pressure, and freeze-dried to obtain the compound MGI471-V1-dTTP.

¹H NMR (600 MHz, D₂O): δ 7.88-7.81 (m, 2H), 7.74-7.71 (m, 1H), 7.27 (d, *J =* 7.3 Hz, 2H), 7.23-7.14 (m, 2H), 7.01-6.95 (m, 1H), 6.88 (d, *J* = 8.1 Hz, 1H), 6.73 (d, *J* = 3.1 Hz, 2H), 5.80 (dt, *J =* 28.9, 7.1 Hz, 1H), 4.78 (d, *J* = 5.0 Hz, 1H), 4.36 (s, 1H), 4.13 - 4.06 (m, 1H), 4.01-3.89 (m, 6H), 3.88 - 3.31 (m, 18H), 3.04 (q, *J =* 7.3 Hz, 1H), 2.35-2.26 (m, 1H), 2.13-2.05 (m, 1H), 1.12 (t, *J = 7.3* Hz, 2H), 1.02 (d, *J =* 6.7 Hz, 6H), 0.96-0.93 (m, 6H), 0.83-0.79 (m, 6H).

³¹P NMR (243 MHz, D₂O) δ -10.44, -11.54 (d, *J =* 19.1 Hz), -22.87.

LCMS: calcd for C₅₈H₆₇N₁₄O₂₆P₃S₂ [M-H]⁻: 1531.30. Found, m/z, [M-2/2]⁻: 765.26.

### Example 25 Synthesis of MGI471-V2

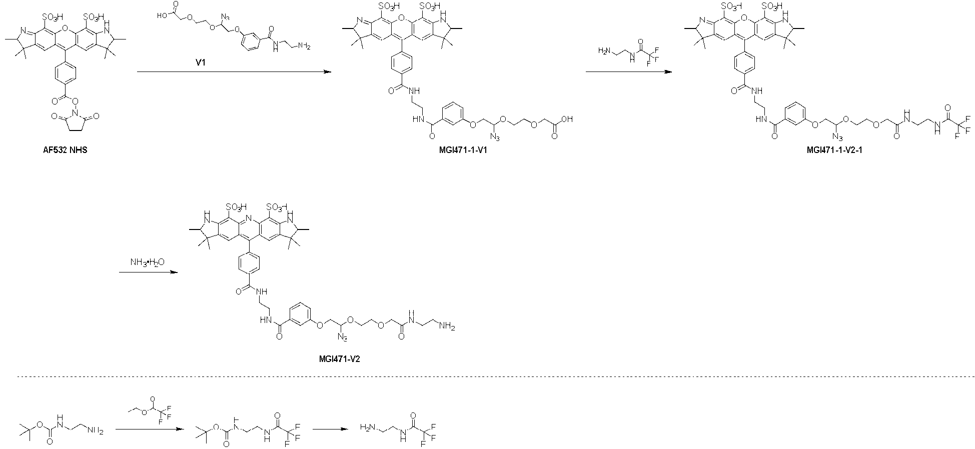

### (1) Synthesis of tert-butyl (2-(trifluoroacetamido)ethyl)carbamate

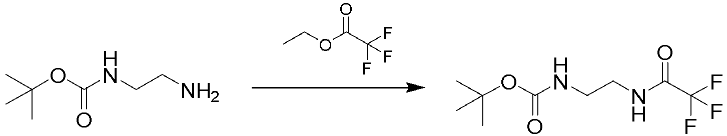

In a 100 mL flask, N-tert-butoxycarbonyl-1,2-ethanediamine (5 g, 31.21 mmol) was dissolved with tetrahydrofuran (30 mL). Under the conditions of an ice-water bath of 0-5°C, ethyl trifluoroacetate (4.88 g, 34.33 mmol) was added. After completion of the addition, the reaction solution was heated up to 24°C and stirred magnetically for 4 hours. The reaction solution was concentrated under reduced pressure to obtain tert-butyl (2-(trifluoroacetamido)ethyl)carbamate.

### (2) Synthesis of N-trifluoroacetyl-1,2-ethylenediamine

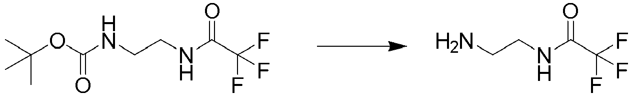

In a 100 mL flask, tert-butyl (2-(trifluoroacetamido)ethyl)carbamate (8 g, 31.21 mmol) was dissolved with formic acid (50 mL), and stirred magnetically at 24°C for 15 hours. The reaction solution was concentrated under reduced pressure, then acetonitrile (50 mL × 2) was added, followed by concentrating under reduced pressure to dryness, to obtain N-trifluoroacetyl-1,2-ethanediamine.

### (3) Synthesis of MGI471-1-V1

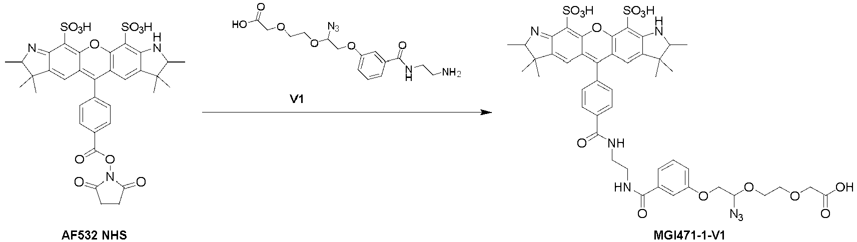

In a 15 mL sample vial, AF532 NHS (100 mg, 138.17 µmol) was dissolved with anhydrous N,N-dimethylformamide (3 mL), then V1 (102 mg, 277.66 µmol) and N,N-diisopropylethylamine (89 mg, 688.60 µmol) were added, followed by stirring magnetically at 22°C for 15 hours. The reaction solution was filtered, the filtrate was purified by flash preparative liquid chromatography (0.1 M TEAB/acetonitrile), and the prepared solution was concentrated under reduced pressure to obtain the compound MGI471-1-V1. LCMS: calcd for C₄₅H₄₉N₇O₁₄S₂ [M-H]⁻: 974.28. Found, m/z, [M-H]⁻: 974.34.

### (4) Synthesis of MGI471-1-V2-1

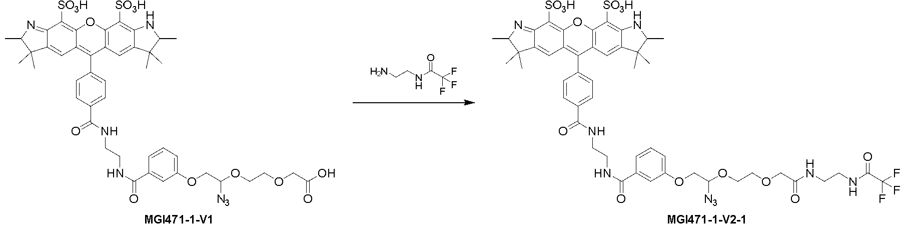

In a 50 mL flask, MGI471-1-V1 (90 mg, 92.21 µmol) was dissolved with anhydrous N,N-dimethylformamide (5 mL), then N,N'-disuccinimidyl carbonate (36 mg, 140.53 µmol) and 4-dimethylaminopyridine (2 mg, 16.37 µmol) were added, followed by stirring magnetically at 24°C for 3 hours. Then N-trifluoroacetyl-1,2-ethanediamine (58 mg, 371.54 µmol) and N,N-diisopropylethylamine (60 mg, 464.23 µmol) were added, followed by further stirring magnetically at 24°C for 15 hours. The reaction solution was filtered, the filtrate was purified by flash preparative liquid chromatography (0.1 M TEAB/acetonitrile), and the prepared solution was concentrated under reduced pressure to obtain MGI471-1-V2-1.LCMS: calcd for C₄₉H₅₄F₃N₉O₁₄S₂ [M-H]⁻: 1112.32. Found, m/z, [M-H]⁻: 1112.49.

### (5) Synthesis of MGI471-V2

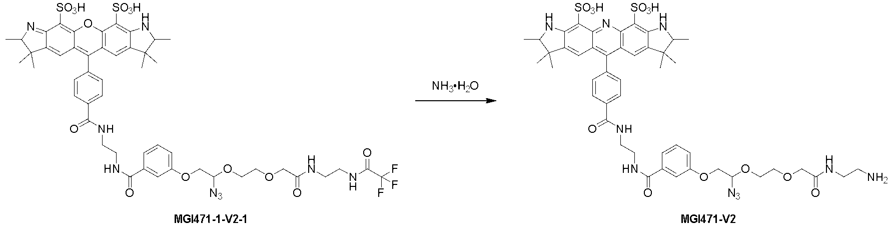

In a 15 mL sample vial, MGI471-1-V2-1 (60 mg, 53.85 µmol) was dissolved with methanol (5 mL), then ammonia (5 mL) was added, followed by stirring magnetically at 25°C for 3 hours. The reaction solution was concentrated under reduced pressure to obtain a crude product, the crude product was purified by preparative HPLC (0.1 M TEAB/acetonitrile), and the prepared solution was concentrated under reduced pressure, and freeze-dried to obtain MGI471-V2.

¹H NMR (600 MHz, DMSO-d₆): δ 14.34 (s, 1H), 8.88-8.84 (m, 1H), 8.67 (t, J = 5.2 Hz, 1H), 8.14 - 8.11 (m, 2H), 7.91 (t, J = 5.9 Hz, 1H), 7.87-7.82 (m, 2H), 7.51 - 7.47 (m, 4H), 7.39 (t, J = 7.9 Hz, 1H), 7.14 - 7.11 (m, 1H), 6.77 (s, 2H), 5.10 (t, J = 5.0 Hz, 1H), 4.21 (dd, J = 10.3, 4.4 Hz, 1H), 4.14 (dd, J = 10.4, 5.5 Hz, 1H), 3.89-3.83 (m, 3H), 3.77 - 3.73 (m, 3H), 3.60 - 3.49 (m, 8H), 3.06 (qd, J = 7.4, 2.4 Hz, 2H), 2.94 - 2.89 (m, 2H), 1.17 - 1.13 (m, 12H), 0.98 (d, J = 3.9 Hz, 6H).

¹³C NMR (151 MHz, DMSO-d₆): δ 170.40, 166.69, 166.61, 158.11, 152.32, 151.48, 143.66, 138.11, 138.02, 136.62, 135.48, 130.04, 129.95, 128.22, 120.69, 120.64, 118.12, 116.85,113.47, 108.09, 89.75, 70.35, 70.22, 69.24, 68.73, 65.21, 58.40, 46.21, 42.65, 41.88, 40.50, 39.28, 36.54, 27.84, 27.69, 23.28, 23.21, 16.20, 16.12, 11.49, 9.28, 8.10.

LCMS: calcd for C₄₇H₅₆N₁₀O₁₂S₂ [M-H]⁻: 1015.35. Found, m/z, [M-H]⁻: 1015.51.

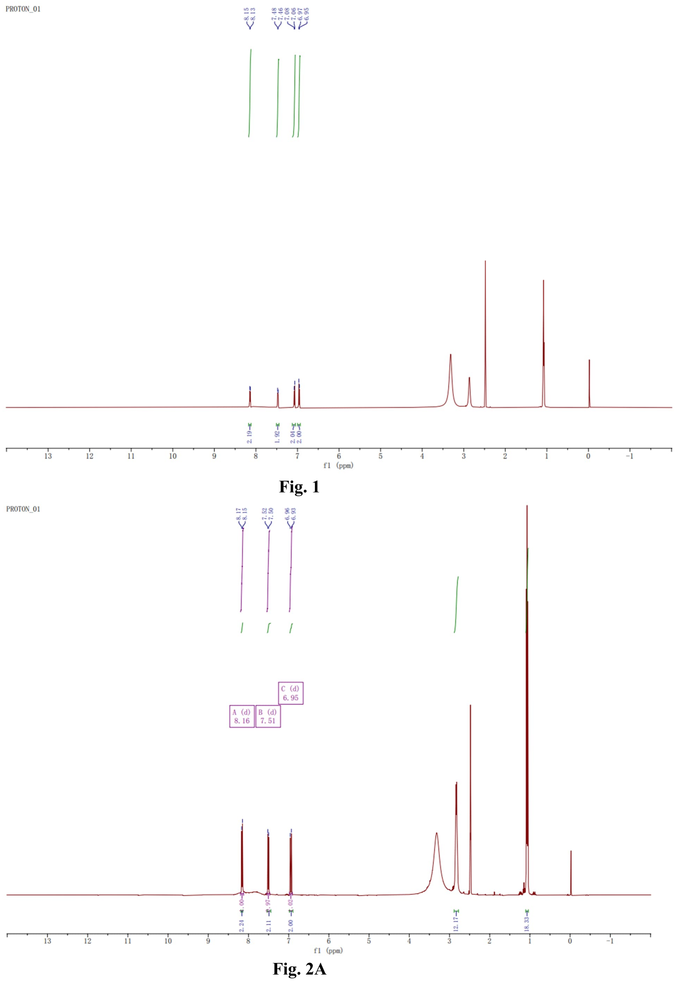

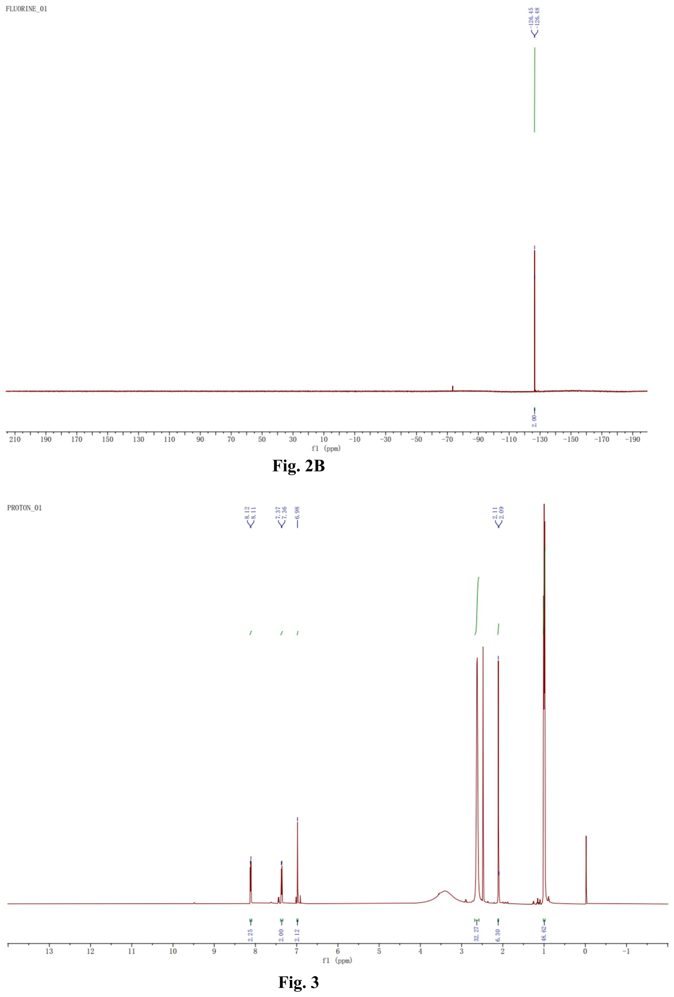

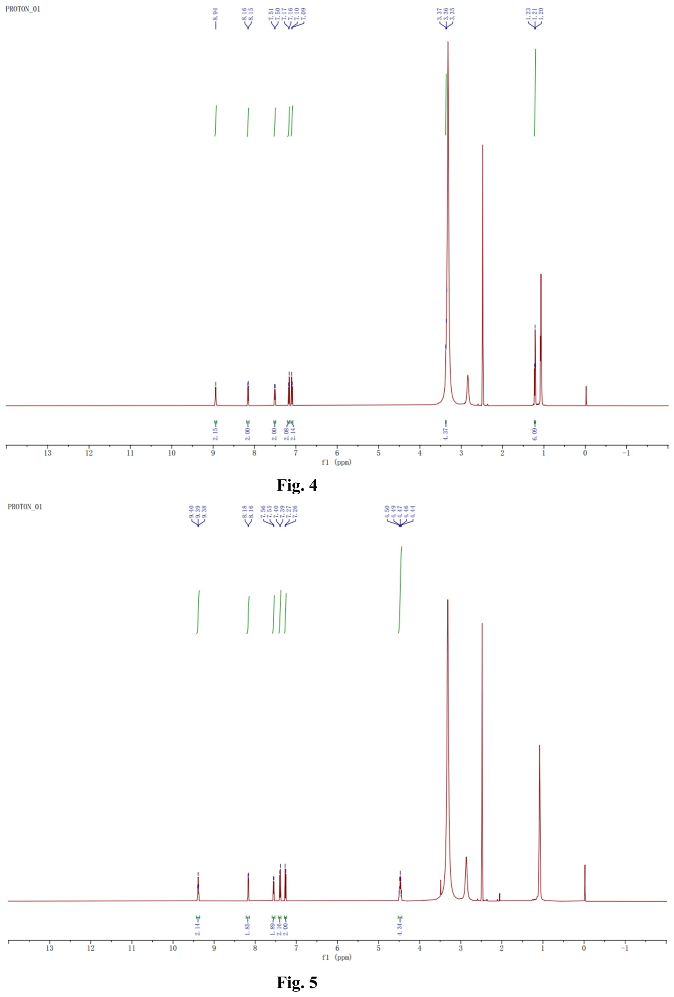

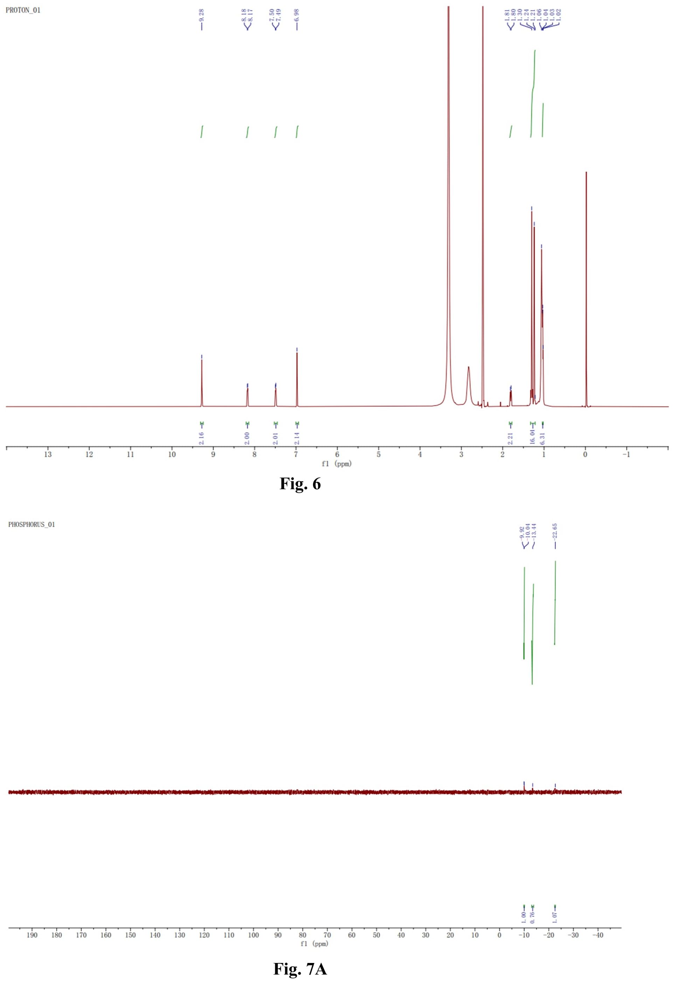

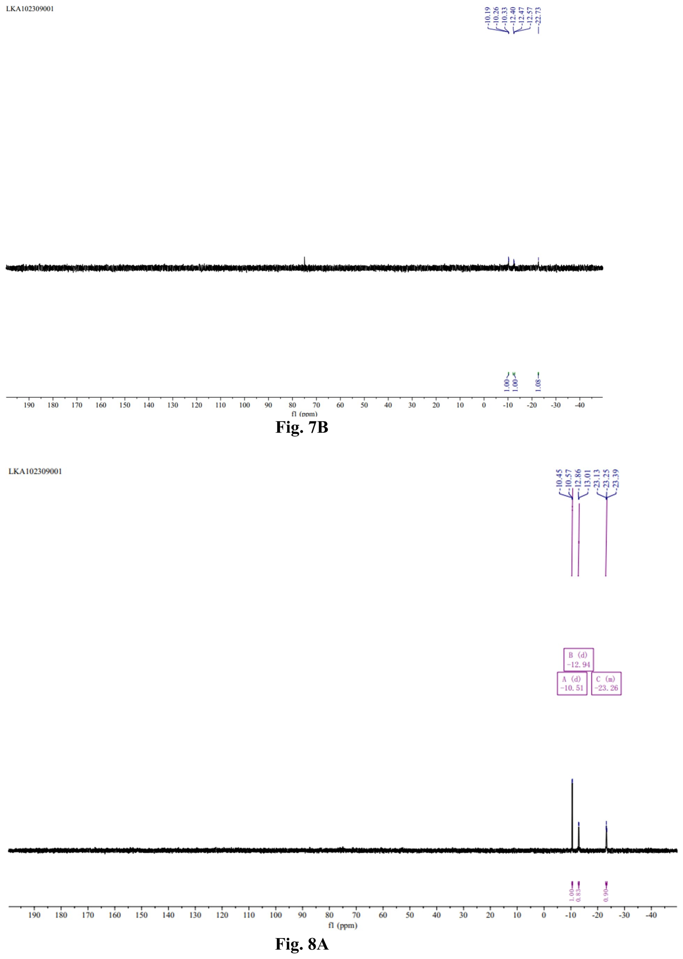

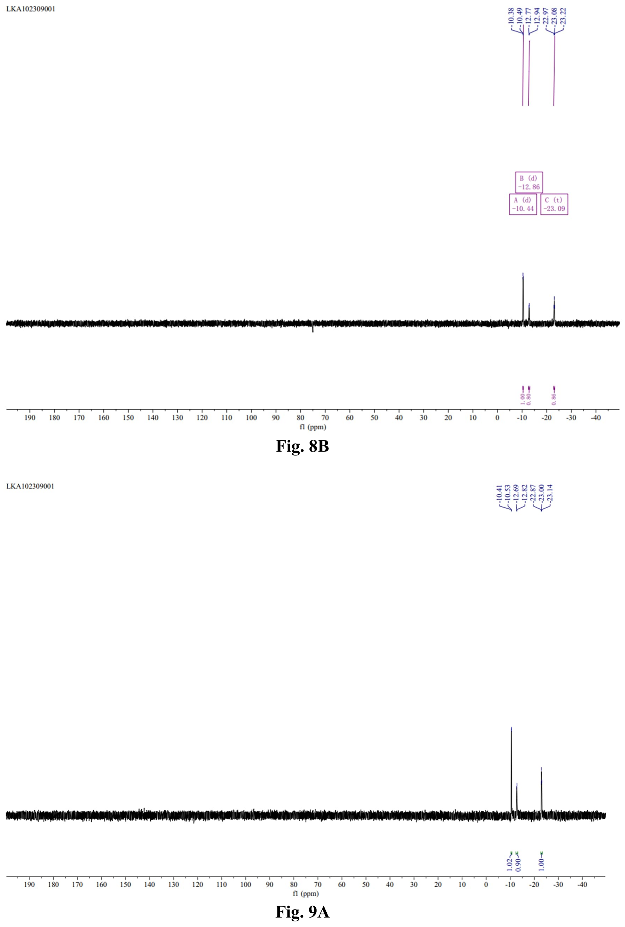

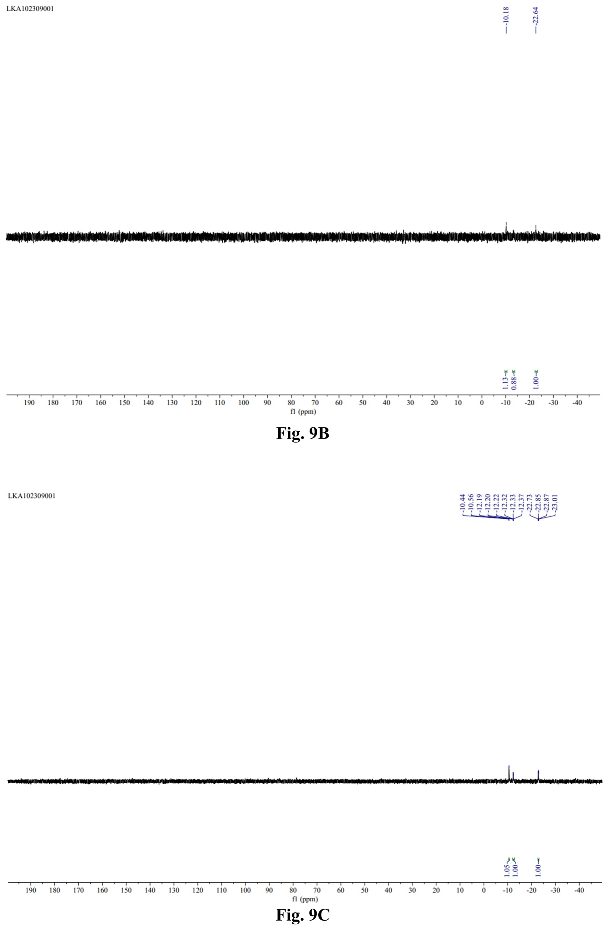

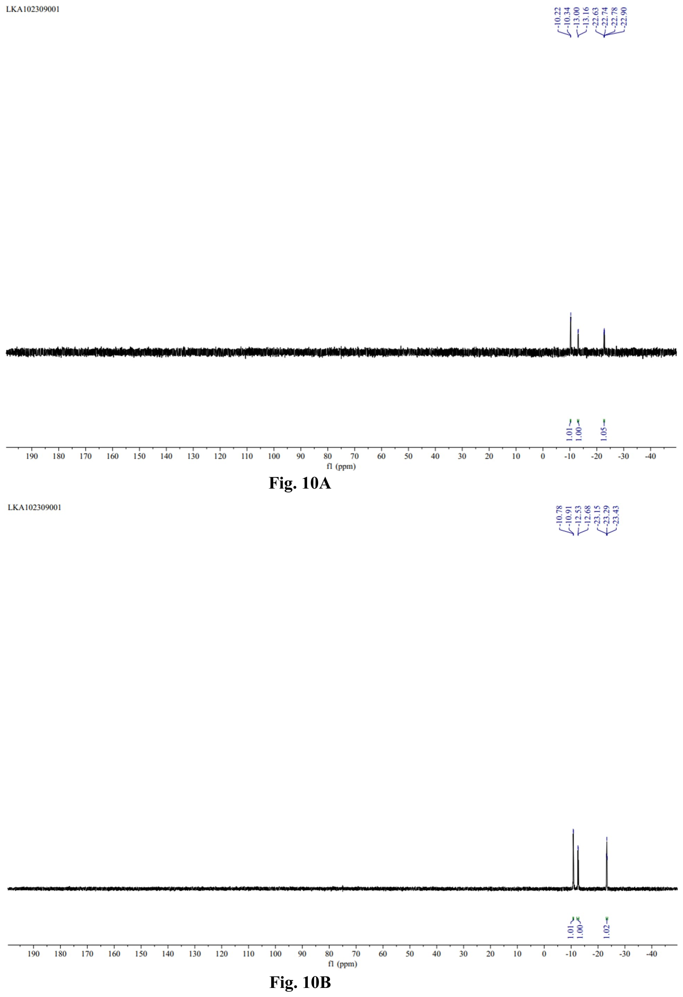

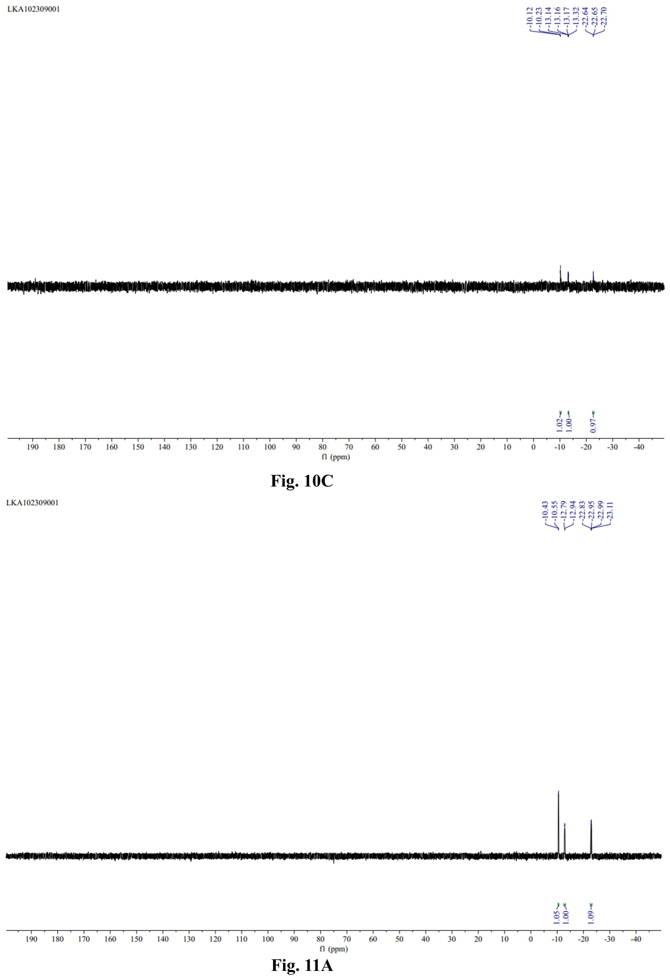

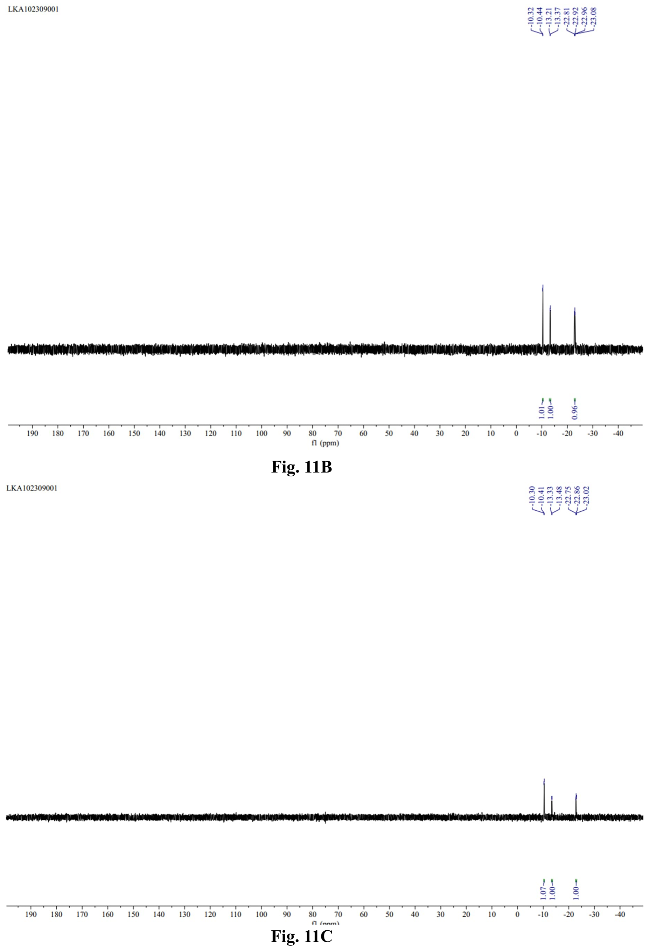

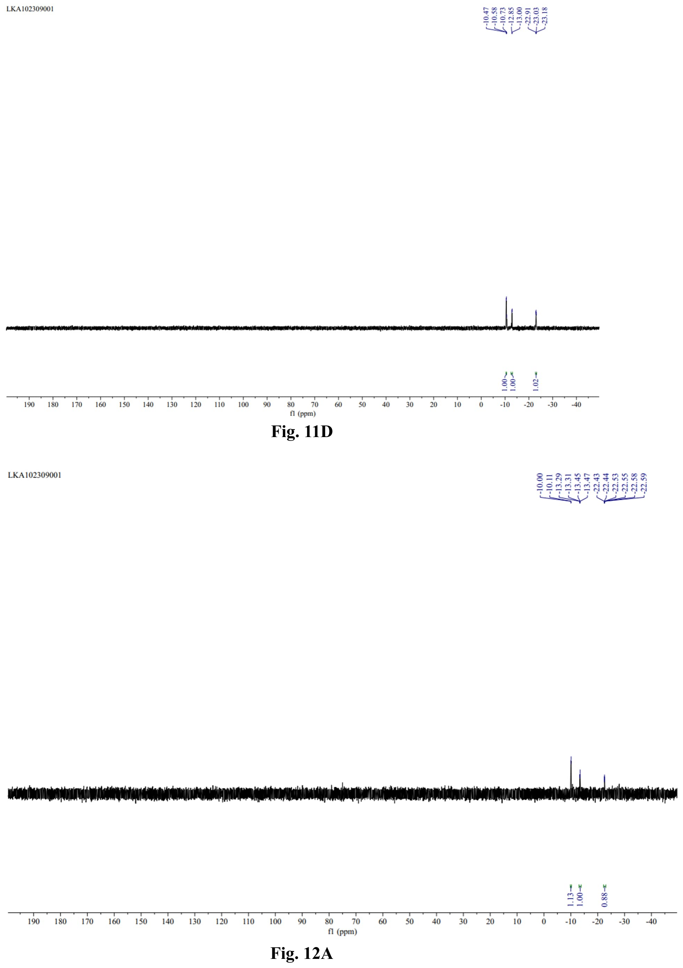

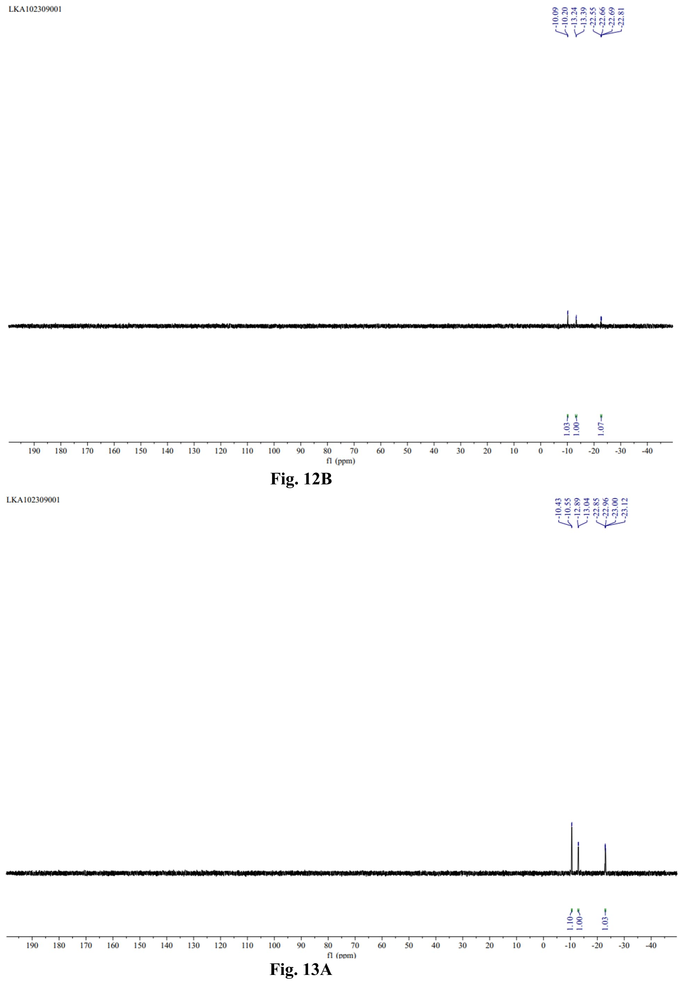

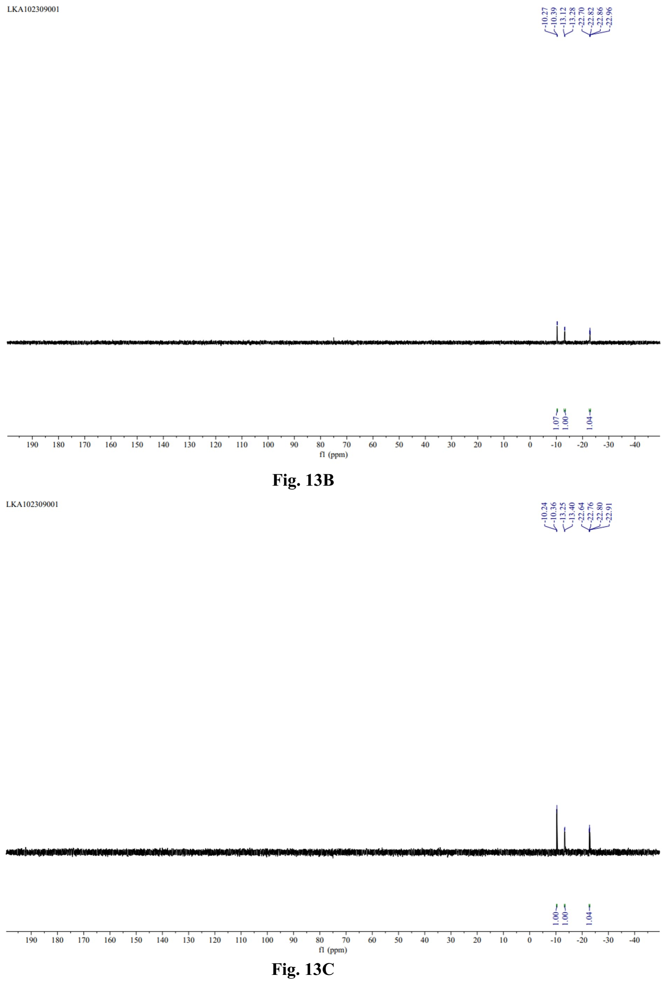

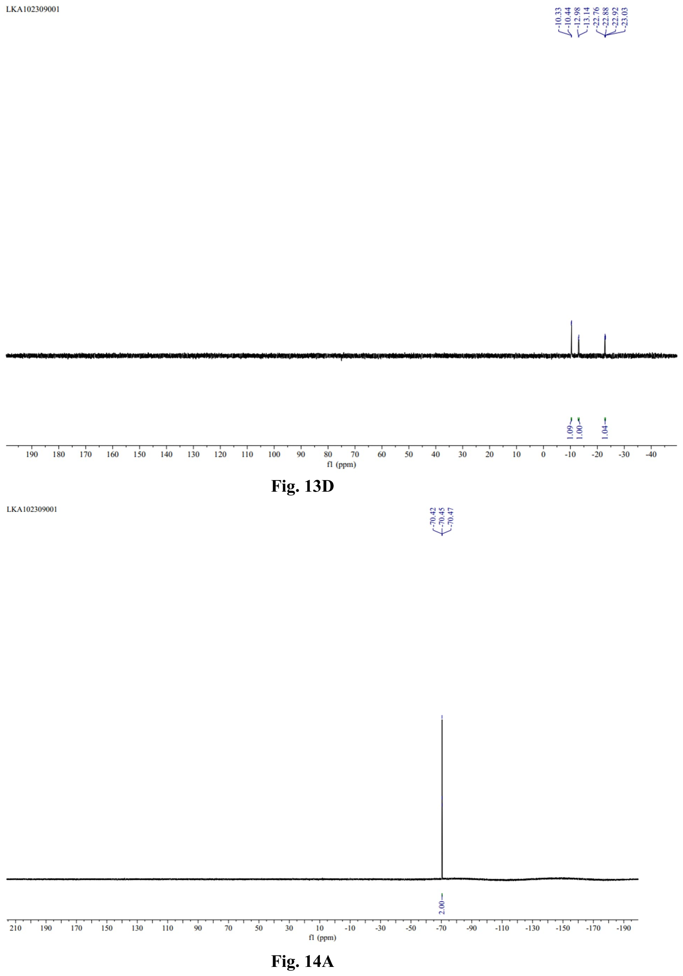

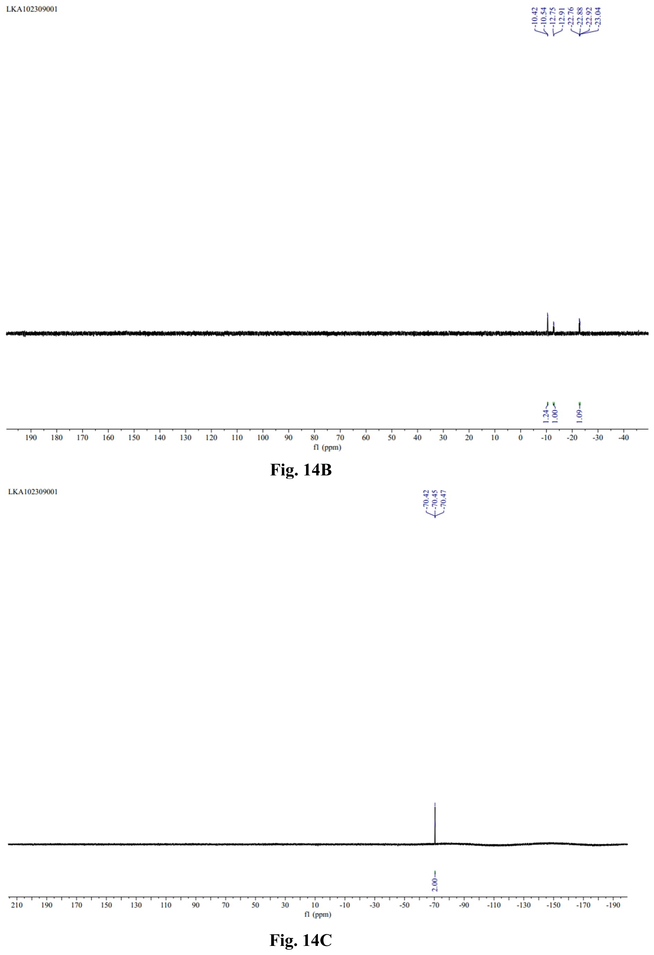

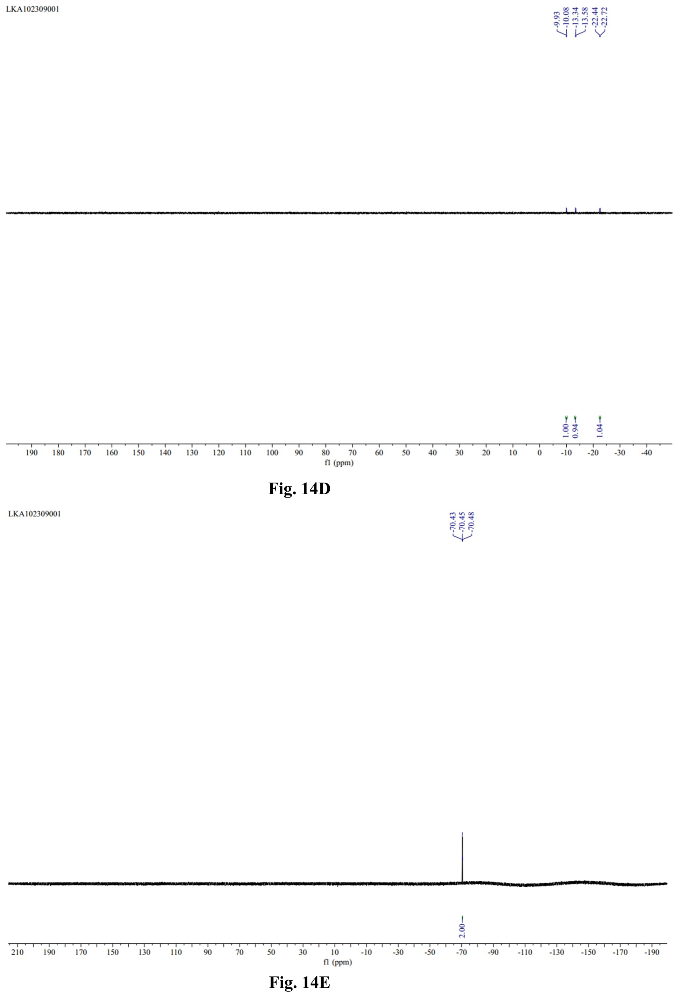

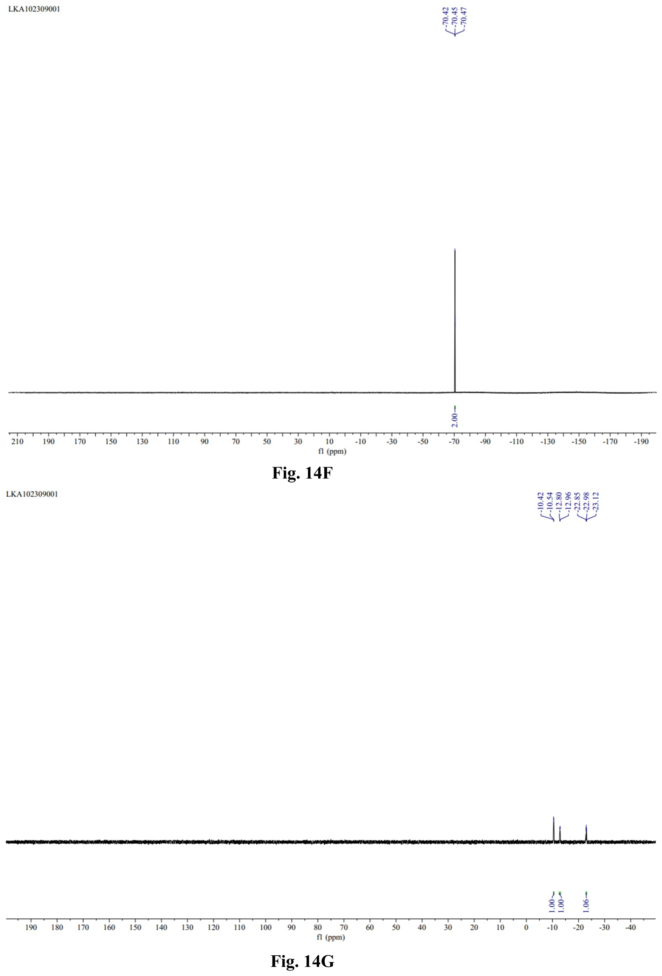

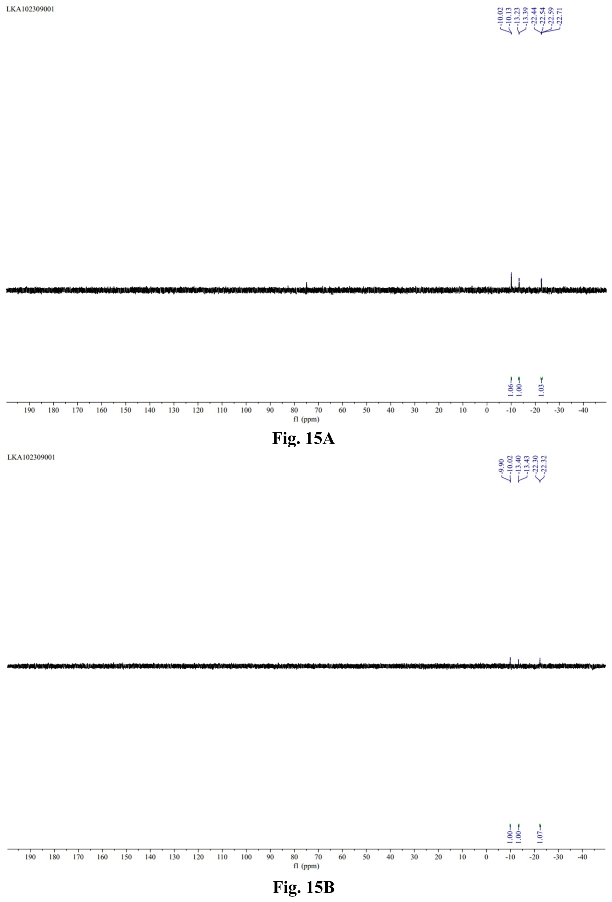

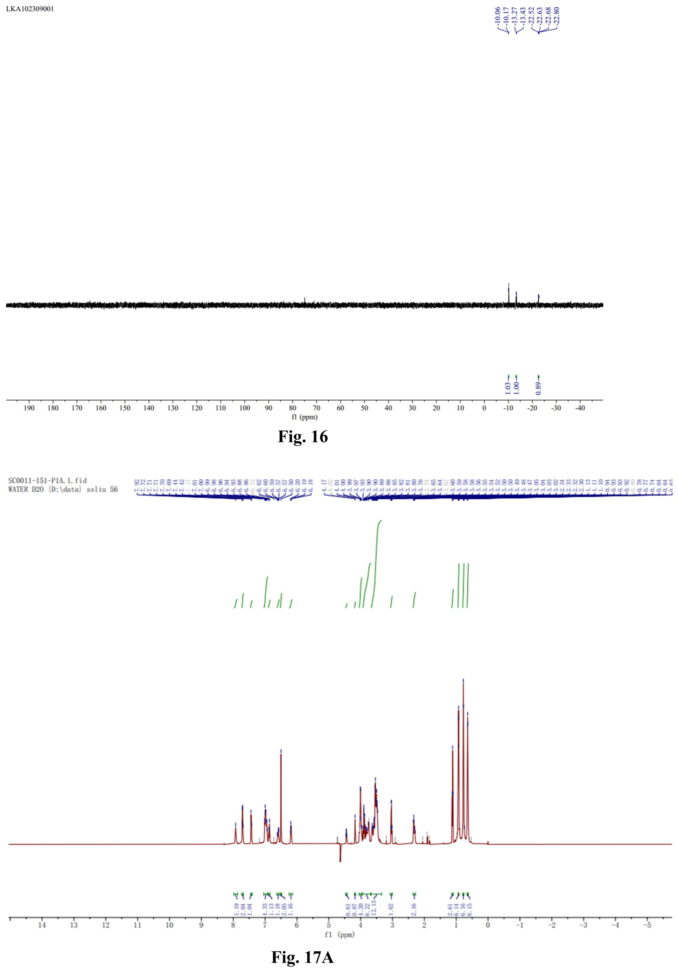

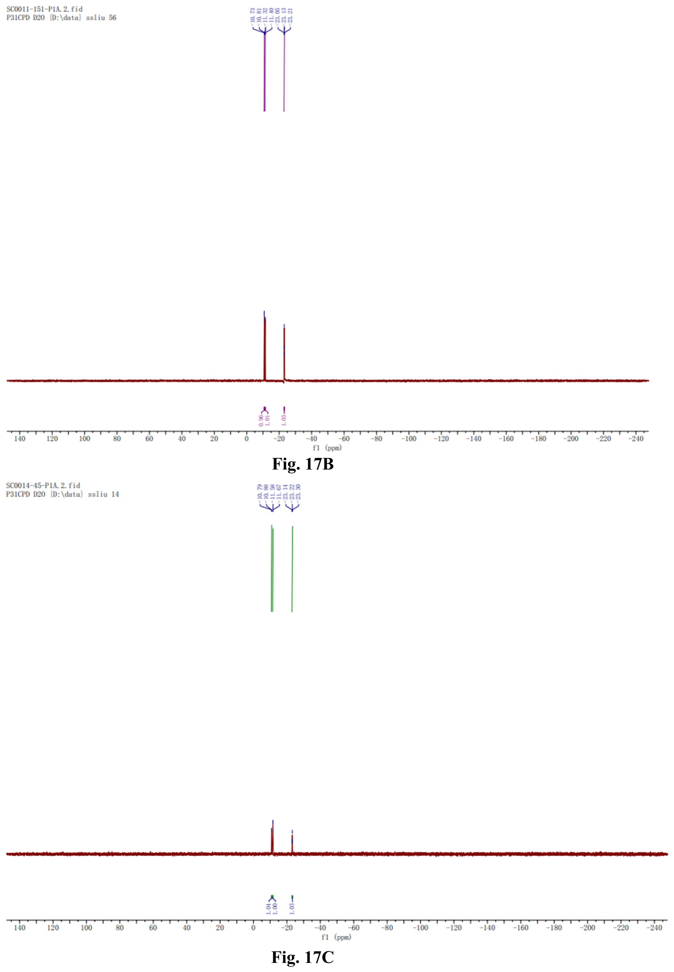

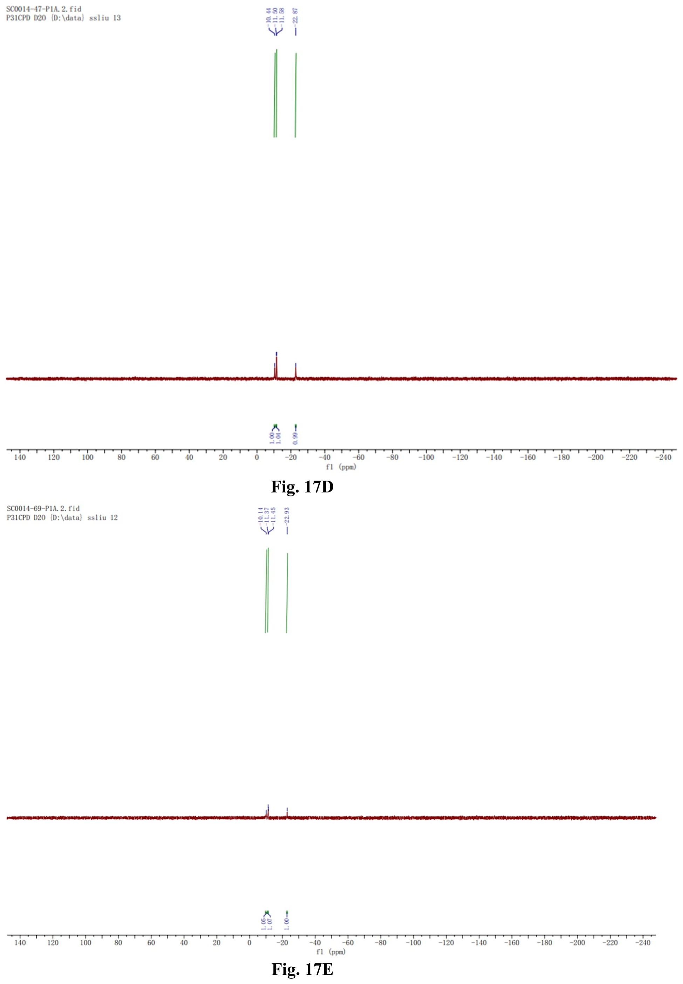

The nuclear magnetic resonance (NMR) characterization spectra of some of the above compounds are shown in Figures 1-17.

### Example 26: Test of the photochemical properties of fluorescent dyes or modified nucleotides

The photochemical properties of fluorescent dyes or modified nucleotides were tested, and the results are shown in Table 1.

**Table 1**

| Compound | Absorption maximum (nm) | Emission maximum (nm) |
|---|---|---|
| MGI443 | 443 | 470 |
| MGI447 | 447 | 476 |
| MGI450a | 450 | 469 |
| MGI450b | 445 | 475 |
| MGI450c | 445 | 473 |
| MGI450d | 451 | 480 |
| MGI453 | 453 | 486 |
| MGI455 | 455 | 478 |
| MGI456 | 456 | 484 |
| MGI471 | 471 | 491 |
| MGI477 | 477 | 502 |
| MGI485 | 485 | 508 |
| MGI488 | 488 | 506 |
| MGI443-V1-dATP | 443 | 475 |
| MGI443-V1-dTTP | 445 | 476 |
| MGI443-V1-dCTP | 444 | 477 |
| MGI443-V1-dGTP | 443 | 472 |
| MGI447-V1-dATP | 451 | 482 |
| MGI447-V1-dTTP | 452 | 481 |
| MGI447-V1-dCTP | 451 | 482 |
| MGI447-V1-dGTP | 451 | 480 |
| MGI450b-V1-dATP | 450 | 481 |
| MGI450b-V1-dTTP | 451 | 481 |
| MGI450b-V1-dCTP | 451 | 481 |
| MGI450b-V1-dGTP | 449 | 480 |
| MGI450c-V1-dATP | 446 | 478 |
| MGI450c-V1-dTTP | 447 | 478 |
| MGI450c-V1-dCTP | 445 | 480 |
| MGI450c-V1-dGTP | 447 | 478 |
| MGI450d-V1-dATP | 453 | 484 |
| MGI450d-V1-dTTP | 453 | 484 |
| MGI450d-V1-dCTP | 456 | 484 |
| MGI456-V1-dATP | 457 | 488 |
| MGI456-V1-dTTP | 459 | 490 |
| MGI456-V1-dCTP | 459 | 490 |
| MGI456-V1-dGTP | 458 | 488 |
| MGI471-V1-dATP | 471 | 494 |
| MGI471-V1-dTTP | 474 | 494 |
| MGI471-V1-dCTP | 474 | 494 |
| MGI471-V1-dGTP | 471 | 494 |
| MGI485-V1-dATP | 489 | 518 |
| MGI485-V1-dCTP | 489 | 516 |
| MGI488-V1-dATP | 487 | 511 |
| MGI488-V1-dCTP | 486 | 512 |
| MGI495-V1-dATP | 495 | 521 |
| MGI495-V1-dCTP | 495 | 520 |
| MGI501-V1-dATP | 501 | 521 |
| MGI501-V1-dCTP | 501 | 522 |

The excitation and emission spectra of the above compounds are shown in Figure 18.

### Example 27: Biochemical experiment

Experimental objective: to verify the performance of modified nucleotides on a sequencer.

### Experimental protocol:

With the use of dNTPs from the MGI471 series, a 2color Hot dNTP mix was prepared according to the double-labeled T base (MGI471-dTTP & AF532-dTTP), AF532-dATP, MGI471-dCTP, G non-luminescent form.

PE100+10 was tested on-machine using a modified DNBSEQ-200RS sequencer (with the H optical channel modified to 465 nm excitation), and the results were compared with those of PE100+10 tested on-machine using an unmodified DNBSEQ-200RS sequencing kit.

### Experimental results

From the results of the PE100 off-machine report, it can be seen that the on-machine Total Reads using MGI471 dNTP are slightly lower than those using conventional 2color reagents, but the data volume can reach over 650M, which is also a good level.

For PE100, the Q30%, SplitRate, and MappingRate are comparable between the two types of dNTPs. The AvgErrorRate!N(%) of MGI471 dNTP monomers is slightly lower than that of the conventional reagent.

In terms of Runon/Lag, the overall Runon of MGI471 dNTP monomers is slightly higher, indicating that the purity of the monomers needs further improvement. The Lag shows little difference from the conventional reagent, demonstrating that the polymerization efficiency of MGI471 dNTPs is excellent.

The shortcomings of MGI471 dNTP monomer lie in signal recovery and Q30% recovery. Due to the short wavelength and high laser energy of MGI471 monomer, the damage to DNB is much stronger than under conventional conditions, which to some extent affects the recovery of the second chain. Therefore, optimization in monomer structure and sequencing reagents to reduce photo damage is needed.

Overall, MGI471 dNTP can meet the application of gene sequencing to a certain extent.

The performance of MGI471 dNTPs on the sequencer is shown in Table 2. The Q30 data of MGI471 dNTPs on the sequencer is shown in Figure 19.

**Table 2**

| Category | S250082993 | S230086182 | S250043831 | S250043716 | S250069603 |
|---|---|---|---|---|---|
| Monomer | Blue light MGI471 | Blue light MGI471 | normal 2 color | normal 2 color | normal 2 color |
| Software Version | 1.5.0.296 | 1.5.0.296 | 1.5.0.296 | 1.5.0.296 | 1.5.0.296 |
| TemplateVersion | 0.8.0 | 0.8.0 | 0.8.0 | 0.8.0 | 0.8.0 |
| Reference | Ecoli | Ecoli | Ecoli | Ecoli | Ecoli |
| CycleNumber | 210 | 210 | 220 | 220 | 210 |
| ChipProductivity(%) | 77.22 | 75.4 | 81.02 | 79.12 | 86.66 |
| ImageArea | 612 | 612 | 612 | 612 | 612 |
| TotalReads(M) | 671.11 | 652.32 | 699.59 | 683.74 | 754.79 |
| Q30(%) | 96.69 | 96.06 | 96.66 | 96.15 | 96.44 |
| SplitRate(%) | 98.68 | 98.23 | 98.04 | 98.12 | 98.9 |
| Runon1(%) | 0.05 | 0.06 | 0.04 | 0.05 | 0.06 |
| Runon2(%) | 0.06 | 0.08 | 0.04 | 0.05 | 0.07 |
| Lag1(%) | 0.06 | 0.08 | 0.08 | 0.08 | 0.07 |
| Lag2(%) | 0.1 | 0.12 | 0.13 | 0.13 | 0.09 |
| ESR(%) | 77.22 | 75.4 | 81.02 | 79.12 | 86.66 |
| MappingRate(%) | 99.22 | 99.22 | 99.32 | 99.25 | 99.62 |
| AvgErrorRate(%) | 0.45 | 0.49 | 0.38 | 0.45 | 0.2 |
| AvgErrorRate!N(%) | 0.1 | 0.09 | 0.12 | 0.16 | 0.11 |
| RecoverValue(AVG) | 1.14 | 1.21 | 1.64 | 1.65 | 1.9 |

Interpretation of key biochemical indicators:
Q30 (%): The percentage of bases in the basecall results with an estimated error rate lower than 0.001 (i.e., accuracy higher than 99.9%).
Runon/Lag: Runon can be understood as the proportion of copy number of advanced reaction in DNB, and Lag can be understood as the proportion of copy number of delayed reaction in DNB.
TotalReads (M): The total number of reads contained in the fq file generated by Zebracall off-machine without splitting.
SplitRate (%): By classifying and splitting the barcode part of the sequencing sequence, the number of sequences corresponding to the barcode list is counted, the proportion thereof accounting for the total fastq sequence number being the splitRate. The denominator for calculating the splitRate is the number of reads (namely, reads left by filtering) in all off-machine fastq files, and at the moment, TotalReads is the numerator for calculating the splitRate.
MappingRate (%): The ratio of MappedReads to TotalReads.
AvgErrorRate!N (%): Representing the average error rate of the remaining mismatch types after removing the part of mismatches caused by call N.

The above examples are only used to illustrate the technical solution of the present invention and not to limit it. Any modification or equivalent replacement made to the technical solution of the present invention without departing from the purpose and scope of the technical solution of the present invention shall all be covered within the protection scope of the present invention.

## Claims

1. A compound represented by formula (I), an ester thereof, or a salt thereof, 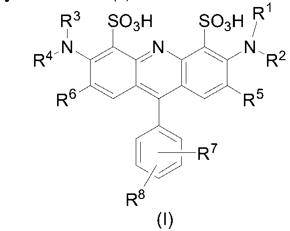
wherein, R¹, R², R³ and R⁴, the same or different from each other, are each independently selected from H, C₁-C₆ alkyl and halo-C₁-C₆ alkyl;
R³ and R⁶, the same or different, are each independently selected from H, C₁-C₆ alkyl, halogen and halo-C₁-C₆ alkyl;
R⁷ and R⁸, the same or different, are each independently selected from H, -COOH, -C(O)NH-(C₁-C₆ alkyl) and -C(O)NH₂, and R⁷ and R⁸ are not simultaneously H;
optionally, -NR¹R², together with the benzene ring to which it is attached, forms a benzo 5-to 6-membered nitrogen-containing heterocyclic group, said 5- to 6-membered nitrogen-containing heterocyclic group being optionally substituted with one or more groups selected from C₁-C₆ alkyl, halogen and halo-C₁-C₆ alkyl;
optionally, -NR³R⁴, together with the benzene ring to which it is attached, forms a benzo 5-to 6-membered nitrogen-containing heterocyclic group, said 5- to 6-membered nitrogen-containing heterocyclic group being optionally substituted with one or more groups selected from C₁-C₆ alkyl, halogen and halo-C₁-C₆ alkyl.

2. The compound, the ester thereof or the salt thereof as claimed in claim 1, wherein, R¹, R², R³, R⁴ are each H; or
R¹, R², R³ and R⁴ are each C₁-C₆ alkyl (such as methyl, ethyl); or
R¹, R², R³ and R⁴ are each Halo-C₁-C₆ alkyl (such as trifluoromethyl, trifluoroethyl); or
R¹ and R³ are the same, each being H; R² and R⁴ are the same, each being halo-C₁-C₆ alkyl (such as trifluoromethyl, trifluoroethyl).

3. The compound, the ester thereof or the salt thereof as claimed in claim 1 or 2, wherein, R⁵ and R⁶ are the same;
preferably, R⁵ and R⁶ are each H, or, R⁵ and R⁶ are each halogen (such as F).

4. The compound, the ester thereof or the salt thereof as claimed in any one of claims 1-3, wherein, R⁷ is carboxyl.

5. The compound, the ester thereof or the salt thereof as claimed in any one of claims 1-4, wherein, R⁸ is H or carboxyl.

6. The compound, the ester thereof or the salt thereof as claimed in any one of claims 1-5, wherein, -NR¹R², together with the benzene ring to which it is attached, forms a benzo 5- to 6-membered nitrogen-containing heterocyclic group, said 5- to 6-membered nitrogen-containing heterocyclic group being optionally substituted with one or more methyl groups;
preferably, -NR³R⁴, together with the benzene ring to which it is attached, forms a benzo 5-to 6-membered nitrogen-containing heterocyclic group, said 5- to 6-membered nitrogen-containing heterocyclic group being optionally substituted with one or more methyl groups.

7. The compound, the ester thereof or the salt thereof as claimed in any one of claims 1-6, wherein, R⁷ or R⁸ is carboxyl, said carboxyl being connected with a cleavable linker, such as a cleavable linker having a structure as shown below:

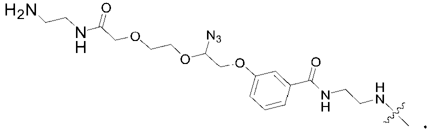

8. The compound, the ester thereof or the salt thereof as claimed in any one of claims 1-7, wherein the compound has a structure represented by formula (II): 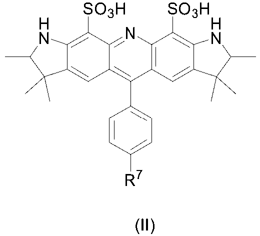 wherein, R⁷ is as defined in claim 1 or 4; or
the compound has a structure represented by formula (III): 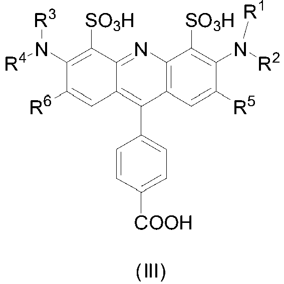
wherein, R¹, R², R³, R⁴, R⁵, R⁶ and R⁷ are as defined in any one of claims 1-4 and 6; or
the compound has a structure represented by formula (IV): 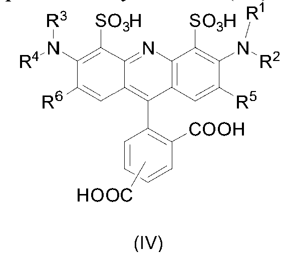
wherein, R¹, R², R³, R⁴, R⁵ and R⁶ are as defined in any one of claims 1-3 and 6.

9. The compound, the ester thereof or the salt thereof as claimed in any one of claims 1-8, wherein the compound has a structure selected from the following:

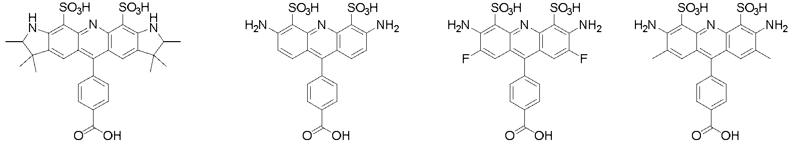

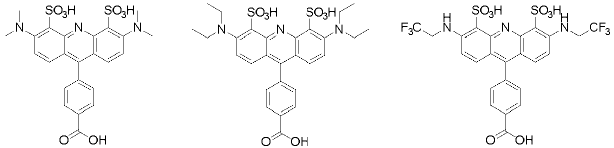

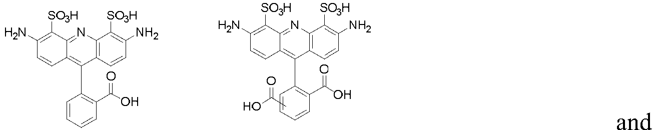

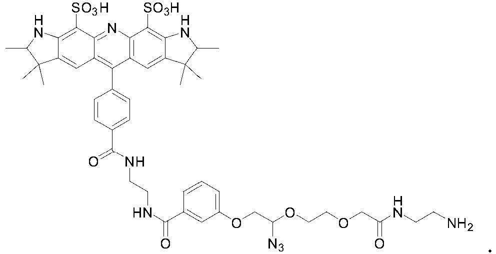

10. A compound represented by formula (I'), an ester thereof, or a salt thereof, 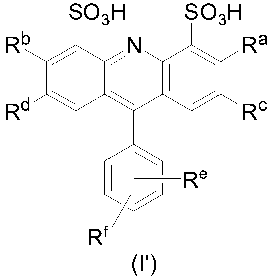
wherein, R^{a}, R^{b}, R^{c} and R^{d}, the same or different from each other, are each independently selected from H, C₁-C₆ alkyl, halo-C₁-C₆ alkyl, -NR^{1'}R^{2'}, hydroxy, hydroxy-substituted C₁-C₆ alkyl, and halogen; R^{1'} and R^{2'}, the same or different from each other, are each independently selected from H, C₁-C₆ alkyl and halo-C₁-C₆ alkyl;
R^{e} and R^{f}, the same or different, are each independently selected from H, -COOH and - C(O)NR^{3'}R^{4'}, and R^{e} and R^{f} are not simultaneously H;
R^{3'} and R^{4'}, the same or different from each other, are each independently selected from H and C₁-C₆ alkyl, said C₁-C₆ alkyl being optionally substituted with carboxyl, -C(O)NH₂ or sulfonic group;
optionally, when R^{a} is -NR^{1'}R^{2'}, -NR^{1'}R^{2'}, together with the benzene ring to which it is attached, forms a benzo 5- to 6-membered nitrogen-containing heterocyclic group, said 5- to 6-membered nitrogen-containing heterocyclic group being optionally substituted with one or more groups selected from C₁-C₆ alkyl, halogen and halo-C₁-C₆ alkyl;
optionally, when R^{b} is -NR^{1'}R^{2'}, -NR^{1'}R^{2'}, together with the benzene ring to which it is attached, forms a benzo 5- to 6-membered nitrogen-containing heterocyclic group, said 5- to 6-membered nitrogen-containing heterocyclic group being optionally substituted with one or more groups selected from C₁-C₆ alkyl, halogen and halo-C₁-C₆ alkyl.

11. The compound, the ester thereof or the salt thereof as claimed in claim 10, wherein, R^{a} and R^{b} are the same, preferably each being -NR^{1'}R^{2'} or each being hydroxy; and/or
R^{c} and R^{d} are the same, preferably each being H or each being halogen;
preferably, R^{1'} and R^{2'} are each H;
preferably, one of R^{1'} and R^{2'} is H, and the other is selected from C₁-C₆ alkyl (such as methyl, ethyl).

12. The compound, the ester thereof or the salt thereof as claimed in claim 10 or 11, wherein, R^{e} and R^{f} are different, each being independently selected from H, -COOH and -C(O)NR^{3'}R^{4'};
preferably, R^{3'} and R^{4'}, the same or different from each other, are each independently selected from H and C₁-C₆ alkyl, said C₁-C₆ alkyl being optionally substituted with carboxyl or sulfonic group.

13. The compound, the ester thereof or the salt thereof as claimed in any one of claims 10-12, wherein, R^{a} is -NR^{1'}R^{2'}; -NR^{1'}R^{2'}, together with the benzene ring to which it is attached, forms a benzo 5- to 6-membered nitrogen-containing heterocyclic group, said 5- to 6-membered nitrogen-containing heterocyclic group being optionally substituted with one or more methyl groups; and/or
R^{b} is -NR^{1'}R^{2'}; -NR^{1'}R^{2'}, together with the benzene ring to which it is attached, forms a benzo 5- to 6-membered nitrogen-containing heterocyclic group, said 5- to 6-membered nitrogen-containing heterocyclic group being optionally substituted with one or more methyl groups.

14. The compound, the ester thereof or the salt thereof as claimed in any one of claims 10-13, wherein the compound has a structure represented by formula (II'): 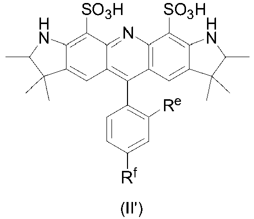 wherein, R^{e} and R^{f} are as defined in claim 10 or 12.

15. The compound, the ester thereof or the salt thereof as claimed in any one of claims 10-14, wherein the compound has a structure represented by formula (III'): 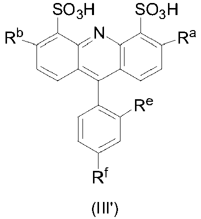 wherein, R^{a}, R^{e} and R^{f} are as defined in any one of claims 10-12.

16. The compound, the ester thereof or the salt thereof as claimed in any one of claims 10-15, wherein the compound has a structure selected from the following:

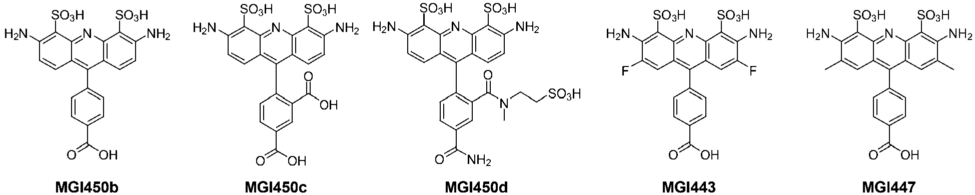

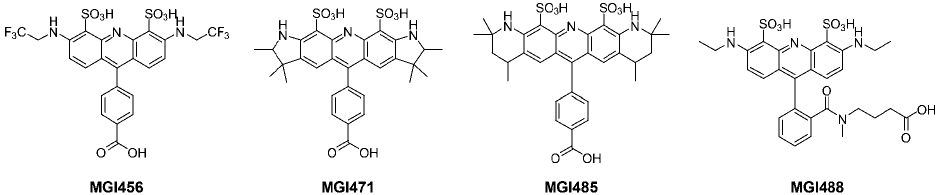

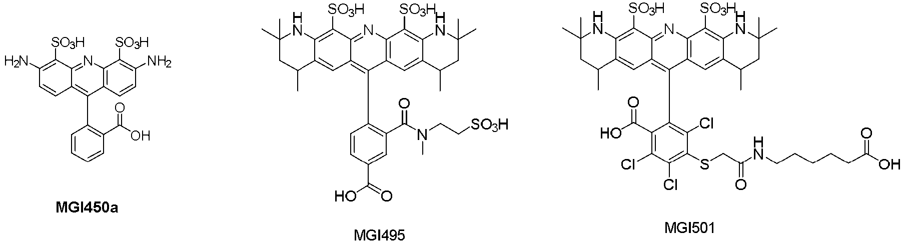

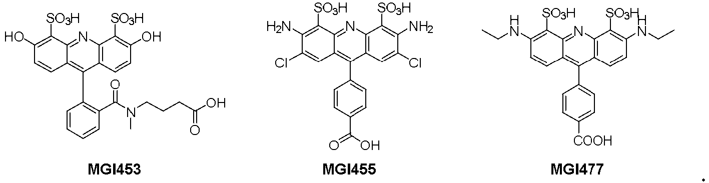

17. A compound represented by formula (i): 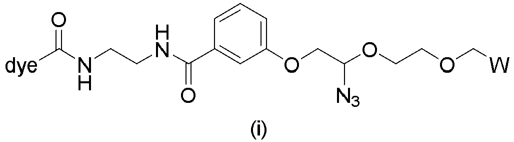 wherein, w is carboxyl or ester group, and the dye is the compound or the salt thereof as claimed in any one of claims 1-16.

18. The compound, the ester thereof or the salt thereof as claimed in any one of claims 1-17, wherein the ester of the compound is an activated ester of carboxyl group, such as nitrophenyl ester, pentafluorophenyl ester, or succinimidyl ester.

19. The compound, the ester thereof or the salt thereof as claimed in any one of claims 1-17, wherein the salt of the compound is a salt formed by the sulfonic group on an acridine ring, such as salts formed by the sulfonic group with alkali metal ions, alkaline earth metal ions, or ammonium ions.

20. A labeled nucleotide or oligonucleotide, wherein the labeled nucleotide or oligonucleotide is labeled with the compound, the ester thereof or the salt thereof as claimed in any one of claims 1-19.

21. The labeled nucleotide or oligonucleotide as claimed in claim 20, wherein the compound is attached to a C5 position of a pyrimidine base or a C7 position of a 7-deazapurine base of the nucleotide or oligonucleotide via a cleavable linker.

22. The labeled nucleotide as claimed in claim 20 or 21, which has a structure represented by formula (1): 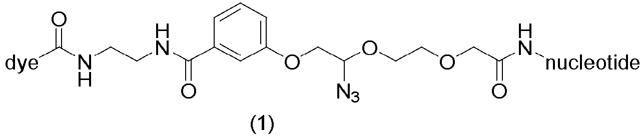 wherein, the dye is the compound, the ester thereof or the salt thereof as claimed in any one of claims 1-16.

23. The labeled nucleotide as claimed in claim 22, which has the following structure: 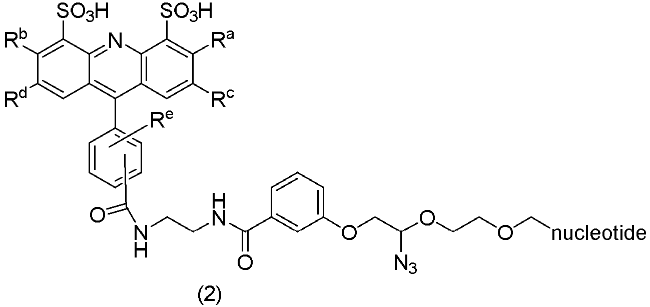 wherein, R^{a}, R^{b}, R^{c}, R^{d} and R^{e} are as defined in any one of claims 10-13.

24. The labeled nucleotide as claimed in claim 22 or 23, which has the following structure: 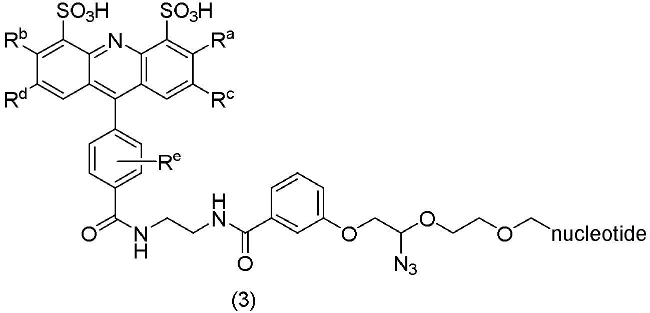 wherein, R^{a}, R^{b}, R^{c}, R^{d} and R^{e} are as defined in any one of claims 10-13.

25. The labeled nucleotide as claimed in any one of claims 22-24, which has the following structure: 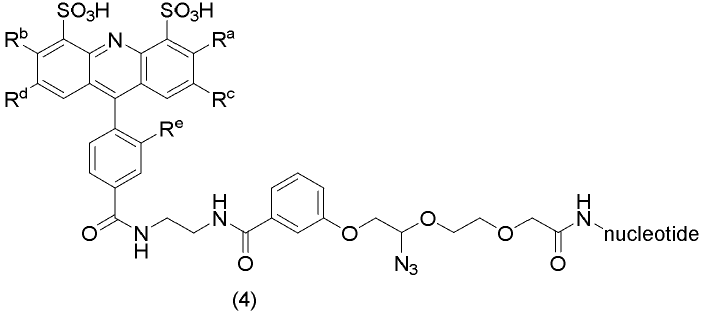 wherein, R^{a}, R^{b}, R^{c}, R^{d} and R^{e} are as defined in any one of claims 10-13.

26. The labeled nucleotide as claimed in any one of claims 22-25, wherein the nucleotides in the formulas are selected from dATP, dGTP, dCTP and dTTP.

27. A sequencing method, which comprises incorporating the labeled nucleotide as claimed in any one of claims 20-26 into a sequencing assay;
preferably, the method further comprises detecting the labeled nucleotide;
preferably, the sequencing assay is performed on an automated sequencer, wherein the automated sequencer includes two light sources operating at different wavelengths.

28. The sequencing method as claimed in claim 27, which comprises:
(a) incorporating at least one labeled nucleotide as claimed in any one of claims 20-26 into a polynucleotide; and
(b) detecting the labeled nucleotide incorporated into the polynucleotide by detecting a fluorescent signal from a novel fluorescent dye attached to a modified nucleotide.

29. A kit, comprising one or more nucleosides, wherein at least one nucleoside is the labeled nucleotide as claimed in any one of claims 20-26;
preferably, the kit comprises two or more labeled nucleotides.

30. Use of the compound, the ester thereof or the salt thereof as claimed in any one of claims 1-16 in fields of sequencing, expression analysis, hybridization analysis, genetic analysis, RNA analysis, protein-binding assays, in vitro diagnostics, immunoassays, and molecular labeling;
preferably, the molecular labeling is used for cell imaging, tissue imaging, or in vivo biological imaging.

31. Use of the compound, the ester thereof or the salt thereof as claimed in any one of claims 1-16 in fluorescent labeling, quantification or detection of proteins, enzymes, or nucleic acids.

32. Use of the labeled nucleotide or oligonucleotide as claimed in any one of claims 20-26 or the kit as claimed in claim 29 in sequencing.

33. A method for preparing the compound, the ester thereof or the salt thereof as claimed in any one of claims 1-16, which comprises subjecting a xanthene structure to a substitution reaction in the presence of ammonia and methanol to convert it into an acridine structure.

34. The method as claimed in claim 33, wherein, a reaction route for synthesizing the compound of formula (I) is as follows: 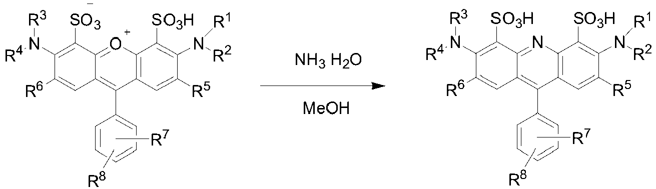 R¹-R⁷ are as defined in any one of claims 1-6.

35. The method as claimed in claim 33, wherein, a reaction route for synthesizing the compound of formula (I') is as follows: 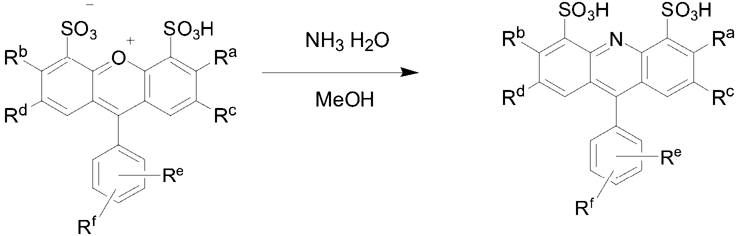 R^{a}-R^{f} are as defined in any one of claims 10-13.

36. A method for preparing a labeled nucleotide, which is carried out according to any one of Method A, Method B, or Method C:
Method A: 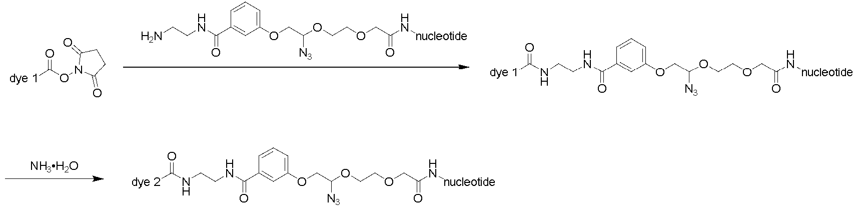 wherein, dye 2 is the compound, the ester thereof or the salt thereof as claimed in any one of claims 1-16, the compound having an acridine structure, and dye 1 is the precursor of dye 2 and has a xanthene structure;
Method B: 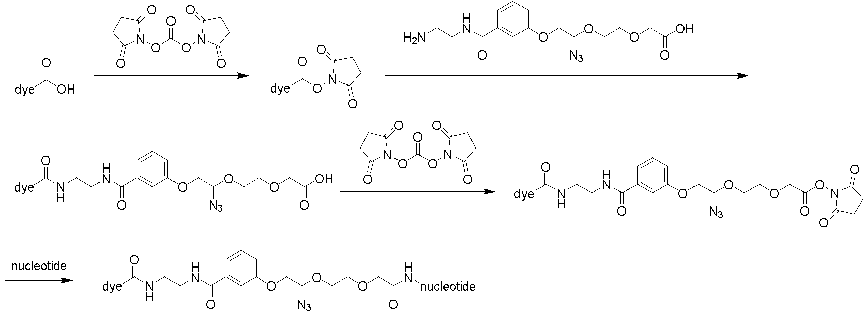 wherein, the dye is the compound, the ester thereof or the salt thereof as claimed in any one of claims 1-16;
Method C: 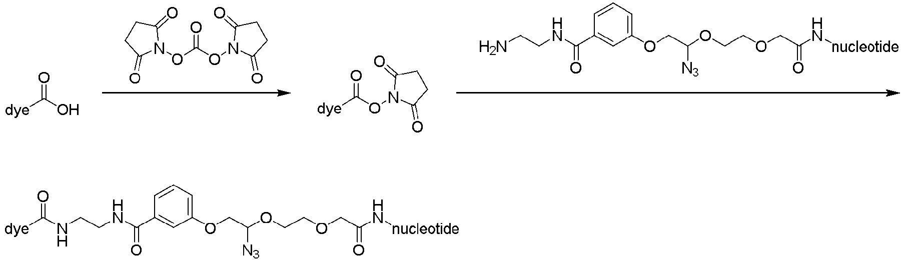 wherein, the dye is the compound, the ester thereof or the salt thereof as claimed in any one of claims 1-16;
Method D: 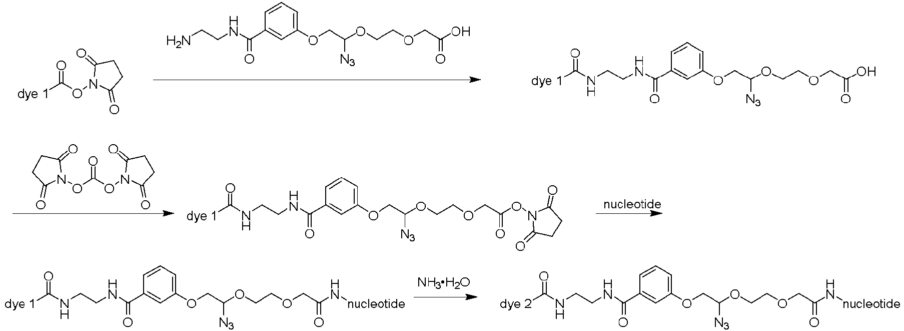 wherein, dye 2 is the compound, the ester thereof or the salt thereof as claimed in any one of claims 1-16, the compound having an acridine structure, and dye 1 is the precursor of dye 2 and has a xanthene structure.

37. The method as claimed in claim 36, wherein, the nucleotide has a structure selected from the following:
